(19) **Europäisches Patentamt**

**European Patent Office**

**Office européen des brevets**

(11) **EP 1 442 794 B1**

(12) **EUROPEAN PATENT SPECIFICATION**

(45) Date of publication and mention
of the grant of the patent:
**29.03.2006 Bulletin 2006/13**

(51) Int Cl.:
*C12Q 1/68* (2006.01)

(21) Application number: **03025810.7**

(22) Date of filing: **04.06.1997**

(54) **System and method for carrying out and monitoring biological processes**

Vorrichtung und Verfahren zur Durchführung und Überprüfung von biologischen Prozessen

Système et procédé d'execution et de suivi de processus biologiques

(84) Designated Contracting States:
**AT BE CH DE DK ES FI FR GB GR IE IT LI LU MC NL PT SE**

(30) Priority: **04.06.1996 US 658993**

(43) Date of publication of application:
**04.08.2004 Bulletin 2004/32**

(60) Divisional application:
**04022352.1 / 1 493 826**

(62) Document number(s) of the earlier application(s) in accordance with Art. 76 EPC:
**97929833.8 / 0 906 449**

(73) Proprietor: **University of Utah Research Foundation**
**Salt Lake City,**
**Utah 84112 (US)**

(72) Inventors:
• **Wittwer, Carl T.**
**Salt Lake City, UT 84109 (US)**
• **Ririe, Kirk M.**
**Salt Lake City, UT 84108 (US)**
• **Rasmussen, Randy P.**
**Salt Lake City, UT 84108 (US)**
• **Hillyard, David R.**
**Salt Lake City, UT 84124 (US)**

(74) Representative: **Elsy, David et al**
**Withers & Rogers LLP**
**Goldings House,**
**2 Hays Lane**
**London SE1 2HW (GB)**

(56) References cited:
EP-A- 0 636 413           WO-A-92/20778
WO-A-93/20240           WO-A-95/21382
US-A- 3 219 416           US-A- 5 455 175

• WITTWER C T ET AL: "RAPID CYCLE DNA AMPLIFICATION" POLYMERASE CHAIN REACTION, BOSTON, BIRKHAUSER, US, 1994, pages 174-181, XP002042556

EP 1 442 794 B1

**Description**

BACKGROUND

1. The Field of the Invention.

This invention relates generally to apparatus which are used for carrying out the polymerase chain reaction. More specifically, the present invention relates to apparatus and methods which carry out thermal cycling and monitoring of the polymerase chain reaction.

2. The Background Art .

In numerous areas of industry, technology, and research there is a need to reliably and reproducibly subject samples to thermal cycling. The need to subject a sample to repeated thermal cycles is particularly acute in biotechnology applications. In the biotechnology field, it is often desirable to repeatedly heat and cool small samples of materials over a short period of time. One such biological process that is regularly carried out is cyclic DNA amplification.

Cyclic DNA amplification, using a thermostable DNA polymerase, allows automated amplification of primer specific DNA, widely known as the "polymerase chain reaction" or "PCR." Automation of this process requires controlled and precise thermal cycling of reaction mixtures usually contained in a plurality of containers. In the past, the container of preference has been a standard, plastic microfuge tube.

Commercial programmable metal heat blocks have been used in the past to effect the temperature cycling of samples in microfuge tubes through the desired temperature versus time profile. However, the inability to quickly and accurately adjust the temperature of the heat blocks through a large temperature range over a short time period, has rendered the use of heat block type devices undesirable as a heat control system when carrying out processes such as the polymerase chain reaction.

Moreover, the microfuge tubes which are generally used have disadvantages. The material of the microfuge tubes, their wall thickness, and the geometry of microfuge tubes is a hindrance to rapid heating and cooling of the sample contained therein. The plastic material and the thickness of the wall of microfuge tubes act as an insulator between the sample contained therein and the surrounding medium thus hindering transfer of thermal energy. Also, the geometry of the microfuge tube presents a small surface area to whatever medium is being used to transfer thermal energy. The continued use of microfuge tubes in the art, with their suboptimal geometry, indicates that the benefits of improved thermal transfer (which come by increasing the surface area of a sample container for a sample of constant volume) has heretofore not been recognized.

Furthermore, devices using water baths with fluidic switching, (or mechanical transfer) have also been used as a thermal cycler for the polymerase chain reaction. Although water baths have been used in cycling a polymerase chain reaction mixture through a desired temperature versus time profile necessary for the reaction to take place, the high thermal mass of the water (and the low thermal conductivity of plastic microfuge tubes), has been significantly limiting as far as performance of the apparatus and the specificity of the reaction are concerned.

Devices using water baths are limited in their performance. This is because the water's thermal mass significantly restricts the maximum temperature versus time gradient which can be achieved thereby. Also, the water bath apparatus has been found to be very cumbersome due to the size and number of water carrying hoses and external temperature controlling devices for the water. Further the need for excessive periodic maintenance and inspection of the water fittings for the purpose of detecting leaks in a water bath apparatus is tedious and time consuming. Finally, it is difficult with the water bath apparatus to control the temperature in the sample tubes with the desired accuracy.

U.S. Patent No. 3,616,264 to Ray shows a thermal forced air apparatus for cycling air to heat or cool biological samples to a constant temperature. Although the Ray device is somewhat effective in maintaining a constant temperature within an air chamber, it does not address the need for rapidly adjusting the temperature in a cyclical manner according to a temperature versus time profile such as is required for biological procedures such as the polymerase chain reaction.

U.S. Patent No. 4,420,679 to Howe and U.S. Patent No. 4,286,456 to Sisti et al. both disclose gas chromatographic ovens. The devices disclosed in the Howe and Sisti et al. patents are suited for carrying out gas chromatography procedures but do not provide thermal cycling which is substantially any more rapid than that provided by any of the earlier described devices. Rapid thermal cycling is useful for carrying out many procedures. Devices such as those described in the Howe and Sisti et al. patents are not suitable for efficiently and rapidly carrying out such reactions.

In particular, the polymerase chain reaction (PCR) is a fundamental DNA amplification technique essential to modern molecular biology. Despite its usefulness and popularity, the current understanding of PCR is not highly advanced. Amplifications must be optimized by trial and error and protocols are often followed blindly. The limited understanding of PCR found in the art is a good example of how those skilled in the art are content to utilize a powerful technique without reflection or comprehension.

Biological processes such as PCR require temperature cycling of the sample. Not only does the prior art, as explained above, carry out temperature cycling slowly, the prior art also ignores the underlying principles which allow PCR to work and could be used to make PCR even more useful. Thus, it would be a great advance in the art to provide methods and apparatus which are particularly adaptable for rapidly carrying out PCR and analyzing the reaction which is taking place, particularly if such reaction is analyzed as it is taking place, that is, in real time.

EP 0 636 413A discloses an apparatus and methods for carrying out PCR in capillary tubes. EP 0 580 362 discloses an apparatus and methods for carrying out PCR using stationary tubes as containers and optical fibres between the tubes and light source and detecting apparatus. EP 0 640 828A shows a system for monitoring all PCR samples in the system simultaneously, for example using a CCD Camera. Wittwer C.T et al (Nucl. Acids Res (1984), Vol 17 (11) pages 4353-4357) shows forced air heating apparatus for PCR.

BRIEF SUMMARY AND OBJECTS OF THE INVENTION

In view of the above described state of the art, the present invention seeks to realize the following objects and advantages.

It is an object of the present invention to provide an apparatus for accurately controlling the temperature of biological samples.

It is a further object of the present invention to provide a thermal cycling apparatus for quickly and accurately varying the temperature of biological samples according to a predetermined temperature versus time profile.

It is another object of the present invention to provide an apparatus suitable for subjecting a number of different biological samples to rapid thermal cycling.

It is also described how to provide a thermal cycling apparatus having a thermal transfer medium of low thermal mass which can effectively subject samples to a large temperature gradient over a very short period of time.

It is a further object of the present invention to provide an apparatus which can subject a biological sample to rapid thermal cycling using air as a thermal transfer medium.

It is an object of the present invention to provide a system and method for performing PCR rapidly and for simultaneously monitoring the reaction.

It is another object of the present invention to provide a system and method for performing PCR rapidly and also continuously monitoring the reaction while it is ongoing.

It is also described how to replace the nucleic acid probes by synthetic nucleic acid analogs or derivatives, e.g., by peptide nucleic acids (PNA), provided that they can also be labeled with fluorescent compounds.

These and other objects and advantages of the invention will become more fully apparent from the description and claims which follow, or may be learned by the practice of the invention.

An apparatus is provided which is particularly suited for subjecting biological samples to rapid thermal cycling in order to carry out one or more of a number of procedures or processes. The invention is defined by the claims. The structure which holds a biological sample, also referred to as a sample chamber, may be provided with an insulation means for retaining thermal energy and also a means for heating the interior of the sample chamber. In further embodiments, cool air is conveyed into and out of a chamber holding the biological sample.

In order to rapidly cool the sample chamber, the preferred apparatus includes a means for forcing air into the sample chamber and a means for dispersing the air forced into the sample chamber. The preferred structures included in some embodiments are a high velocity fan which functions to force air into the sample chamber and a rotating paddle which functions to disperse the air into the chamber. In some embodiments, a means for venting allows the air to escape from the sample chamber taking the unwanted heat with it. The present invention allows heating and cooling of a sample to take place both quickly and uniformly.

In accordance with the method and the apparatus of the present invention; a control structure provides means for operating the thermal cycling means.

The controller allows the biological samples to pass through a predetermined temperature cycle corresponding to the denaturation, annealing and elongation steps in the polymerase chain reaction. In use, the apparatus allows rapid optimization of denaturation, annealing, and elongation steps in terms of time and temperature, and shortened time periods (ramp times) between the temperatures at each step.

The present invention particularly decreases the total time required for completion of polymerase chain reaction cycling over prior art thermal cycling devices while at the same time significantly increasing specificity and yield.

The present invention provides methods and apparatus for continuous fluorescence monitoring of the polymerase chain reaction procedure. In the present invention, optical components are combined with structures to provide rapid temperature cycling in order to continuously monitor DNA amplification by a variety of different fluorescence techniques. Glass capillary sample containers allow rapid heat transfer from the preferred thermal transfer medium (allowing 30 amplification cycles in less than 15 minutes when a gas such as air is used as the thermal transfer medium) and simultaneous monitoring of the reaction.

In accordance the present invention, optical techniques are used to monitor the progress of the reaction as the reaction is ongoing. In some preferred embodiments of the invention, flourescent probes are added to the reaction mixture. The present invention then monitors the fluorescence at least once during a temperature transition, and preferably the fluorescence is acquired two or more times during a temperature transition, either from a single sample or from multiple samples. A rotating carousel is included to sequentially move the samples, one-by-one, to a monitoring location with all of the samples being simultaneously subjected to rapid thermal cycling. Desirably, embodiments of the present invention provide for monitoring of fluorescence once per amplification cycle or monitoring temperature, time, and fluorescence continuously throughout each amplification cycle.

Using the present invention, a 3-dimensional plot of temperature, time, and fluorescence, can be obtained. Fluorescence vs. temperature plots of hybridization probes discriminate between the cumulative, irreversible signal of exonuclease cleavage and the temperature-dependent, reversible hybridization of adjacent probes. Hybridization probes are more useful than hydrolysis probes because the temperature dependence of fluorescence can be followed and used to detect alterations in product sequence, i.e., polymorphisms and mutations. Using dyes that fluoresce in the presence of double stranded DNA, product denaturation, reannealing and extension can be followed within each cycle. The present invention provides apparatus and methods for rapidly carrying out PCR amplification reactions which combines amplification and analysis of the reaction in under fifteen minutes and more preferably in under fifteen minutes and most preferably in under ten minutes.

BRIEF DESCRIPTION OF THE DRAWINGS

In order to better appreciate how the above-recited and other advantages and objects of the invention are obtained, a more particular description of the invention briefly described above will be rendered by reference to the appended drawings. Understanding that these drawings are not to be considered limiting of its scope, the invention will be described and explained with additional specificity and detail through the use of the accompanying drawings which do not all show embodiments falling within the claimed invention.

Figure 1 shows a perspective view of a thermal cycling apparatus adapted for thermal cycling of biological samples and adapted especially for use in cyclic DNA amplification.

Figure 2 is a side elevation view of the fluid chamber portion of the apparatus of Figure 1.

Figure 3 is an interior plan view of the fluid chamber portion of the apparatus illustrated in Figure 1.

Figure 4 shows an interior plan view of another fluid chamber of another embodiment of the present invention.

Figure 5 shows an optimized temperature versus time profile for a polymerase chain reaction using the thermal cycling device.

Figure 6 shows graphically the effect of denaturation time on polymerase chain reaction yields using one thermal cycling device.

Figure 7 shows graphically the effect of annealing time on polymerase chain reaction specificity and yields using the thermal cycling device.

Figures 8A-B, which are perspective and elevational cross sectioned views, respectively, of another preferred embodiment of a comparative example.

Figure 8C is a diagrammatic representation of the relationship of the heat producing element and the capillary tubes holding the biological samples in the comparative example illustrated in Figures 8A-B.

Figure 9A is a comparative example and shows the results of four different temperature/time profiles (A-D) and their resultant amplification products after thirty cycles (A-D).

Figure 9B is a comparative example and shows a cycle of another preferred temperature/time profile. Figures 9C-G show exemplary cycles of other preferred temperature/time profiles.

Figure 10 provides a block diagram of a temperature slope control circuit of the comparative example.

Figure 10A is a graphical representation of the effect of the temperature transition rate from the product denaturation temperature to the primer annealing temperature on reaction product specificity.

Figure 11 is a schematic view of a preferred rapid temperature cycler with fluorescence detection in accordance with the present invention.

Figure 11A is a temperature v. time chart of showing one preferred operation of the apparatus of Figure 11.

Figure 12 is a representation of three dimensional plots of temperature, time, and fluorescence during amplification of a hepatitis B DNA fragment in the presence of SYBR Green I.

Figures 12A-C are representations of two dimensional plots of temperature vs. time, fluorescence vs. time, and fluorescence vs. temperature which are together shown as a three dimensional plot in Figure 12.

Figure 13 is a plot of fluorescence vs. temperature during the amplification of a 536 base pair fragment of the human β-globin gene in the presence of SYBR Green I.

Figure 14 is a plot of fluorescence vs. cycle number obtained in accordance with an aspect of the present invention.

Figure 14A provides a legend for Figure 14. and subsequent figures, indicating different initial template copy numbers.

Figure 15 is a plot of fluorescence vs. cycle number obtained in accordance with an aspect of the present invention.

Figure 16 is a fluorescence ratio vs. temperature plot obtained in accordance with one aspect of the present invention.

Figure 17 is a fluorescence ratio vs. temperature plot obtained in accordance with one aspect of the present invention.

Figure 18A is a graph representing an equilibrium PCR paradigm.

Figure 18B is a graph representing a kinetic PCR paradigm.

Figure 18C is a graph representing different time/temperature profiles near an annealing temperature.

Figure 19 represents a comparative example configured for continuous monitoring of a sample.

Figures 19A-19D are representations of different sample container configurations.

Figure 19E is a chart which shows the effect of the different sample container configurations of Figures 19A-D on the temperature response of the sample itself.

Figures 19F and 19G are side and end views, respectively, of one preferred sample container.

Figures 19H and 19I, respectively, show two possible orientations of a rectangular capillary tube when detecting fluorescence of the sample.

Figure 20 shows the optical layout to provide continuous monitoring of a sample undergoing DNA amplification.

Figure 21 is a schematic representation which is a rapid temperature cycler with fluorescence detection at the tip of the sample containers.

Figures 21A-0 show composite plastic/glass containers into which biological samples are loaded.

Figure 22 illustrates useful temperature vs. time segments for fluorescence hybridization monitoring.

Figure 22A charts the effectiveness of light piping by viewing the tip rather than the side of capillary sample container.

Figure 22B charts the efficiency of light piping by two different sizes of capillary sample tubes.

Figure 22C is a high level block diagram showing the tasks which are performed by one preferred embodiment of the present invention which includes a rapid temperature cycler with epifluorescence detection.

Figure 22D is a plot of temperature vs. time for a PCR reaction in which fluorescence feedback was used to control reaction parameters.

Figure 22E is a plot of fluorescence vs. time for a PCR reaction in which fluorescence feedback was used to control reaction parameters.

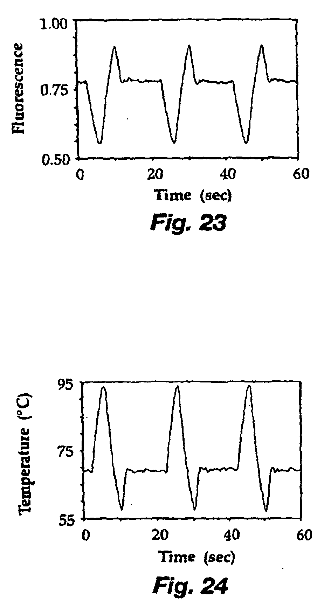

Figure 23 is a plot of fluorescence vs. time showing showing the inverse relationship between temperature and fluorescence.

Figure 24 is a plot of temperature vs. time showing the inverse relationship between temperature and fluorescence.

Figure 25 is a plot of fluorescence vs. temperature for three different PCR products in the presence of SYBR Green 1 acquired during a 0.2 degree per second temperature transition through the product melting temperatures.

Figure 26 is a plot of fluorescence vs. time showing product annealing for different concentrations of PCR product in the presence of SYBR Green 1.

Figures 27A and 27B are cross sectional schematic views of the embodiment represented in Figure 28 in a run mode and a load mode, respectively.

Figure 28 is a schematic representation of a rapid temperature cycler with fluorescence detection at the tip of the sample containers and which includes positioning for fluorescence detection in two dimensions to optimize detection.

Figure 29 is a perspective view of the exterior of the embodiment illustrated in the schematic representation of Figure 28.

Figures 30A-30V are detailed schematic diagrams of the electrical components.

Figures 31A and 31B are perspective and cross sectional views, respectively, of a sample handling system.

Figure 32 is a schematic representation of another embodiment which accommodates multiple sample handling trays.

## DETAILED DESCRIPTION OF THE PREFERRED EMBODIMENTS

The present invention is directed to an apparatus and method as defined in the claims for carrying out thermal cycling and monitoring of biological reactions.

In accordance with one embodiment, the step of placing the first PCR sample comprises the step of placing a volume of the PCR samples in each container, the volume of the PCR samples being not greater than about 10,000 $\mu$l containers being at least partially fabricated from glass.

The system can further comprise means for adjusting the control means in accordance with the reaction parameter. In one embodiment, the means for determining at least one reaction parameter in accordance with the detected fluoresce comprises computing means for determining a reaction parameter selected from the group consisting of product melting temperature and time, product reannealing temperature and time, probe melting temperature and time, primer annealing extension temperature and time.

A carousel may comprise:

a disc having a top surface, a bottom surface, an outer edge extending therebetween, a sample receiving port in the top surface, a sample vessel port in the outer edge and a sample passageway communicating with said sample receiving port and the sample vessel port, said sample vessel port and passageway formed for receiving and fixing a sample vessel to the disc.

The carousel may further comprise a closure for the sample receiving port. In addition, the passageways of the carousel may include a barrier that prevents a liquid sample delivered through the sample receiving port from flowing to the sample vessel port absent a biasing force on said liquid sample.

In one embodiment the sample vessel is a capillary tube having a volume to surface area of greater than 1mm. In one embodiment, the passageway of the carousel communicates with the sample vessel port, the sample receiving port, and at least one additional port formed in said top surface. In accordance with one embodiment, the carousel further comprises a predetermined reagent mixture in the passageway between the sample receiving port and the sample vessel port.

Reference will now be made to the drawings wherein like structures will be provided with like reference designations.

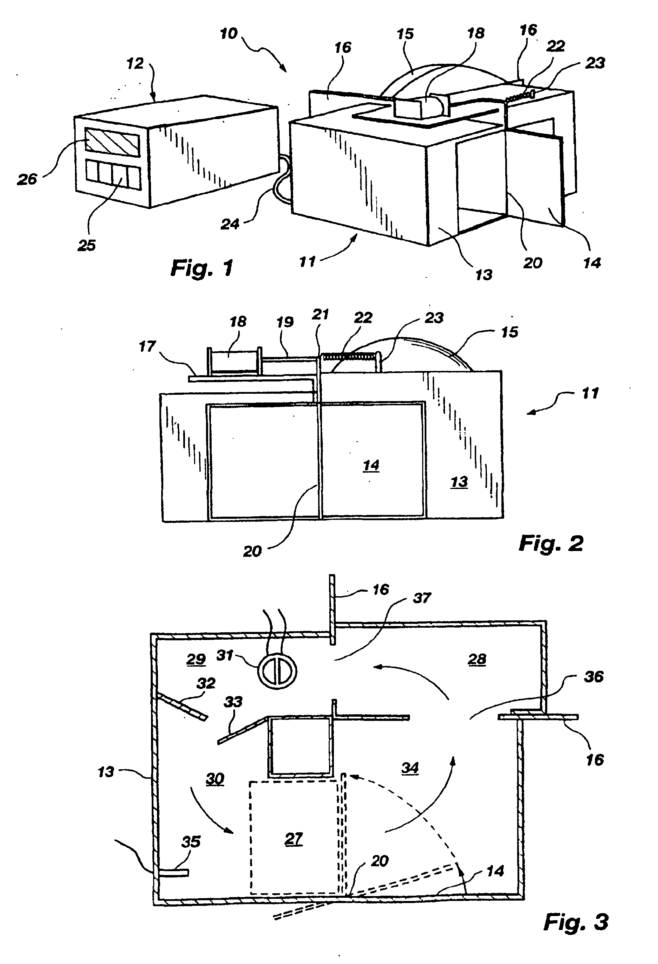

As shown in Figure 1, the one preferred thermal cycling device 10 includes a closed loop fluid (most preferably air) chamber, generally designated at 11, which is adapted to accept samples to be cycled through vent door 14. The closed loop fluid chamber 11 includes a plurality of compartments each of which will be described shortly. The device 10 also includes a controller 12 which can be programmed by means of input keys 25 and display 26 to cause the chamber 11 to be cycled through a series of temperatures over a predetermined period of time. The thermal cycling of chamber 11 can be used to carry out numerous procedures and is particularly suited for amplification of primer specific DNA from samples containing reaction mixtures as will be explained below.

The closed loop fluid chamber 11 is enclosed in a generally box shaped configuration by housing 13. Blower mounting boards 16, if desired, can be located so as to section off a smaller rectangular section of the chamber 11 and function to support and secure a generally cylindrically shaped lower housing 15 thereto. Alternatively, the fan of the blower 28 may be housed integrally within chamber housing 13.

The interior of blower housing 15 contains the blades and shaft of the blower. The blower motor (not shown) is located externally of blower housing 15, and therefore exteriorly of the enclosed chamber 11. In this configuration, the blades and shaft are the only parts of the blower which become exposed to the circulating hot fluid within chamber 11. It would be disadvantageous to mount the motor within the chamber which would subject the motor to temperature variations and also would add the thermal mass of the motor to that which is subject to heating and cooling. The reduction of thermal mass exposed to the fluid in chamber 11 is desirable to the overall performance of the device 10 in its function of subjecting samples placed therein to a desired temperature versus time profiles, using either predetermined profiles or by altering one or more reaction parameters as the reaction continues, as will be more fully explained below.

The blower 28 is a well known type of blower usually identified as an "in line" type blower which preferably employs a propeller type fan, due to its generally low thermal mass, or if desired, a squirrel cage type fan, the fan preferably having a 75 cubic feet per minute minimum capacity.

The solenoid platform 17 has secured thereto a solenoid 18. The solenoid armature 19 is attached to upper end 21 of rod 20 which is rigidly attached to vent door 14 and rotatably attached to housing 13 at points above and below the vent door 14. The rod 20 therefore allows vent door 14 to freely rotate relative to the housing 13 about the rod's longitudinal axis.

A spring 22 is attached at one of its ends to the housing 13 by support post 23. The opposite end of spring 22 is attached to the top end 21 of rod 20 directly adjacent the attachment of solenoid armature 19. The spring 22 is drawn between these two attachment points so as to be in tension. The spring 22 therefore tends to draw top end 21 toward the support post 23, which in turn tends to rotate vent door 14 to its closed position. When solenoid 18 is actuated, armature 19 tends to pull top end 21 of the rod 20 in the direction of the solenoid 18, which is opposite the direction of pull of spring 22, and which tends to open the vent door 14.

Controller, generally designated at 12, is electrically attached to the chamber 11 by means of a transmission cable 24. The cable 24 also supplies power to the blower motor (not shown), and to the heat coil 31. Further, the controller 12 also is connected to thermocouple sensor 35 for receiving signals corresponding to temperature data, and to solenoid 18 for triggering the solenoid armature.

Controller 12 can be any well known type of temperature controller unit which is programmable to control the heat coil 31, vent door 14, and blower so as to achieve predetermined temperatures as a function of time within the chamber 11, and which is also capable of being programmed to actuate a relay output for driving a solenoid at predetermined time periods and chamber temperature levels. A preferred temperature controller 12 for use in the embodiment of Figures 1-3 is a Partlow MIC-6000 proportional temperature controller, available through Omega Engineering Inc, of Stanford, Connecticut, as the Model No. CN8600 process controller.

As shown in Figures 2 and 3, the interior of chamber 11 is sectioned off into four main compartments. The blower compartment 28 is formed of the blower housing 15 and the blower mounting plates 16. The entirety of blower compartment 28 is filled with the fan and shaft portions of a blower as has been described above. The blower can be any of a number of well-known designs, as has been described above, and has therefore been omitted from Figure 3 for purposes of clarity. It is sufficient to understand that the fan located in blower compartment 28 draws fluid into the blower compartment 28 through inlet opening 36 and pushes the fluid out of exit opening 37.

It is preferred that the fluid be driven by the blower at a rate of at least 75 cubic feet per minute. It is important however, to realize that the fluid located in chamber 11 only contacts the fan and a portion of the drive shaft of the blower, the blower motor itself being located outside of the blower housing 15 so as to avoid any contact thereof with fluid in the chamber 11. This consideration contributes to the speed of operation to minimize the material which contacts the fluid inside the chamber 11 so as to minimize the thermal mass of material which must be heated and/or cooled thereby during the cycling process. By minimizing the thermal mass which must be heated or cooled by the fluid, the response time necessary to bring the contents of chamber 11 to a uniform temperature is greatly diminished.

Fluid exiting blower compartment 28 through outlet opening 37 enters heating compartment 29. Fluid passing into heating compartment 29 must pass by heating coils 31. If the heating coils 31 get hotter than the fluid passing into heating compartment 29, the fluid will become heated thereby as it is forced through the compartment. The heating coil is preferably a 1,000 watt (125 VAC) nichrome wire coil wound around a microsupport. However, any heating unit suitable for heating the type of fluid present in the chamber may be used. The particular heating coil of embodiment of Figures 1-3 is manufactured by Johnstone Supply, of Portland, Oregon.

The heating coil is activated by an output relay included in the controller 12. The preferred relay is a 25 A, 125 VAC solid state relay manufactured by Omega Engineering Inc. of Stanford, Connecticut as Model No. Omega SSR 240 D25.

Fluid passing through heating compartment 29 becomes incident on baffles 32 and 33 before passing into the reaction compartment 30. Baffles 32 and 33 tend to break up any laminar fluid flow and generate turbulence therein to effectively mix the fluid so that it arrives in reaction compartment 30 at an homogenous temperature.

Thermocouple sensor 35 provides an electrical input signal to controller 12 which corresponds to the fluid temperature in the reaction compartment 30. Temperature monitoring during operation of the thermal cycling device 10 is preferably achieved by a 30-gauge iron-constantan "J-type" thermocouple. The controller uses this information to regulate the heat coil 31 according to the predetermined temperature versus time profiles programmed therein and to actuate solenoid 18, as will be explained momentarily.

The fluid passing from the reaction compartment 30 to the return air compartment 34 must pass through sample compartment 27 (as shown in dashed lines). Sample compartment 27 will also be explained momentarily.

The fluid in return compartment 34 has been slightly cooled due to the heat transfer therefrom into samples located in sample compartment 27. The fluid in return compartment 34 is drawn through inlet opening 36 into blower compartment 28 where it is again forced, by action of the fan, out through outlet opening 37 into the heating compartment 39. Thus, the fluid chamber 11, when operating with vent door 14 closed, is a closed loop fluid chamber which continuously recirculates the fluid along a closed loop path through each compartment thereof in order to bring the contents therein to a uniform temperature. Continuous circulation of the air in the air chamber 11 allows the samples in sample compartment 27 to be brought to a predetermined temperature as quickly as possible, and then to be held at that temperature, if desired.

When the device 10 must be used to not only heat material located in the reaction compartment 27, but also to subsequently cool these materials as quickly as possible to a temperature at or above the ambient fluid (air) temperature, the controller 12 can be programmed to actuate solenoid 18 to cause vent door 14 to open and allow large quantities of ambient fluid to immediately flood the compartment 11 while heated fluid therein simultaneously escapes.

Deactivation of the heating coil 31 while continuing activation of the blower with vent door 14 open, will draw ambient fluid into return compartment 34 and from there into the blower compartment 28. The blower will then push this ambient fluid through heating compartment 29 where it will pass directly into reaction compartment 30 without being heated by coil 31. The ambient fluid then passes through the sample compartment 27 and escapes out of chamber 11 through the vent door 14. Due to the minimum thermal mass of material located in chamber 11, and the action of the blower fan, vast quantities of ambient fluid will be forced past the sample compartment 27, and from there out of the chamber 11. Thus, rapid cooling of samples or material located in the reaction compartment 27 is obtained.

The sample compartment 27 is sized so as to allow a plurality of samples, such as hollow elongate glass tubes containing a sample therein, to be easily located in a spaced apart orientation so that fluid may be evenly distributed around each sample. If desired, the sample compartment 27 may be sized and configured so as to allow insertion of a rack, basket, or the like which has been configured so as to accept a plurality of samples in uniform spaced apart configuration so as to simplify loading the samples into the sample chamber 27.

Access to sample compartment 27 is accomplished by rotation of the vent door 14 to its open position. Once the vent door 14 is rotated to approximately 90 degrees from it's closed position, the sample compartment 27 is easily accessible there through. Also, as can be seen in Figures 1-3, rotation of vent door 14 approximately 90 degrees from its closed position causes return fluid compartment 34 to be substantially closed off from the reaction compartment 30.

Thus, when the device 10 is in a "cooling" mode, ambient fluid enters directly into the return fluid compartment 34 and is forced through the blower compartment 28, heating compartment 29, reaction compartment 30, and sample compartment 27 substantially along the same path as the closed loop fluid flow path described above. The fluid is then forced out of the air chamber 11 and prevented from passing back into air return compartment 34 by the positioning of the vent door 14 between the sample compartment 27 and the return fluid compartment 34.

Thus, the vent door 14 not only allows ambient fluid to enter the chamber 11, it can also prevent the fluid from recirculating in a loop fashion through the chamber 11. Instead, fluid is forced to pass through the sample compartment 27 and then out of the chamber 11 to aid in the rapid cooling of the sample contents and chamber 11.

When the device 10 is used for cyclic DNA amplification, repetitive cycling through different temperatures is required. Samples containing a reaction mixture for the polymerase chain reaction generally must be cycled approximately 30 times through temperature changes which correspond to the denaturation, annealing and elongation phases of the amplification process.

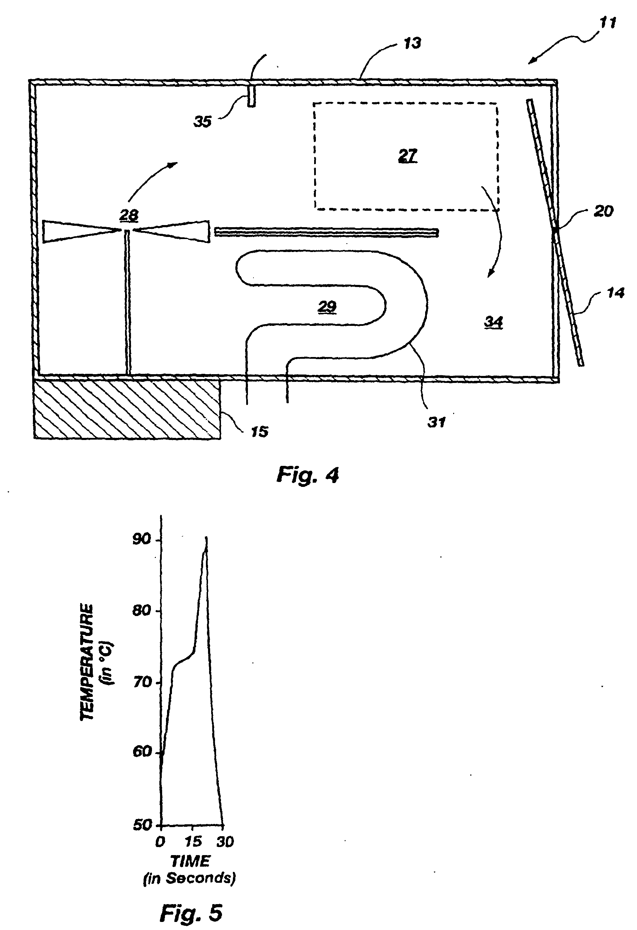

The device 10, due to its novel characteristics described above, is capable of cycling samples in significantly shortened periods compared to the prior art. For example, the DNA amplification application represented in the figures can pass through a temperature versus time profile cycle in 30-60 seconds (see Figure 5). This same cycle using prior art devices would take approximately 5-10 times longer. These low cycle times have proven also to increase yield and specificity of the polymerase chain reaction over prior art cycling.

## Example 1

The polymerase chain reaction was run in a 10 $\mu\ell$ volume with 50 ng of human genomic template DHAes, 0.5 mM of each deoxynucleotide, 500 nM of each of two oligonucleotide primers GGTTGGCCAATCTACTCCCAGG (SEQ ID NO:5) and GCTCACTCAGTGTGGCAAAG (SEQ ID NO:6) in a reaction buffer consisting of 50 mM Tris-HCl (pH 8.5 at 25°C), 3.0 mM magnesium chloride, 20 mM KCl, and 500 $\mu$g/ml bovine serum albumin. Thermus aquatics DNA polymerase (0.4 $\mu$) was added, the samples placed in B cm long, thin-walled capillary tubes (manufactured by Kimble, Kimax 46485-1), and the ends fused with a laboratory gas burner so that an air bubble was present on both ends of each tube.

The capillary tubes were then placed vertically in a holder constructed of 1 mm thick "prepunched perfboard" (manufactured by Radio Shack). The mixture was cycled 30 times through denaturation (90-92°C), annealing (50-55°C), and elongation (72-75 °C) to give the temperature versus time profile of Figure 5. Temperature monitoring of the capillary tubes was done with a miniature thermocouple (IT-23, Sensortek, Clifton, NJ) placed in 10 $\mu\ell$ of deionized water and connected to a thermocouple monitor (BAT-12, Sensortek). Amplification products were fractionated by electrophoresis on a 1.5% agarose gel. Specific amplification products were obtained in good yield.

Due to the fact that the device 10 uses air as the thermal transfer medium instead of water, it has the advantage that heat transfer occurs through a low heat capacity medium (air) which can be warmed very rapidly.

The response time for sample cooling is very fast due to the use of thin walled glass capillary tubes for holding samples, instead of plastic microfuge tubes as has been done in the past with prior art processes, and by minimizing the thermal mass of material inside the chamber 11 (see Figure 5). Such response times can allow for optimization of the time and temperature requirements for the denaturation, annealing, and elongation steps in the polymerase chain reaction.

Further, shortened "ramp" times are obtained, i.e., the time required to bring the temperature of the sample from one temperature level to the next temperature level corresponding to phases in the amplification process is shortened. This decreases the time required for a complete amplification, as well as allowing specific study of annealing, denaturation and enzyme kinetics within a polymerase chain reaction protocol.

The baffles 32 and 33 (as shown in Figure 3) may be used if desired to achieve better temperature homogeneity within the sample compartment 27. As shown, baffles 32 and 33 decrease the temperature variation in the reaction compartment 30 from about 10°C, to about 2°C. If desired, further (or more complicated) baffles may be used to further decrease the temperature variation in reaction compartment 30. Alternately, as shown in Figure 4 the fan may be positioned downstream from the heating coil 31, but before the sample compartment 27 to achieve more uniform mixing.

Amplification products obtained through the use of apparatus 10 are at least qualitatively and quantitatively as desirable as those obtained through the manual water bath cycling method. However, advantages in specificity and yield are possible with rapid thermal control of the reaction mixture.

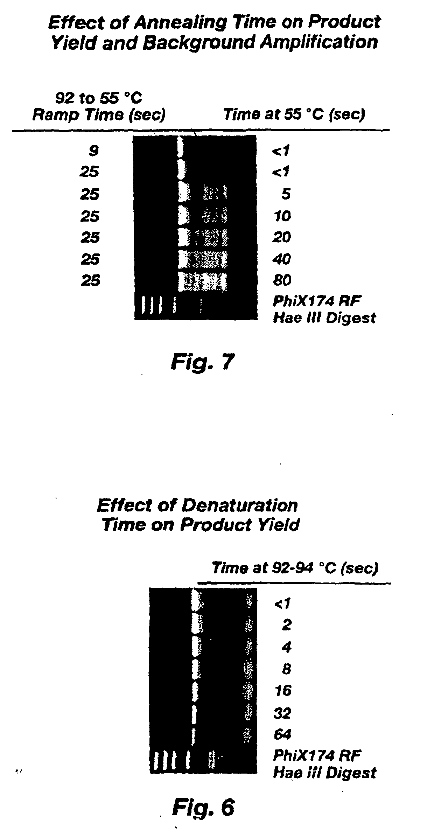

Figure 6 shows the effect of the temperature versus time profile of Figure 5 as used with the thermal cycling apparatus 10 on specificity (i.e., one specific product yield as opposed to a plurality of similar or "shadow" products). As can be seen, the shorter the ramp and annealing time, the greater the product specificity. The rapid temperature response of the apparatus 10 allows improved specificity and yield which is not possible with prior art systems.

Figure 7 shows the effect of varying the denaturation time of the temperature versus time profile of Figure 5 as used with the thermal cycling apparatus 10 of the present invention on DNA amplification yields. The brighter vertical lines each correspond to a particular time at a denaturation temperature. As can be seen, the yield is greatest at the shortest possible denaturation time. Such a result is not possible with prior art systems.

As has been shown, by decreasing the thermal capacity [thermal mass] of the apparatus 10, it can markedly decrease the total time required for carrying out the polymerase chain reaction. In addition, the use of small sample volumes further shortens the total time required for the reaction and also reduces the amounts of expensive reagents which must be used by up to about 90%, thus further reducing the cost of carrying out procedures. For example, in the comparative example represented in Figures 1-3, capillary tubes 108 having inner diameters in the range from about 0.25mm to about 1.0mm can desirably be used. In some applications, capillary tubes 108 having inner diameters in the range from about 0.02mm to about 0.1mm can also be desirably used.

The apparatus 10 is useful for amplifying DNA from any source. Although particular configurations and arrangements have been discussed in connection with the thermal cycling device 10, other arrangements and configurations may be utilized. For example, various fluids other than air, of generally low thermal mass, may alternatively be used in the device 10.

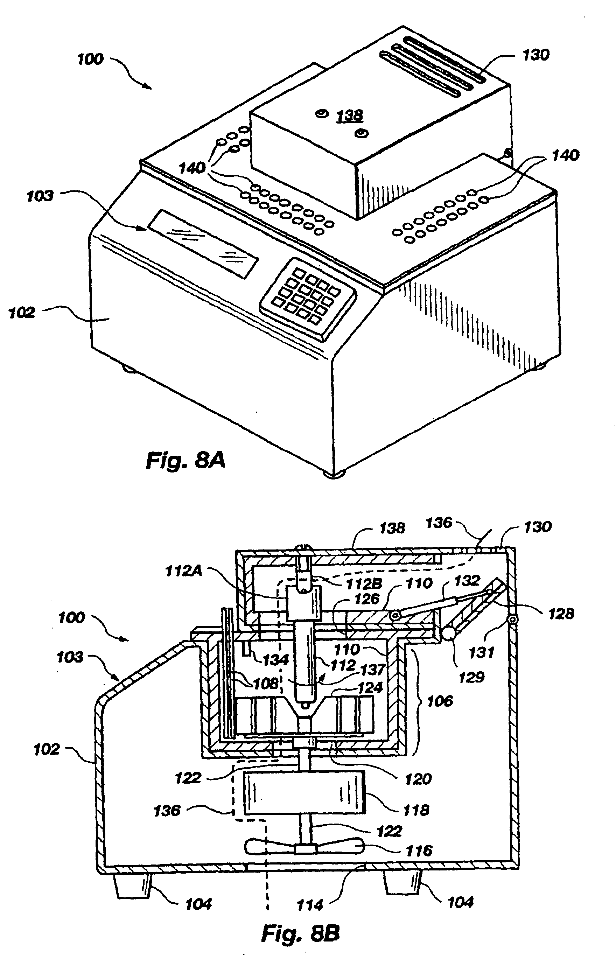

A further comparative example is represented in Figures 8A-C. Figure 8A is a perspective view and Figure 8B is an elevational cross sectioned view of the additional embodiment. It will be understood that many of the earlier explained components and teachings also have application in the example illustrated in Figures 8A-C. Thus, only example the pertinent additional information concerning this example will be provided below. Importantly, in the example of Figures 8A-C, the heat producing element is adjacent to the biological sample containers allowing faster heating and cooling of biological samples as explained below.

As will be appreciated shortly, the apparatus of Figures 8A-C provides even greater improvement over the prior art in the speed at which thermal cycling can be carried out, e.g., 15 or 30 cycles of DNA amplification in 30, 15, 10, or even fewer, minutes. Furthermore, the apparatus 100 provides better thermal homogenization throughout the samples than previously possible.

Shown in Figure 8A is the general configuration of the housing 102 of the example. The housing 102 rests on feet 104 (best seen in Figure 8B) and functions to hold the other described structures in place and to isolate those structures which become hot from the surrounding environment. Included in the feature 100 of Figure 8A are input keys 25 and a display 26 as in the previously described apparatus 10. The previously described control structures can readily be modified or used as a pattern for a control means for use in the example of Figures 8A-C.

As shown best in the cross sectional view of Figure 8B, a sample chamber is designated by bracket 106. A lid 138 connected to the housing 102 by a hinge 131 can be opened to allow access to the sample chamber 106. The sample chamber 106 is preferably cylindrical in shape but can be of any shape or size required by the particular application.

The sample chamber 106 is preferably lined with a black colored foam material 110 whose surface has light absorbing characteristics with the bulk of the thickness of the foam having insulating characteristics. The black foam material can be one which is readily available in the art and one fabricated from a plastic material. The foam 110 is preferably a material which is readily cooled by the air passing there over, i.e., the material has low thermal conductivity and a porous surface.

The dark or black porous surface of the material converts shorter wavelength radiation striking the surface into longer wavelength radiation, i.e., heat, which is radiated into the sample chamber.

The foam 110 functions to thermally isolate the sample chamber from the surrounding air space in the housing and also to convert the light emitted by lamp 112 into thermal energy. The foam 110 can be replaced with other structures. For example, a material having a black, dark, or other nonreflective surface, such as a thin sheet of polycarbonate having one surface painted black, can be backed by an insulative material, such as a fiberglass or foam material. The black or dark surface, which can be painted on a number of different substrates, converts shorter wavelength radiation striking it into thermal radiation while the insulative material thermally isolates the sample chamber from the surrounding environment. Thus, using the teachings provided herein, those skilled in the art can utilize many different materials and structures as a lining for the sample chamber.

The lamp 112 is preferably a 500 watt halogen lamp. If appropriate control devices are used, higher power lamps or a plurality of lamps, such as four 500 watt halogen lamps, can be used. A lamp socket 112A is attached to the housing 102 by a support 112B. The lamp 112 is able to very rapidly and uniformly heat the sample chamber 106 to the desired temperature. Other sources of heat, i.e. infrared radiation, such as the earlier described nichrome wire element, can also be used.

Represented in Figure 8B are two thin-walled capillary tubes 108 such as those described earlier. While two thin-walled capillary tubes 108 are shown, the sample chamber 106 can hold many such tubes. The thin-walled capillary tubes 108 have several important advantages over previously used devices as described earlier and, together with the sample chamber 106, function as the one presently preferred example of a means for holding a biological sample.

It will be appreciated that many other structures performing equivalent or similar functions can also be used. The thin-walled capillary tubes 108 are preferably left partially extending out of the sample chamber through apertures 140 for ease of access but may be completely contained within the sample chamber 106 as may numerous other fluid holding structures which are suited to particular applications. The preferred thin-walled capillary tubes 108 have a capacity of about 10 $\mu\ell$. As will be understood, the volume of the sample should be keep small, and the surface area of the sample holding structure relatively large, and together they present a relatively small thermal mass. It is also preferred that the sample holding structure contain a volume anywhere from about 1 $p\ell$ to about 10,000 $\mu\ell$ but those skilled in the art will appreciate that other volumes of samples can also be used if the different thermal mass of the structure is considered.

The lamp 112 and the insulative foam 110 together provide rapid and uniform heating of the sample contained in the thin-walled capillary tubes 108 and the air contained within the sample chamber 106. A thermocouple 134 is included within the sample chamber 106 to sense the temperature within the chamber and is used to maintain the desired temperature within the sample chamber as earlier described.

The thermocouple 134 is preferably one available in the art whose thermal response substantially matches the thermal response of the biological sample and the container holding the same. Such thermocouples can be commercially obtained from sources such as Idaho Labs which manufactures a line of thermocouples referred to as metal sheathed, J-type thermocouples. The matching of the thermal response of the thermocouple to that of the biological sample and container can be preferably carried out by inserting a micro thermocouple, such as the model IT-23 thermocouple available from PhysiTemp as known in the art, into a typical biological sample being held by the chosen container and subjecting the sample and the thermocouple under test to the same temperature changes. The thermocouple under test, or some external criteria, can be changed until the thermal response of the thermocouple suitably matches the thermal response of the sample and its container.

The arrangement represented in Figure 8B provides more uniform heating and cooling of the sample than previously available devices. In previously available devices, transfer of heat throughout the sample is carried out by convection through the sample. Convection induced movement of the sample within whatever structure is used to hold the sample is caused by temperature gradients or differences in the generally small biological samples (e.g., 10-100 $\mu\ell$).

The effect of temperature gradients within the sample become more pronounced and more difficult to control as the cycle time for a sample decreases. The existence of uneven temperatures within a sample, and particularly the reliance on "mixing by convection" within the sample relied upon by the prior art devices, generally increases the cycle time for a sample and likely has deleterious effects on the biological sample. The apparatus 100 is capable of providing heating and cooling such that thermal differences within a 10 $\mu\ell$ sample are maintained at not greater than ±1°C at all times during a 30 second cycle.

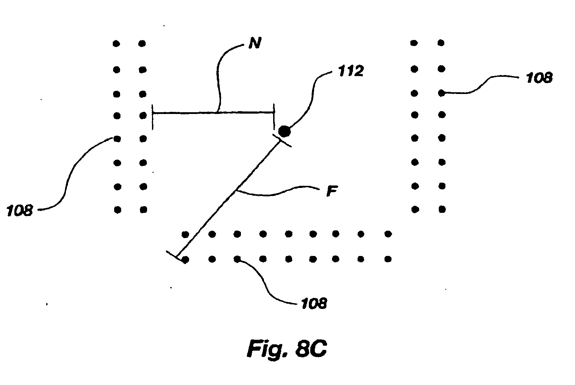

In order to promote uniform heating and cooling, it is preferred that the thin-walled capillary tubes 108 be at least somewhat uniformly spaced from the heat source, for example, lamp 112 in apparatus 100. Figure 8C provides a diagrammatic top view of the lamp 112 and the plurality of thin-walled capillary tubes 108 as arranged in the apparatus 100 represented in Figures 8A-B.

In the arrangement represented in Figure 8C, the thin-walled capillary tubes 108 which are farthest from the lamp 112 (as indicated by line F) are preferably no more than substantially 40%, and more preferably no more than substantially 25%, farther from the lamp 112 than the distance between the lamp 112 and those thin-walled capillary tubes 108 which are closest to the lamp 112 (as indicated by line N). For example, the distance indicated by line N can be about 7.3 cm while the distance indicated by line F can be about 8.5 cm.

It will be appreciated that the arrangement of the thin-walled capillary tubes 108 can be other than that represented in the figures, for example, circular or semi-circular. Moreover, it will appreciated that the point from which to measure the distance between the heat producing element and the capillary tubes will vary as the type and size of the heat producing element varies. For example, the heat producing element may comprise a plurality of lamps or electric resistive elements which vary in shape and size. In some embodiments, it may also become important to consider the distance from the sample chamber wall the sample containers are positioned. In the illustrated example, the apertures 140 (see Figure 8A) function as a means for holding the sample containers but other structures performing equivalent functions can also be used in accordance with the present invention.

The apparatus 100 also cools the samples contained in the capillary tubes 108 very rapidly and uniformly. In order to cool the sample chamber 106, air from outside the housing 102 is drawn into the interior of the housing through a lower housing portal 114 by a fan 116 which is connected to a motor shaft 122 driven by a motor 119. Since rapid cooling of the sample chamber is desired, it is preferred that the combination of the motor 118 and the fan 116 be able to move sufficient volumes of air into the sample chamber 106 and then disperse that air inside the sample chamber 106, as will be explained shortly.

The use of air as the thermal transfer medium, in contrast to other gases and liquids, has the advantages of being inexpensive, readily available, easily mixed, and never making a mess. In the case of the described embodiments, the high surface area-to-volume ratio of the sample containing capillary tubes provides for rapid thermal transfer using air as the thermal transfer medium.

During cooling portions of the thermal cycle, the action of the fan 116 draws ambient temperature air into the housing 102. A vent door 128, articulating on hinge 129, is provided. The vent door 128 is automatically opened by way of a solenoid 132 so that the interior of the housing 102 is sealed off from the upper housing portal 130. In some embodiments, the solenoid 132 is preferably replaced by a stepper motor as is known in the art. The use of a stepper motor allows the vent door 128 to be accurately and incrementally opened and closed in accordance with the needs for heating and cooling the samples. Those skilled in the art will be able to derive an appropriate control mechanism for use with a stepper motor, for example an SC-149 stepper motor controller (available from Alpha Products) as known in the art, using the information set forth herein.

Due to the arrangement of the lower sample chamber portal 120 and the larger cross sectional area and position of the upper sample chamber portal 126, room temperature air is moved into the sample chamber 106 and is dispersed and mixed within the sample chamber 106 by a paddle 124 which is connected to the motor shaft 122. The paddle 124 should rotate at a relatively high rate, for example, fast enough to create air velocities of around preferably about 250, more preferably 500, and most preferably 1000 meters per minute within the sample chamber 106. with the paddle 124, which can be a single or a multivane paddle, rotating at a high speed, air is moved, or drawn, into the sample chamber 106 and vented out of the sample chamber 106 following the path indicated by the dashed line 136. The rotation of the paddle 124 also promotes mixing of the air entering the sample chamber 106 and ensures the most efficient transfer of thermal energy from the surfaces of the thin-walled capillary tubes 108 to the air passing through the sample chamber 106. It will be appreciated that structures other than those illustrated herein can perform equivalent functions.

As the solenoid 132 is actuated to open the vent door 128, all of the room temperature air moved into the sample chamber 106 is exhausted through a sample chamber upper portal 126 and then through the upper housing portal 130 carrying the heat from the sample chamber 106 to the surrounding atmosphere. The rapid mixing of the air that passes through, and is disbursed in, the sample chamber 106 results in rapid and uniform cooling of the samples.

Example 2

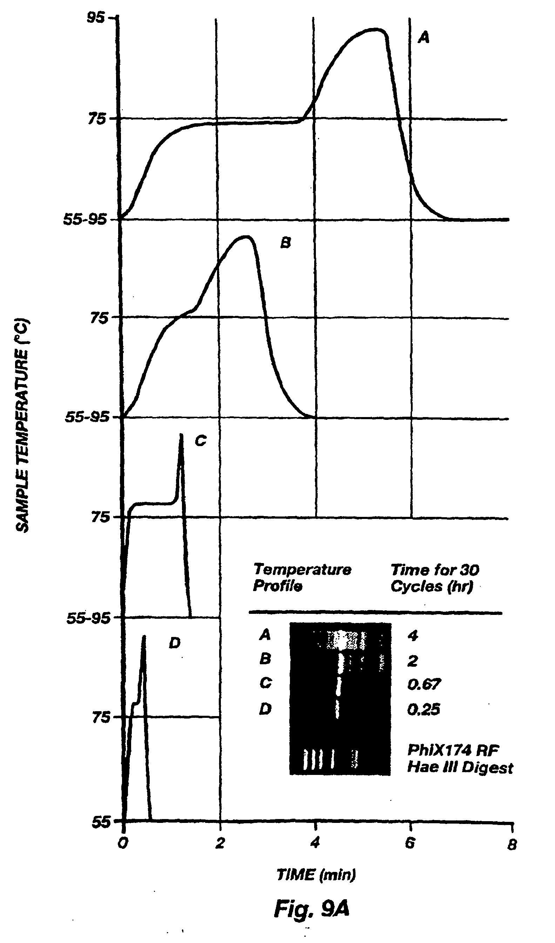

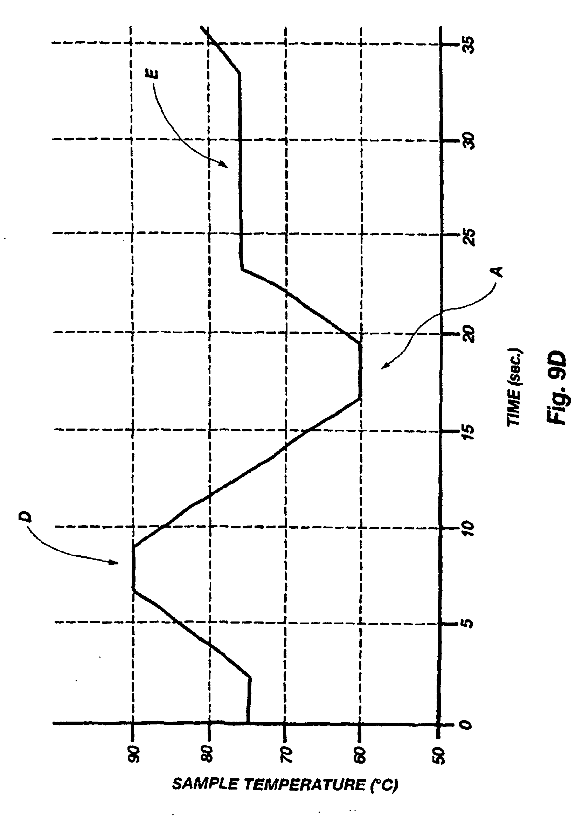

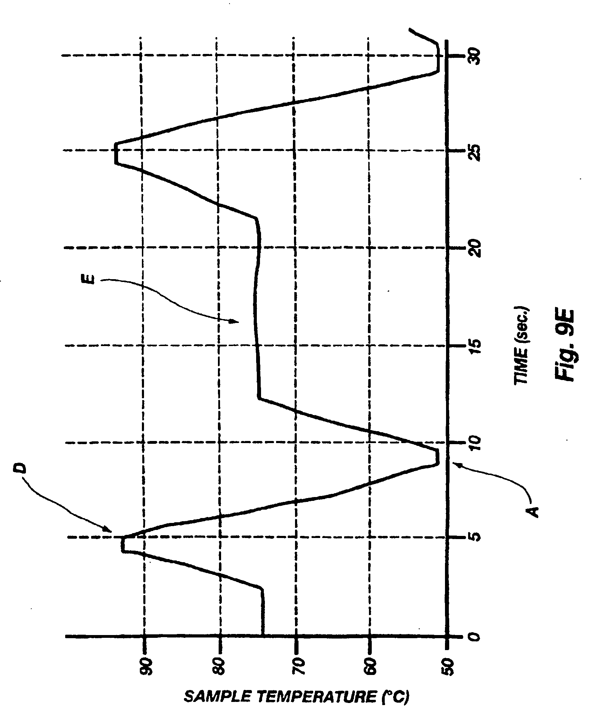

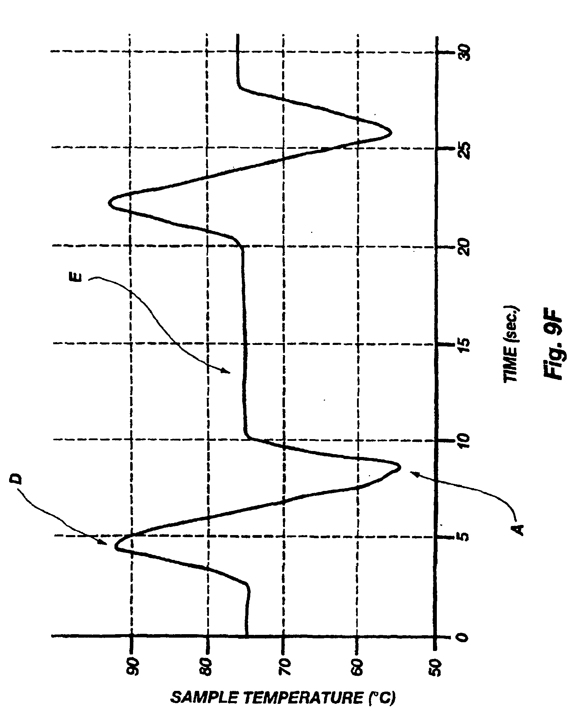

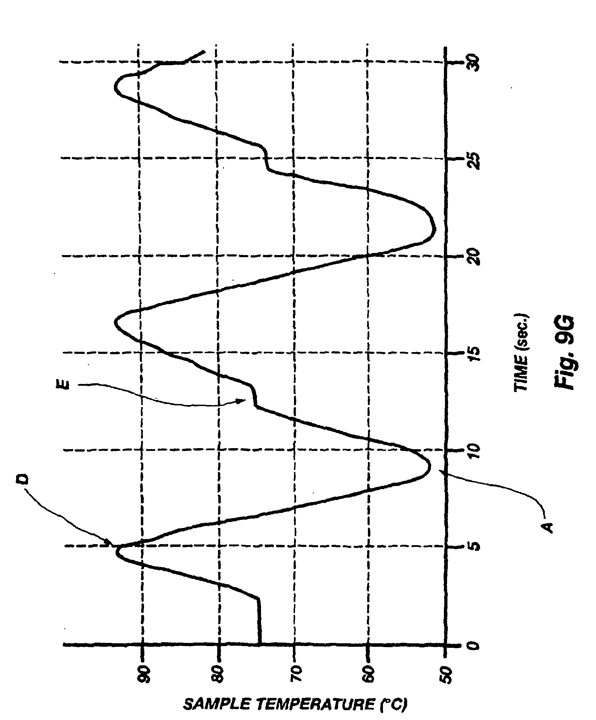

Figure 9A shows the results of four different temperature/time profiles (A-D) and their resultant amplification products after thirty cycles (A-D). The profiles A and B in Figure 9A were obtained using a prior art heating block device using the prior art microfuge tube. As can be seen in Figure 9A, the transitions between temperatures are slow and many nonspecific bands are present in profiles A and B. Profile B shows improvement in eliminating some of the nonspecific bands (in contrast to profile A) by limiting the time each sample remains at each temperature thus indicating that shorter times produce more desirable results.

Profiles C and D were obtained using the apparatus of Figures 8A-B. As can be seen in Figure 9A, amplification is specific and, desirably, even though yield is maximal in C (60 second elongation) it is still entirely adequate in D (10 seconds elongation).

The optimal times and temperatures for the amplification of a 536 bp fragment of β-globin from human genomic DNA were also determined. Amplification yield and product specificity were optimal when denaturation (93°C) and annealing (55°C) were less than 1 second. No advantage was found to longer denaturation or annealing times. The yield increased with longer elongation times at (77°C) but there was little change with elongation times longer than 10-20 seconds. These unexpected results indicate that the previously available devices used for DNA amplification are not maximizing the conditions needed to optimize the physical and enzymatic requirements of the reaction.

Further information can be obtained from: Wittwer, Carl T., Marshall, Bruce C., Reed, Gudrun B., and Cherry, Joshua L., "Rapid Cycle Allele-Specific Amplification with Cystic Fibrosis $\Delta F_{508}$ Locus," 39 Clinical Chemistry 804 (1993) and Wittwer, Carl T., Reed, Gudrun H., and Rire, Kirk M. , "Rapid DNA Amplification. "THE POLYMERASE CHAIN REACTION 174 (1994).

From the information provided in Figure 9A, it can be seen that the samples placed therein are subjected to rapid thermal cycling wherein the temperature of the sample is increased and decreased at a rate preferably at least as great as 0.5°C/second. In the case of the present invention carrying out the polymerase chain reaction, the temperature change is preferably carried out over an approximate range of between 30°C to 50°C. It is preferred that the thermal cycles be carried out quickly enough to complete at least thirty thermal cycles in forty minutes and more preferably complete thirty thermal cycles in twenty minutes and most preferably complete thirty thermal cycles in ten minutes.

The apparatus 100 more preferably increases and decreases the temperature of the sample at a rate at least as great as 1.0°C/second and even more preferably at a rate at least as great as 4.0°C/second and most preferably at a rate at least as great as 10.0°C/seeond. Critically, the biological sample, not just the surrounding medium and/or the sample container, must undergo the specified thermal change. The previously available devices, while having the drawback of not being able to perform thermal changes rapidly, also did not recognize the problem of changing the temperature of the sample, not just the temperature of the surrounding medium and container, rapidly and uniformly.

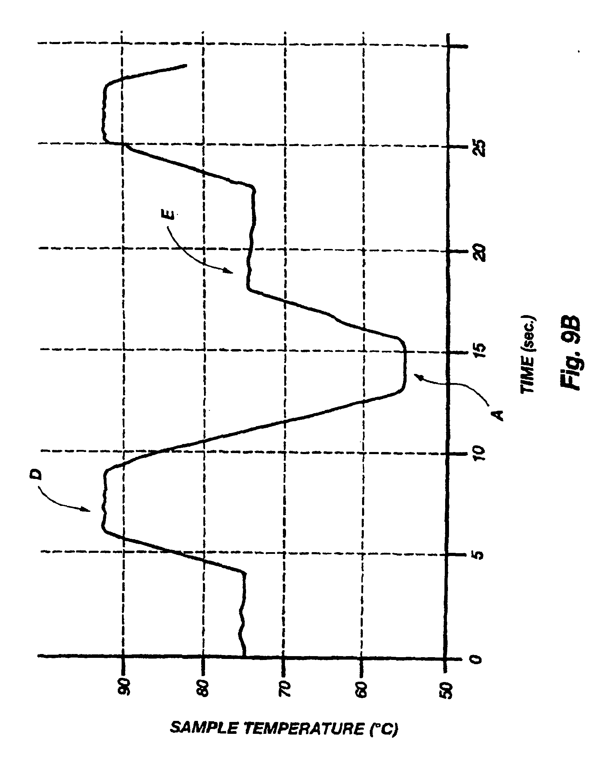

Referring now to the chart of Figure 9B, the method can desirably achieve thermal cycling preferably at a rate at least as great as 10°C/sec., and more preferably at a rate at least as great as 20°C/sec., over a temperature range of greater than about 20°C, more preferably over a temperature range of greater than about 30°C, and most preferably over a temperature range of about 40°C. Figure 9B shows the temperature in °C of the biological sample, not just the surrounding air or container, as the biological sample undergoes thermal cycling. Figure 9B shows a PCR sample beginning at about 74°C and being heated to a denaturation temperature, indicated at D, of about 92°C for 2 seconds. The sample is then cooled to an annealing temperature, indicated at A. of about 55 °C for two seconds. The transition between the denaturation temperature and the annealing temperature covers a range of 37°C in just under 4 seconds providing a rate at least as great as 10°C/sec. The sample is then warmed to an extension temperature of 74°C for five seconds as indicated at E in Figure 9B. The cycling of the sample through the denaturation temperature, the annealing temperature, and the extension temperature is repeated thirty times or as many times as desired.

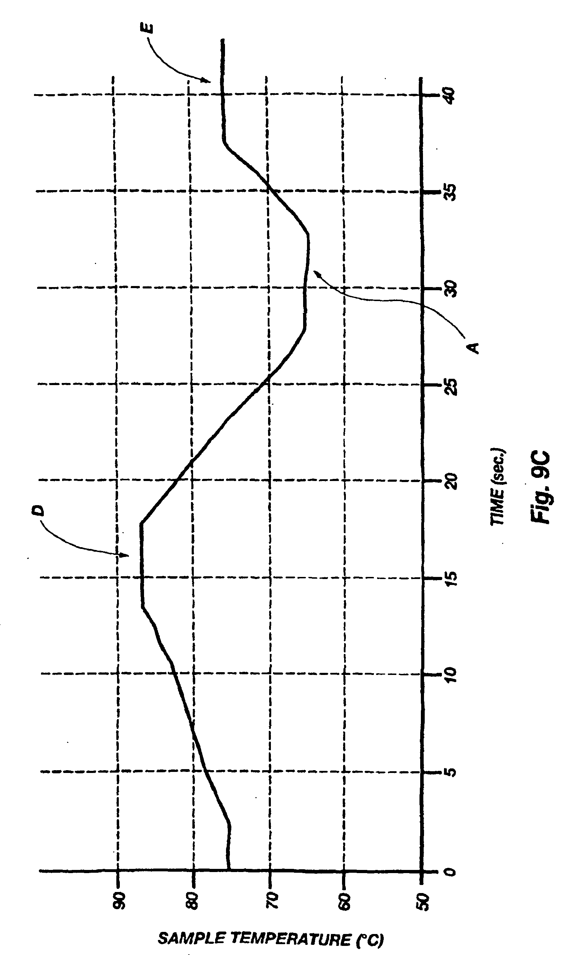

Figures 9C-G show exemplary cycles of other preferred temperature/ time profiles. It will be understood that those skilled in the art can alter the represented temperature/time profiles to carry out specific processes Those skilled in the art will also appreciate that the previously available devices and methods, such as devices which conduct heat to and from the sample via a solid or liquid, cannot provide the resulting temperature/time profiles described herein. Moreover, the previously available devices and methods do not suggest or teach the temperature/time profiles described herein. Furthermore, it will be appreciated that the previously available devices and methods utilizing air as the transfer medium, for example previously available chromatographic ovens, cannot provide, and do not suggest or teach, the temperature/time profiles which are described herein.

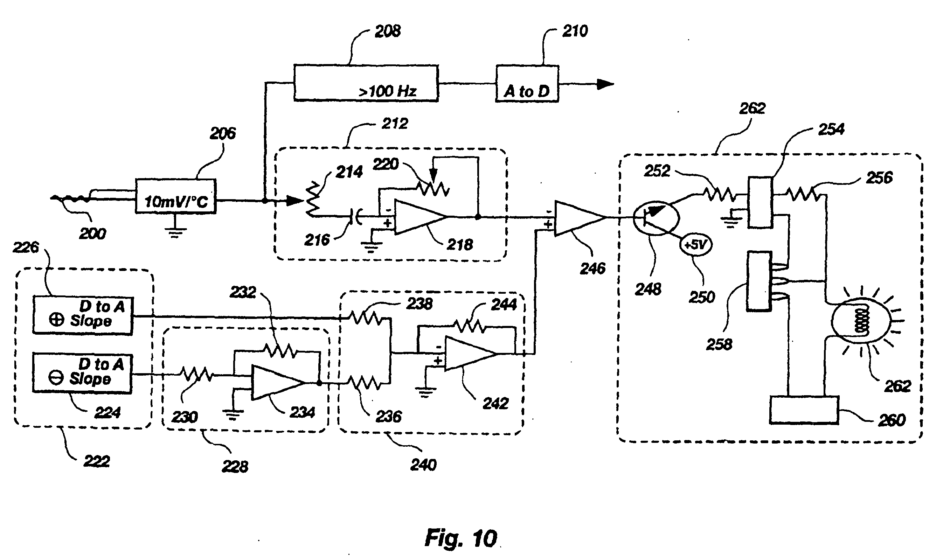

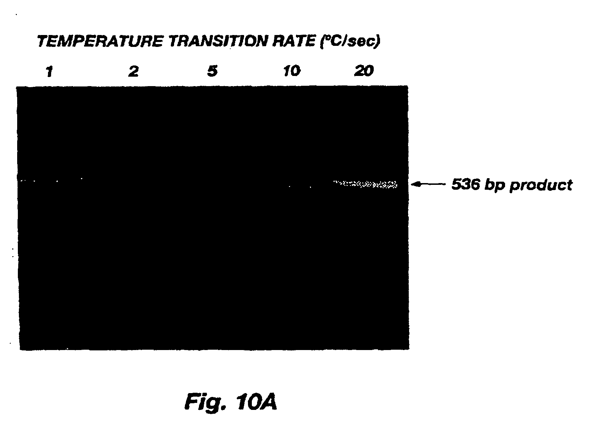

In order to provide the fastest thermal cycling time, it is preferred that the lamp (112 in Figures 8A and 8B) be rated at 2000 watts or a plurality of lamps be included which provide similar output. It is also preferred to include a temperature slope control circuit which is represented in Figure 10 in conjunction with an A-bus controller/acquisition system using an 8052 micro controller board with a clock and high level program interpreter available from Alpha Products (model no. SP-127) of Darian, Connecticut. Exemplary programming code used in connection with the described micro controller is included in the Programming Code Appendix A forming port of the description. The programming code provided in Appendix A is a BASIC52 file for serial downloading into the micro controller and provides exemplary temperature slope control during thermal cycling. Use of the 2000 watt heat producing device and the described control structures allows thermal cycling rates of 20°C/sec. to be desirably obtained.

The preferred arrangement for the temperature slope control circuit represented in Figure 10 will be explained with the understanding the additional necessary components not explicitly illustrated in Figure 10 can readily be supplied by those skilled in the art.

The temperature slope control circuit of Figure 10 includes a thermocouple 200 matched to the sample temperature response as explained earlier. The thermocouple 200 is connected to an integrated circuit 206, which preferably is one known in the art as an AD595, whose output is conveyed to a 4th order low pass filter 208 with a cutoff frequency of 100 Hz and to a 12 bit analog-to-digital convertor 210 whose output is used to provide a digital display of the temperature.

The output of the circuit 206 is also conveyed to a measured slope circuit 212. The measured slope circuit 212 preferably includes a 353 operational amplifier 218, a 100 KΩ potentiometer 214, a 1 MΩ potentiometer 230, and a 22 $\mu$F capacitor. The measured slope circuit 212 outputs a signal to the inverting input of a 353 operational amplifier 246.

A slope set circuit 222 includes a positive slope set digital-to-analog converter 226 and a negative slope set digital-to-analog converter 224. The digital-to-analog converters 224 and 226 are preferably 8-bit digital-to-analog converters referred to in the art as DA147. The slope set circuit can preferably receive instructions from another digital device (not illustrated in Figure 10) such as a personal computer. The output of the slope set circuit 228 is communicated to a summing circuit 240.

The summing circuit 240 preferably includes 100 KΩ resistors 236, 238, and 244 and a 353 operational amplifier 242. The output of the summing circuit 240 is conveyed to the non-inverting input of the operational amplifier 246 and represents the desired slope of the temperature change. The output of the operational amplifier 246 is provided to a transistor 248 contained within a power switching circuit 262.

The power switching circuit 262 includes a 5 VDC supply 250 providing current to the transistor 248. The transistor 248 has its emitter connected to a 3010 circuit 254 by way of resistor 252 which is preferably a 330 Ω resistor. The 3010 circuit 254 includes an output connected in series with a resistor 256 which preferably is a 180 Ω resistor. A triac 258 is preferably used to control the current delivered to a lamp 262, or other heat producing device, from a source of AC current 260.

The temperature slope control circuit represented in Figure 10, in cooperation with the other described system components, provides thermal cycling of biological samples as great as 20°C/sec over a temperature range of 30°C, and most preferably over a temperature range of 40°C, with homogeneity being maintained throughout the biological sample.

As indicated earlier, and in contrast to the teachings of the prior art, the polymerase chain reaction can be performed rapidly. Using the methods and apparatus described herein, the necessary number of temperature cycles can routinely be completed in much less time than possible with the prior art devices, for example in less than 15 minutes. By minimizing denaturation and annealing times, the specificity and yield of rapidly cycled amplifications are also improved to an extent not otherwise previously possible. Moreover, in addition to facilitating rapid heat transfer, the use of optically clear sample containers, such as clear capillary tubes, allows for continuous fluorescence monitoring of DNA amplification in accordance with the present invention.

Figure 10A shows graphically the effect of temperature transition rates on PCR reaction specificity and yield using a comparative apparatus. The results of Figure 10A were obtained using a 536 base pair fragment of the beta globin gene which was amplified from 50 ng of human genomic DNA with 50 mM Tris, pH 8.3, 2 mM MgCl$_2$, 50 $\mu$g/m$\ell$ bovine serum albumin, 0.5 $\mu$M each primer, 0.2 mM each dNTP, and 0.4 U native Taq DNA polymerase in a 10 $\mu\ell$ reaction. The human beta-globin primers RS42 and KM29 (536 base pairs) are described in C.T. Wittwer, G.C. Fillmore and D.R. Hillyard, "Automated Polymerase Chain Reaction in Capillary Tubes with Hot Air," Nucl. Acids. Res. 17: 4353-4357. Temperature cycling parameters were 94°C for 0 sec., 55°C for 0 sec., and 72°C for 10 sec. Thirty five cycles of amplification were performed with the indicated rates between all temperatures. The samples were electrophoresed on 1.5% agarose gels and stained with 0.5 $\mu$g/m$\ell$ ethidium bromide. Specificity and yield both decrease as the temperature transition rate decreases.

Fluorescent probes can be used to detect and monitor DNA amplification. As known to those skilled in the art, useful probes include double-stranded-DNA-specific dyes and sequence-specific probes. With the intercalater ethidium bromide, UV-excited red fluorescence increases after amplification. While microfuge tubes have been used as a sample container for DNA amplification, the methods and apparatuses described herein advantageously utilize sample containers with many of the characteristics of structures referred to herein as capillary tubes.

The use of the sample containers described herein allows detection of fluorescence while the sample is held within the container, as will be explained more fully hereinafter. Those skilled in the art will appreciate the number of different schemes of fluorescence detection of DNA amplification which are now available. For example, sequence-specific fluorescence detection is readily possible using the present invention and oligonucleotide hybridization probes. As another example, dual-labeled fluorescein/rhodamine probes can be cleaved during polymerase extension by 5'-exonuclease activity, separating the fluorophores and increasing the fluorescein/rhodamine fluorescence ratio.

Using the embodiments of the present invention described hereinafter, fluorescence can be measured after temperature cycling is complete, once per cycle as a monitor of product accumulation, two or more times during a temperature transition, or continuously within each cycle. In contrast to the present invention, previously available methods only cycle relatively slowly and do not teach acquisition and analysis of fluorescence during temperature changes.

The present invention allows cycle-by-cycle monitoring for quantification of initial template copy number. To carry out such cycle-by-cycle monitoring, fluorescence is acquired during the extension or combined annealing/extension phase of each cycle and related to product concentration. For example, a quantitative assay for hepatitis C RNA using the intercalater YO-PRO-1™ is known in the art and can be used in accordance with the present invention. For more information see Ishiguro, T., J. Saitch. H. Yawata, H. Yamagishi, S. Iwasaki, and Y. Mitoma, 1995, "Homogeneous quantitative assay of hepatitis C virus RNA by polymerase chain reaction in the presence of a fluorescent intercalater," Anal. Biochem, 229:207-213. Prior to the present invention, continuous fluorescence monitoring within each cycle during temperature transitions has not been attempted.

A rapid temperature cycler integrated with 2-color fluorescence optics to provide continuous fluorescence monitoring may be used. As will be more fully discussed below, different preferred fluorescence techniques for monitoring DNA amplification are provided herein as specific examples of carrying out this aspect. Those skilled in the art will be familiar with the use of ethidium bromide in fluorescence techniques. In one example described below, it is preferred that SYBR® Green I, which is well known in the art and available from Molecular Probes of Eugene, Oregon, be used as a double-strand-specific dye.

In one example, time, temperature, and fluorescence is acquired every 200 msec. during the amplification reaction. By acquiring data regularly during the reaction, the acquisition of such data reveals fine details of product denaturation, reannealing, and extension which is not available in the previously available apparatus and methods.

As will be appreciated by those skilled in the art, once-per-cycle monitoring of multiple samples undergoing DNA amplification is a powerful quantitative tool. Importantly, as will be appreciated by an understanding of this disclosure, continuous monitoring within a cycle can identify the nature of probe fluorescence, provide insight into DNA amplification mechanics not previously available in the art, and assess PCR product and probe melting curves to identify amplification products and mutations.

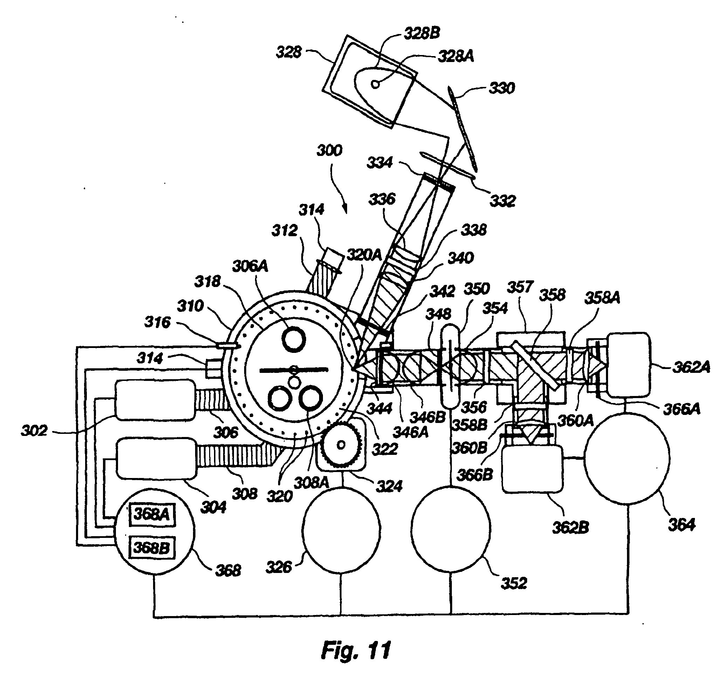

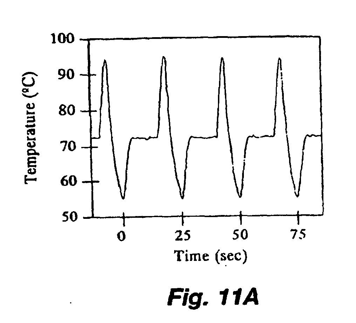

Referring now to Figure 11, a schematic view of a preferred rapid temperature cycler with fluorescence detection is provided, generally designated at 300. A forced air hot air source 302 is preferably provided. The forced air hot air source 302 is preferably a commercially available device including a 1600 watt heating coil and fan. A cool forced air cool air source 304 is also preferably provided. The cool forced air source 304 is preferably a 2200 rpm shaded pole blower available in the art from Dayton of Niles, Illinois, model no. 4C006B. It is preferred that the cool air source 304 provide ambient temperature air, but it is within the scope of the present invention to utilize a means for providing fluid that is at a temperature lower than ambient air temperature.

In the embodiment of Figure 11, ducts 306 and 308 connect the forced hot air source 302 and the forced cool air source 304, respectively, to a sample chamber 310. The ducts 306 and 30B are preferably corrugated black nylon tubing having a 2.5 cm diameter. The duct 306 is connected to the sample chamber 310 via a port 306A and the duct 308 is connected to the sample chamber 310 via a port 308A. A vent 312 and an exhaust fan 314 function to move air out of the sample chamber 310. Moreover, a means for shielding the interior of the sample chamber 310 from ambient light is integral with the sample chamber 310.

The temperature of the samples within the sample chamber 310 is preferably monitored by a tubular, metal-sheathed thermocouple 316, available from Idaho Technology of Idaho Falls, Idaho, model no. 1844, which is matched in thermal response to the samples held in the capillary tubes. Importantly, temperature homogeneity within the sample chamber 310 is achieved by mixing the air within the sample chamber 310. It is preferred that such mixing of the air within the sample camber 310 be carried out by a central sample chamber fan 318. The sample chamber fan preferably includes a 1.7 X 11 cm fan blade available from Idaho Technology, model no. 1862, and a motor available from Idaho Technology, model no. 1861, which creates air velocities of at least 800 to 1000 meters per minute within the sample chamber 310. Such rapid air velocities may not be needed in all applications of the present invention but rapid air velocities promote extensive mixing and temperature homogeneity within the sample chamber 310.

Within the sample chamber 310, a plurality of samples are held in capillary tubes, some of which are indicted at 320, and are placed in a vertical orientation on a rotatable carousel 322. The carousel 322 is preferably fourteen centimeters in diameter and rotated by a 400 step per revolution stepper motor 324 controlled by a micro stepping drive module 326. The stepper motor 324 is preferably one available from New England Affiliated Technologies of Lawrence, Massachusetts, model no. 2198364, and the micro stepping drive module 326 is preferably one also available from New England Affiliated Technologies, model no. MDM7 micro stepping drive module, which provides 12,800 steps per rotation of the carousel 322.

Still referring to Figure 11, a fluorescence excitation source 328 is provided. One preferred arrangement for the excitation path will now be described with one preferred arrangement for the collection path in accordance with the present invention will subsequently be described. The fluorescence excitation source 328 preferably includes a 75 watt xenon arc source 328A focused with an elliptical reflector 328B. The xenon arc source 328A is preferably available from Photon Technology International of South Brunswick, New Jersey, model no. A1010, with f/2.5 elliptical reflector 328B. The power supply and other components needed to operate the fluorescence excitation source 328 are well known to those skilled in the art. Alternatively, a light emitting diode can be used as a fluorescence excitation source. Those skilled in the art will appreciate that many different excitation sources can be used within the scope of the present invention.

The radiation emitted by the fluorescence excitation source 328 is focused to about 2 mm using an adjustable iris 334 such as one available in the industry from Rolyn (Covina, California), model no. 75.0125. The light emitted from the fluorescence excitation source 328 impinges upon a cold mirror 330, which is preferably available from Rolyn, model no. 60.4400, and passes through heat absorbing glass 332, which is preferably one available from Rolyn, model no. 65.3130. After collimation through a planoconvex lens 336, preferably one available from Rolyn, model no. 10.0260, a 450-490 nm bandpass interference filter 338, preferably one available from Omega Optical of Brattleboro, Vermont, model no. 470RDF40, a focusing planoconvex lens 340, preferably available from Rolyn, model no. 10.0260, and a 1 mm silica window 342, preferably available from Omega, to prevent condensation on the just described optical components during temperature cycling. Using the described excitation path, a 5-7 mm section of one capillary sample tube 320A is illuminated.

Still referring to Figure 11, the collection path for collecting the fluorescence emitted from the sample 320A will be described next. The optics of the collection path include a 1 mm silica window 344 which is placed in the optical path to prevent condensation on the other optical components. Two opposed aspheric lenses 346A&B, preferably available from Rolyn, model no. 17.1175, function to focus emitted fluorescence onto a 2 x 10 mm slit 348. The slit 348 can preferably be fabricated from cutting exposed X-ray film and the slit 348 functions as a spatial filter. After the slit 348 (acting as a spatial filter), the emitted fluorescence is imposed upon a 35 mm electronic shutter 350 operated via an electronic shutter control 352. The 35 mm electronic shutter 350 is preferably a Uniblitz shutter model no. VS35 and the electronic shutter control 352 is preferably driver model no. D122, both available from Vincent Associates of Rochester, New York. A collimating aspheric lens 354, preferably one available from Rolyn model no. 17.1175, is also provided.

A filter 356 is also included when detection of SYBR® Green I emissions is desired. The filter 356 is preferably a 520-580 nm band pass filter, available from Omega as model no. 550RDF60, which is preferably used for single wavelength acquisition. For detection of other emissions, for example, a combination of a dichroic filter 358 and wavelength filters 358A and 358B can be used. For example, for separation of fluorescein and rhodamine emissions, the dichroic filter 358 preferably consists of a 560 nm dichroic filter, preferably available from Omega, model no. 560 DRLP, and a 520-550 nm band pass filter (358A), preferably available from Omega, model no. 535DF30, for detection of fluorescein, and a 580-620 nm band pass filter (358B), preferably available from Omega, model no. 600DF40, for detection of rhodamine. For separation of fluorescein and Cy5 emissions, the dichroic filter 358 preferably is a 590 nm dichroic filter, available from Omega, model no. 590 DRLP, and filters 358A&B preferably consist of a 520-550 nm band pass filter (358A), available from Omega, model no. 535DF30, for detection of fluorescein, and a 660-680 nm band pass filter (358B), available from Omega, model no. 670DF20, for Cy5 detection. Those skilled in the art will readily appreciate that the use of other components can be readily implemented using the information set forth herein in order to accommodate other flourescent wavelengths.

Still referring to Figure 11, after being subjected to the respective filter 358A or 358B, the emitted fluorescence is focused through two planoconvex lenses 360A & 360B, each preferably available from Edmund of Barrington, New Jersey, model no. 32970, and onto photomultiplier tubes 362A and 362B, respectively. The photomultiplier tubes ("PMT") 362A and 362B are preferably available from Hamamatsu of Middlesex, New Jersey, model no. R928, and are each enclosed in a suitable housing including appropriate circuitry, preferably one available from Photon Technology International, model no. 714, with analog acquisition capabilities. A PMT and data acquisition control module 364 is also preferably provided. Manual PMT shutters 366A and 366B, as known in the art, are also provided.

The forgoing described optical components are preferably five centimeters in diameter and mounted in five centimeter universal lens mounts, such as those available from Rolyn, model no. 90.0190. As can be carried out by those skilled in the art, many of the necessary structural components were machined from black Delrin™ using techniques known in the industry.

Those skilled in the art will appreciate that the rapid temperature cycler with fluorescence detection 300 can advantageously be constructed using light emitting diodes (LEDs) and photodiodes in place of similarly functioning components represented in Figure 11. Thus, the function of the fluorescence excitation source 328 can be carried out by light emitting diodes. The photomultiplier tubes 362A&B can also be replaced with photodiodes. Additional information regarding suitable light emitting diodes and photodiodes will be provided later herein. It will be appreciated that technique sensitivity is limited by background fluorescence, most of which comes from the probes, not the detection system. Significantly, stability is generally more important than absolute sensitivity.

Those versed in the art will appreciate that the rapid temperature cycler with fluorescence detection 300 represented in Figure 11 includes the beneficial characteristics of a fluorimetry device with rapid temperature control, a combination nowhere suggested or taught in the art. PCR can be performed and analyzed during ten to twenty minutes of temperature cycling. The present invention's combination of 1) fluorescence monitoring within each temperature cycle and 2) analysis of the temperature and time dependence of hybridization provides advantages not otherwise obtainable.

The present invention also makes possible single-color fluorescence methods to monitor product purity and quantify template during PCR. Dyes that monitor DNA strand status are added to PCR reactions for observation during temperature cycling using embodiments of the present invention.

In order to explain some of the benefits which accrue with the present invention, specific examples using the apparatus represented in Figure 11 will now be provided. DNA amplification was performed in 50 mM Tris, pH 8.3 (25°C), 3 mM $MgCl_2$, 500 $\mu$g/ml bovine serum albumin, 0.5 $\mu$M of each primer, 0.2 mM of each deoxynucleoside triphosphate and 0.2 U of Taq polymerase per 5 $\mu\ell$ sample unless otherwise stated in the following examples. Also in the following examples, human genomic DNA (denatured for 1 min by boiling) or purified amplification product was used as DNA template. Purified amplification product was obtained by phenol/chloroform extraction and ethanol precipitation (see D.M. Wallace 1987, Large- and small-scale phenol extractions and precipitation of nucleic acids (as described at p. 33-48, in S.L. Berger and A.R. Kimmel (Eds.), Guide to Molecular Cloning Techniques (Methods in Enzymology, Vol. 152) Academic Press, Orlando), followed by removal of primers by repeated washing through a Centricon 30 micro concentrator (available from Amicon of Danvers, Massachusetts). Template concentrations were determined by absorbence at 260 nm. $A_{260}/A_{280}$ ratios of templates were greater than 1.7.

In these examples, primers were synthesized by standard phosphoramidite chemistry, as known in the art, namely, using Pharmacia Biotech Gene Assembler Plus (Piscataway, New Jersey). The 180 base pair fragment of the hepatitis B surface antigen gene was amplified using primers 5'-CGTGGTGGACTTCTCTCAAT-3' (SEQ ID NO: 1), and 5'-AGAAGATGAGGCATAGCAGC-3' (SEQ ID NO:2) (Genbank sequence HVHEPB). SYBR® Green I dye was obtained from Molecular Probes (Eugene, Oregon). The β-actin primers and fluorescein/rhodamine dual probe were obtained from Perkin Elmer (Foster City, California) (no. N808-0230,). The human β-globin primers RS42/KM29 (536 base pairs) and PC03/PC04 (110 base pairs) are described in C.T. Wittwer, G.C. Fillmore and D.R. Hillyard, "Automated Polymerase Chain Reaction in Capillary Tubes with Hot Air," <u>Nucl. Acids. Res.</u> 17:4353-4357. The single labeled probes:

```
5'-CAAACAGACACCATGGTGCACCTGACTCCTGAGGA-fluorescein-3'

                    (SEQ ID NO:3) and
```

```
5'-Cy5-AAGTCTGCCGTTACTGCCCTGTGGGGCAAG-phosphate-3'

                    (SEQ ID NO:4)
```

were synthesized using a fluorescein phosphoramidite (available from Glen Research of Sterling, Virginia, no. 10-1963) a Cy5™ phosphoramidite (available from Pharmacia no. 27-1801-02), and a chemical phosphorylation reagent (available from Glen Research no. 10-1900). These adjacent probes hybridize internal to the PCO3/PCO4 β-globin primer pair on the same DNA strand and are separated by one base pair. Probes were purified by reverse phase C-18 high pressure liquid chromatography and homogeneity checked by polyacrylamide electrophoresis and absorbance ($A_{260}$ and the absorbance maximum of the fluorophore). Hybridization probes (β-actin and β-globin) were used at 0.2 $\mu$M each.

In the pertinent examples described herein, amplification samples of 5 $\mu\ell$ were loaded into capillary sample tubes, some of which are represented in Figure 11 at 320. The preferred capillary sample tubes are those available from Idaho Technology, model no. 1705, having dimensions of 1.02 mm O.D. and 0.56 mm I.D. Once loaded, the capillary sample tubes were sealed with a butane flame. The surface of the capillary sample tube was cleaned with optical grade methanol before it was loaded into the carousel 322 of the rapid temperature cycler with fluorescence detection 300.

Control of the components represented in Figure 11 was achieved by use of a graphical programming language known as LabView (available from National Instruments, Austin, Texas) and a 12-bit multifunction input/output card 368A (available from National Instruments under the designation AT-MIO-E2) in a PC compatible computer 368 utilizing an Intel® 80486 microprocessor running at a clock speed of 120 MHZ. Analog output channels on the input/output card 368A were used to control the sensitivity, i.e, the PMT voltage, of each of the photomultiplier tubes 362A&B. Analog input channels on the input/output card 368A receive the signals from each of the photomultiplier tubes 362A&B. The PC compatible computer 368, through the input/output card 368A, controls the position, rate and direction of movement of the carousel 322. For example, when multiple capillary sample tubes are loaded, the carousel 322 rapidly positions each capillary sample tube 320 sequentially at a monitoring location (the location represented by capillary sample tube 320A) for a 10 - 100 msec acquisition period. For continuous monitoring of a single capillary sample tube, the capillary sample tube is held in the monitoring position while data is preferably acquired every 200 msec. and is averaged in accordance with well-known techniques. Time, temperature, and preferably two channels of fluorescence are continuously displayed via a monitor 368B associated with the computer 368 as fluorescence vs. cycle number and fluorescence vs. temperature plots.

The carousel 322 should be positioned where maximal fluorescence and signals are acquired. When a single capillary sample tube, such as the capillary sample tube 320A, is monitored the signals are acquired every 200 msec with an integrating time constant set on the photomultiplier tube 362A or 362B. or both, at 50 msec. For multiple sample tubes, the time constant is set at 0.5 msec and the carousel is rotated once to locate the precise position where each capillary sample tube 320 provides the maximum fluorescence in each of the two channels. After positioning the capillary sample tube 320A at a location where maximum fluorescence is obtained, the sensitivity of each PMT 362A&B is adjusted and the carousel rotated again to count and locate the position of all the capillary sample tubes 320 in the carousel 322. When only a signal fluorescence acquisition is desired once each amplification cycle during extension, each capillary sample tube 320 is sequentially positioned on the carousel 322 at the monitoring position for 100 msec. Continous acquisition for multiple tubes can also be obtained by continuously rotating the carousel 322. Temperature control programming was based upon, and modified from, a commercial rapid temperature cycler available from Idaho Technology under the trademark Rapidcycler™ using an 8051 cross compiler available from Systronics, Salt Lake City, Utah, designated BCI51 and Dallas development system (also available from Systronics under the designation DPB2).

In practice, the temperature response of the rapid temperature cycler with fluorescence detection 300 is similar to the response obtained with the apparatus disclosed in Figures 8A&B allowing 20-30 second cycles (30 cycles in 10-15 min) as represented in the temperature vs. time chart of Figure 11A (which shows a few cycles of one preferred temperature profile). When a double strand-specific fluorescent dye is present during amplification, fluorescence generally increases as more double stranded product is made. See R. Higuchi, G. Dollinger, P.S. Walsh, and R. Griffith, 1992, "Simultaneous Amplification and Detection of Specific DNA Sequences," Bio/Technology 10:413-417.

Moreover, it will also be appreciated that double strand specific dyes such as ethidium bromide or SYBR® Green I can be used as generic indicators of amplification. SYBR® Green I dye is preferred over ethidium bromide in many applications because it has an excitation maximum near fluorescein and often provides a stronger signal with DNA than visible excitation of ethidium bromide.

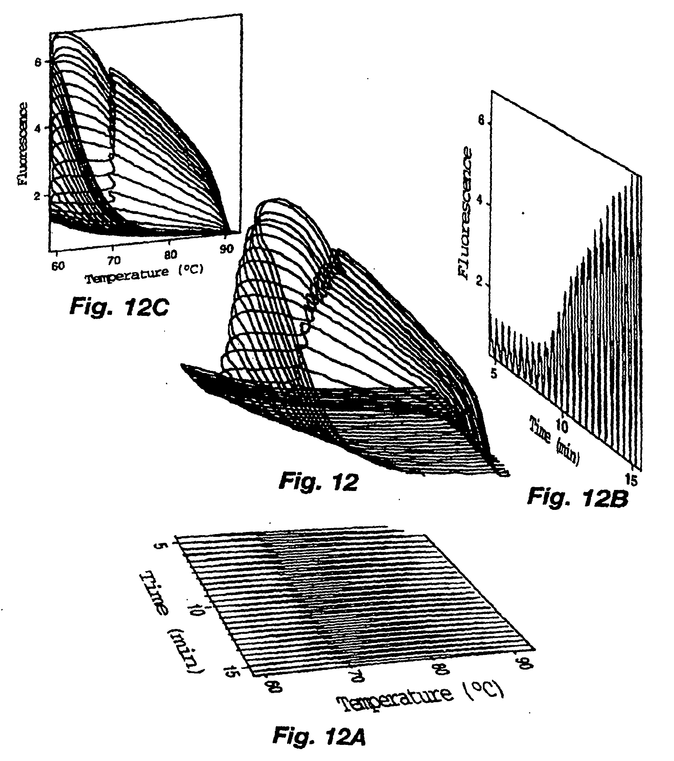

Fluorescence also depends on temperature, a confounding effect during temperature cycling that is usually eliminated by considering fluorescence once per cycle at a constant extension temperature. However, if temperature, time, and fluorescence are acquired every 200 msec during rapid cycle amplification, a three dimensional spiral is shown on the monitor 368B as represented in Figure 12. The three dimensional plot represented in Figure 12 is also projected in Figure 12A as a two dimensional plot of temperature vs. time, projected in Figure 12B as a two dimensional plot of fluorescence vs. time, and projected in Figure 12C as fluorescence vs. temperature. The temperature vs. time projection of Figure 12A repeats each cycle and provides essentially the same information as set forth in Figure 11A. Because fluorescence varies inversely with temperature, the fluorescence vs. time projection shown in Figure 12B at early cycles is a scaled mirror image of the temperature vs. time plot. As product accumulates, the fluorescence increases at all temperatures where double stranded product is present. However at denaturation temperatures, fluorescence returns to baseline since only single stranded DNA is present.

The fluorescence vs. temperature projection of double stranded dyes shown in Figure 12C eliminates the time axis and shows the temperature dependence of strand status during DNA amplification. The fluorescence vs. temperature projection shown in Figure 12C is for a 180 base pair fragment of hepatitis B virus DNA.

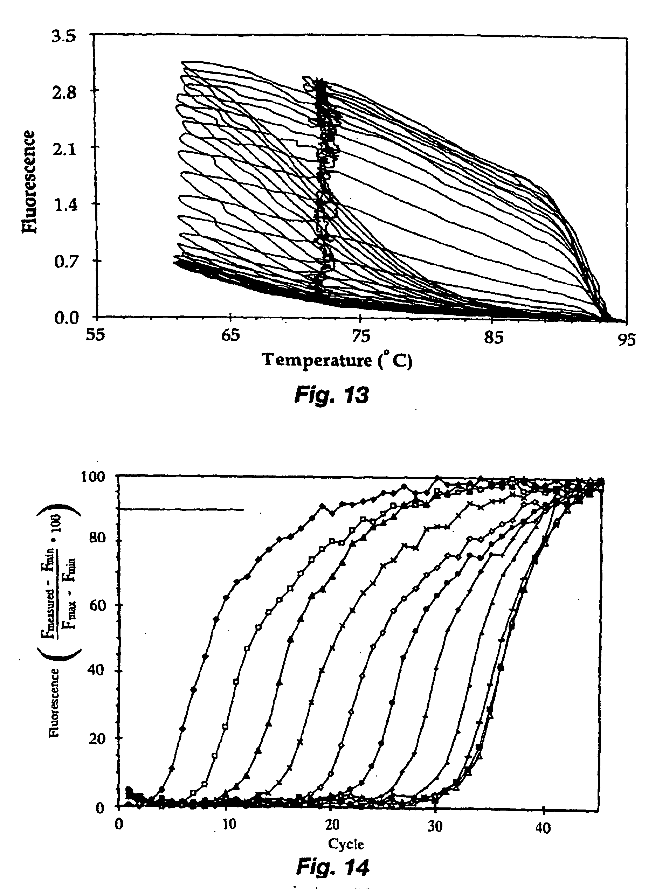

Another fluorescence vs. temperature projection is shown in Figure 13. The projection represented in Figure 13 is for a 536 base pair fragment of human β-globin DNA. Early cycles represented in Figure 13 appear identical, with a nonlinear increase in fluorescence at lower temperatures. As amplification proceeds, later cycles appear as rising loops between annealing and denaturation temperatures that show significant hysteresis. That is, the observed fluorescence during heating is greater than that during cooling. As the sample is heated, fluorescence is high until denaturation occurs (apparent as a sharp drop in fluorescence). As can be seen in Figure 13, as the sample cools from denaturation to annealing temperatures, double strand signal increases rapidly. Also as can be seen in Figure 13, the fluorescence continues to increase during extension while the temperature is held constant.

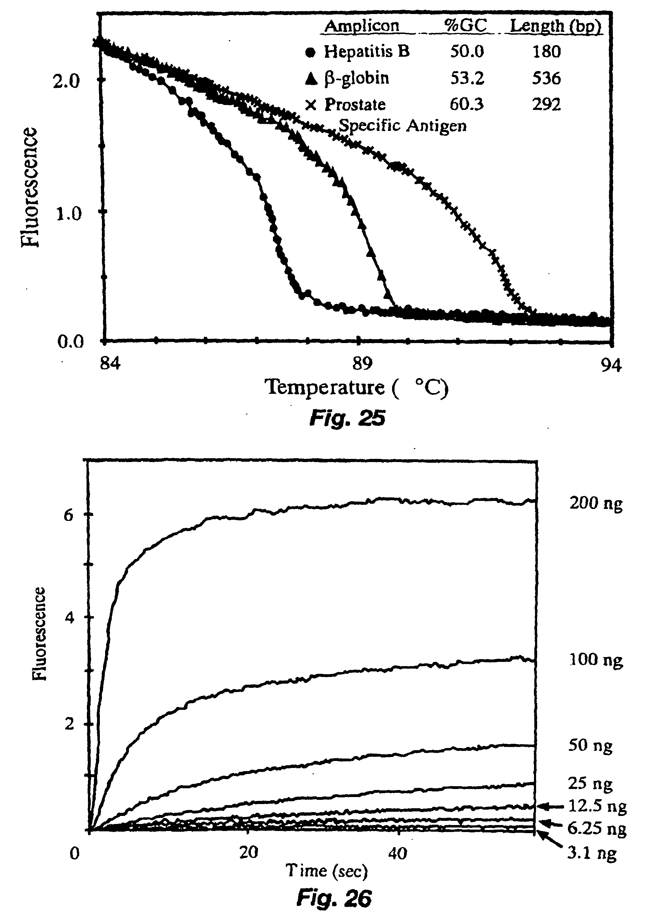

Double strand specific dyes can also be used in accordance with various aspects of the present invention. The strand status of PCR products can be followed with dyes that fluoresce in the presence of dsDNA. When SYBR® Green I is present during amplification, fluorescence increases as more dsDNA is made. However, temperature cycling introduces a confounding effect because fluorescence is inversely proportional to temperature as shown in Figures 26A and 26B. As product accumulates, the fluorescence increases except at denaturation temperatures, where the fluorescence returns to baseline as shown in Figure 12C.

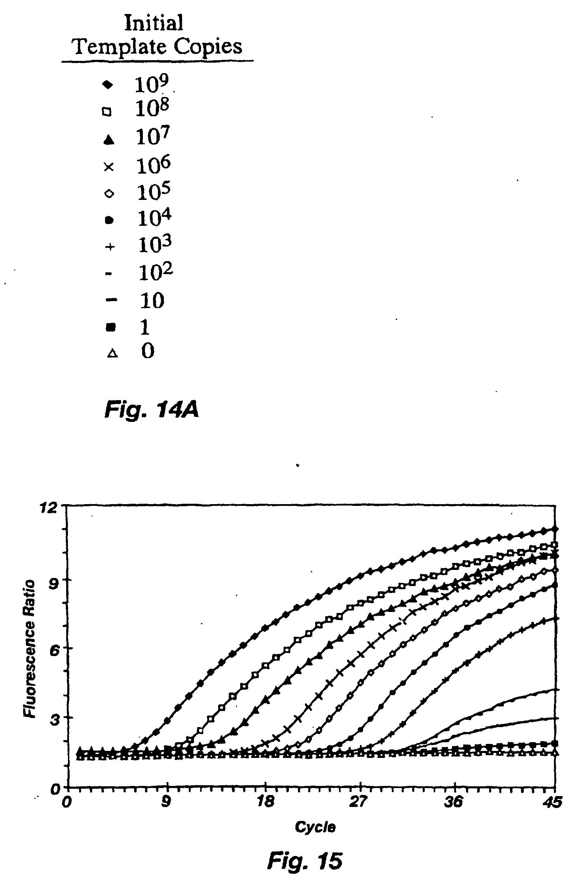

When multiple samples are monitored, using the rapid temperature cycler with fluorescence detection 300, once each cycle with SYBR® Green I, a $10^7$- $10^8$ range of initial template concentration can be discerned as represented in Figure 14. Figure 14A provides a legend for the indicia provided on the different plots in Figure 14, and subsequent figures, for different initial template copy number. When the data are normalized as the percent maximal fluorescence of each capillary sample tube 320, one hundred initial copies are clearly separated from ten copies. However, the difference between one and ten copies is marginal, and no difference is observed between zero and one average copies per capillary sample tube 320.

Double strand dyes depend on the specificity inherent in the amplification primers. As will be appreciated by those skilled in the art, nonspecific amplification at high cycle numbers can limit detection sensitivity to about one hundred initial template copies (see Figure 14). With rapid cycling taught herein, further improvements in amplification specificity are obtained further improving the overall DNA amplification performance.

Quantitifcation with sequence-specific probes has a similar dynamic range as double stranded DNA dyes but, as shown in the plots of Figures 15A and 15B, appear to discriminate even a single initial template copy from negative controls.

When low copy number detection and quantification are needed, additional specificity is provided by fluorescent probes that require hybridization for signal generation. Cleavage of a dual-labeled exonuclease probe is one technique which is capable of distinguishing a single template copy from a negative control as shown by Figure 15. Figure 15 show plots of fluorescence ratio vs. cycle number for different initial template copy number, according to the legend provided in Figure 14A.

Signal generation with 5'-exonuclease probes is dependent not only on DNA synthesis, but requires hybridization and hydrolysis between the fluorophores of the dual-labeled probe. This hydrolysis reduces quenching and the fluorescence ratio of fluorescein to rhodamine emission increases. For more information on this technique, see L.G. Lee, C.R. Connell and W. Bloch, 1993, "Allelic Discrimination by Nick-translation PCR with Fluorogenic Probes," Nucl. Acids Res. 21:3761-3766 & Livak, K.J., S.J.A. Flood, J. Marmaro, W. Giusti and K. Deetz, 1995, "Oligonucleotides with Fluorescent Dyes at Opposite Ends Provide a Quenched Probe System Useful for Detecting PCR Product and Nucleic Acid Hybridization," PCR Meth. Appl. 4:357-362).

Figure 25 shows fluorescence PCR results from a probe with five intervening bases between fluorescein and rhodamine labels. The forty-five cycle amplification was completed in 20 minutes using the rapid temperature cycler with fluorescence detection 300 of Figure 11. By monitoring the fluorescence ratio once per cycle, a 10' fold range of initial template concentration could be distinguished. The amplification curves are shifted approximately 3-4 cycles for each 10-fold change in initial template concentration.

Although the final fluorescence signal is decreased when low copy numbers are amplified (presumably because of decreased amplification efficiency), quantification between zero and one hundred copies is readily possible. The signal generated by exonuclease probes is cumulative and only indirectly related to product concentration. Hence, the fluorescence signal continues to increase even after the amount of product has reached a plateau. Using the information contained herein, those skilled in the art can formulate appropriate standards to control for efficiency of amplification and cleavage in order to carry out absolute quantification.

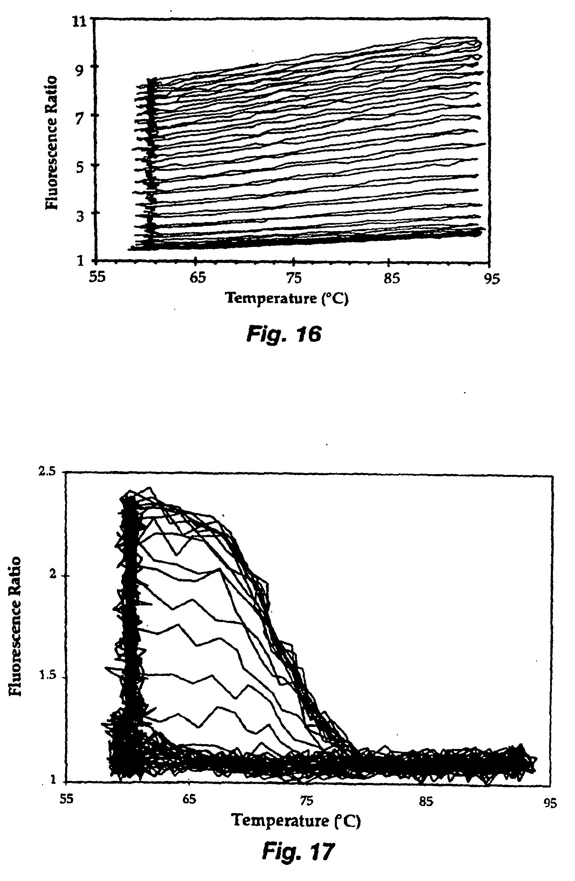

Fluorescence vs. temperature plots of 5'-exonuclease probes confirm that probe hydrolysis is the mechanism of signal generation. In Figure 16, a fluorescence vs. temperature plot of two-temperature cycling is shown with the β-actin exonuclease probe. In each cycle the fluorescence ratio varies linearly with temperature and there is little, if any, hysteresis. The signal increases each cycle during the annealing/extension phase when probe hydrolysis occurs. Although the fluorescence of both fluorescein and rhodamine decreases with increasing temperature (data not shown in the figures), the rate of change is greater for rhodamine, resulting in an increasing ratio with increasing temperature. No temperature-dependent hybridization effects are apparent with the 5'-exonuclease probe.

In contrast, when the fluorescence signal is dependent only on hybridization, fluorescence ratio vs. temperature plots show a different pattern with hysteresis during two-temperature cycling, as plotted in Figure 17. The plots in Figure 17 represent the results obtained using two adjacent hybridization probes which are present, an upstream probe labeled 3' with fluorescein and a downstream probe labeled 5' with Cy5™. The probes are separated by a 1 base pair gap. During the annealing/extension phase of the reaction, the probes hybridize resulting in accumulating product and the Cy5™ to fluorescein fluorescence ratio increasing. During heating to product denaturation temperatures, the probes dissociate between 65°C and 75°C, returning the fluorescence ratio to background levels. The change in fluorescence ratio during hybridization is largely due to an increase in Cy5™ fluorescence from resonance energy transfer. The temperature dependence of hybridization can be used to detect mutations by a shift in the melting curve. Adjacent hybridization probes are also very useful for quantification, as shown in Figure 15B.

From the foregoing discussion, it will be appreciated that fluorescence monitoring during DNA amplification is an extraordinarily powerful analytical technique. Using the rapid temperature cycler with fluorescence detection 300, productive and cost efficient real time monitoring, sequence-specific detection, and quantification can be achieved in five to twenty minutes, depending on the number of initial template copies present.

Furthermore, the system and results represented in Figures 11-17 is particularly suited for continuous monitoring of a biological reaction using fluorescent dyes. For example, with precise temperature control and double-strand-specific dyes, product purity can be estimated by melting curves. With rapid temperature control provided by the present invention, absolute product concentration can be determined by reannealing kinetics. The present invention advantageously provides rapid temperature changes and strict intra-sample temperature homogeneity which is not available in the prior art. In contrast to the prior art, the present invention utilizes sample containers with a high surface area to volume ratio, (for example by using the preferred capillary sample tubes 320 in Figure 11) and uses air as the thermal transfer medium providing rapid control of sample temperature not otherwise obtainable. For example, sample temperature vs. time plots obtained when processing samples in the sample containers of the present invention show sharp spikes at denaturation and annealing temperatures (showing rapid temperature response) in contrast to the prior art conical plastic tubes which require several seconds for all of the sample to reach thermal equilibrium. Moreover, the sample containers of the present invention provide improved results over using etched silicon or glass chips as sample containers since the thermal cycle times and thermal homogeneity of the present invention are superior than the thermal cycle times and thermal homogeneity possible using such other structures.

Using the present invention, many aspects of DNA amplification which have heretofore been little understood are discernable. For example, product denaturation occurs in less than one second, yet the prior art calls for ten seconds to one minute of denaturation. Observing product melting by real time fluorescence monitoring with double strand dyes in accordance with the present invention (see Figures 12 and 13) shows that use of shorter denaturation times is very effective. As another example, many causes of the known "plateau effect" have been proposed, but few data are available to distinguish between alternatives. As shown in Figure 13, product reannealing is very rapid. In fact, during later cycles of amplification, a majority of product is reannealed each cycle during cooling before the primer annealing temperature is reached. This occurs with cooling rates of 5-10°C/second. Product reannealing with slower, prior art temperature cyclers will even be greater because more time is required to transition between denaturation and annealing temperature. This undesirable effect limits product yield, and is a major cause of the "plateau effect" known in the art.

Furthermore, the present invention provides an inexpensive instrument that can be used in commercial applications and that continuously monitors fluorescence during rapid cycle amplification. The thermal cycler of the present invention is capable of carrying out DNA amplification in no more than 10-20.minutes and the optical and detection components of the present invention discern one, two, three, or more fluorophores. The preferred embodiments of the present invention monitor a number of individual samples, for example, 24 samples (capillary sample tubes 320 in Figure 11) from once every few seconds, preferably once a second, and more preferably ten times each second.

Advantageously, preferred embodiments of the present invention utilize fluorescence feedback for real time control and optimization of the biological process, for example DNA amplification, as the process is ongoing. Thus, with the preferred embodiments disclosed herein, the fluorescence which is detected is used to control temperature cycling. Using embodiments of the present invention disclosed herein, and using the preferred continuous monitoring techniques with dsDNA-specific dyes, extension will be terminated each thermal cycle after the detected fluorescence stops increasing. Further, in accordance with the present invention, denaturation conditions are also controlled by increasing the temperature only until the product is completely melted. Still further, in accordance with the present invention, primer annealing is monitored with resonance energy transfer between fluorescein and Cy5-labeled oligonucleotides. Moreover, using the present invention, temperature cycling of the sample is automatically terminated after a predetermined amount of product has been made.

In accordance with the present invention and as is possible using the apparatus of the present invention, rapid temperature cycling with minimal annealing and denaturation times improves quantitative PCR and increases the discrimination of allele specific amplification. Rapid cycling for cycle sequencing reduces sequencing ambiguities and minimizes "shadow banding" in dinucleotide repeat amplifications. In accordance with the present invention, for long PCR up to 35 kb, yield is improved when the sample is exposed as little as possible to high denaturation temperatures.

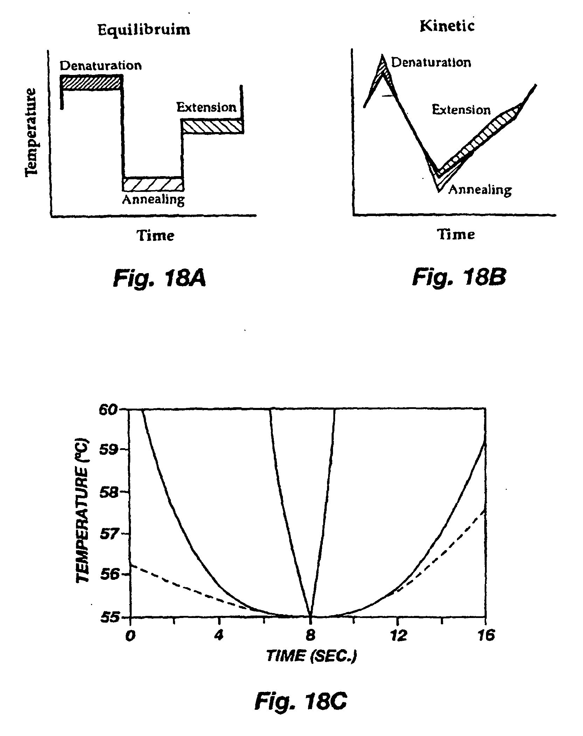

In contrast to the previous approach to PCR which treat PCR as three reactions, denaturation, annealing, extension, each of which occur at three different temperatures (as represented in Figure 18A), one aspect of the present invention provides that a kinetic paradigm for PCR renders important improvements. Using a kinetic paradigm for PCR (as represented in Figure 18B), the temperature vs. time curve consists of continuous transitions between overlapping reactions. The method and apparatus of the present invention is particularly efficient at carrying out PCR under the kinetic paradigm. Figure 18C is a graph representing different time/temperature profiles near an annealing temperature of 55°C. In Figure 18C, the solid trace shows a centrally positioned "spike" representing the temperature of response of a 10 $\mu\ell$ sample. In contrast, the traces shown as short and long line segments in Figure 18C represent the temperature responses of samples obtained using heat block instruments. As can be seen from Figure 18C, the embodiments of the present invention produce annealing segment "spikes," with the advantages discussed herein, in contrast to the temperatures "plateaus" according to the conventional wisdom in the art.

The previously available instrumentation used for detection presented many drawbacks. Rapid, precise temperature cycling is provided by the system of the present invention described herein, in contrast to previously available instrumentation that is five to ten times slower. With the continuous fluorescence monitoring also provided by the system of the present invention, the temperature dependence of hybridization can be followed. By following hybridization during temperature cycling, the number of probes and/or spectral colors required can be minimized. That is, different products and mutations can be detected by their dynamic melting characteristics, rather than going to the trouble of synthesizing different fluorophore-labeled probes for each DNA species that is to be detected.

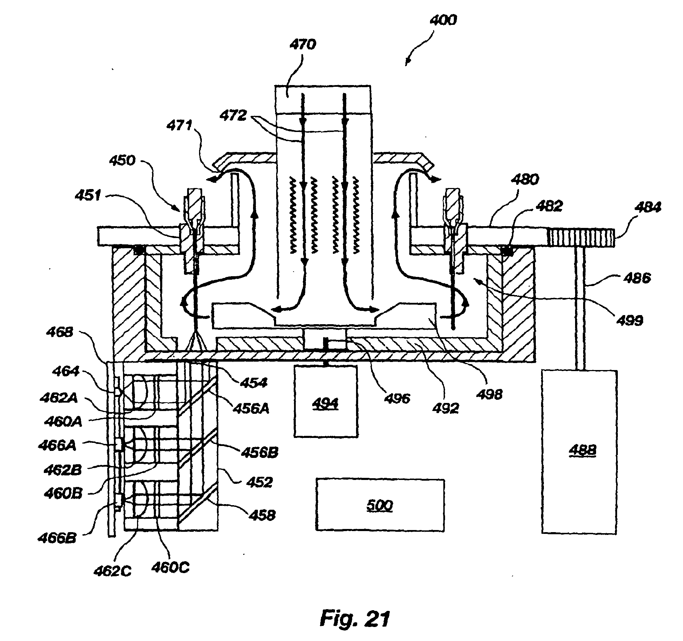

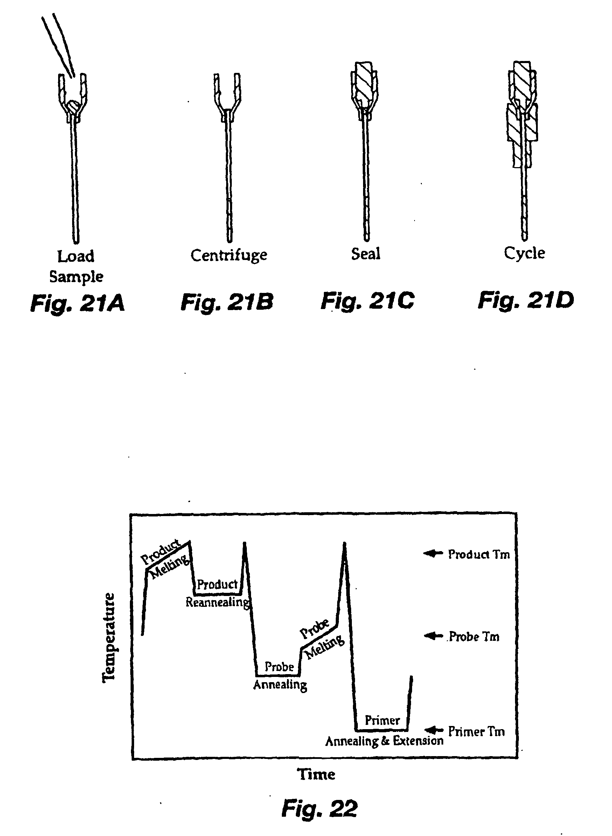

Most cost effective is a high intensity light emitting diode used instead of a xenon arc source or a laser for sample illumination, and photodiodes for detection. Samples are loaded into glass capillary sample tubes, or alternatively into composite glass/plastic sample containers (see Figure 21A-D) in a 96-well format that does not require heat sealing. Real time fluorescence control of temperature cycling improves amplification quality. For example, if the temperature of samples is increased only until denaturation occurs, product exposure to high temperatures is minimized. This increases yield by limiting product and enzyme degradation and increases specificity by limiting amplification of products with a high melting temperature.

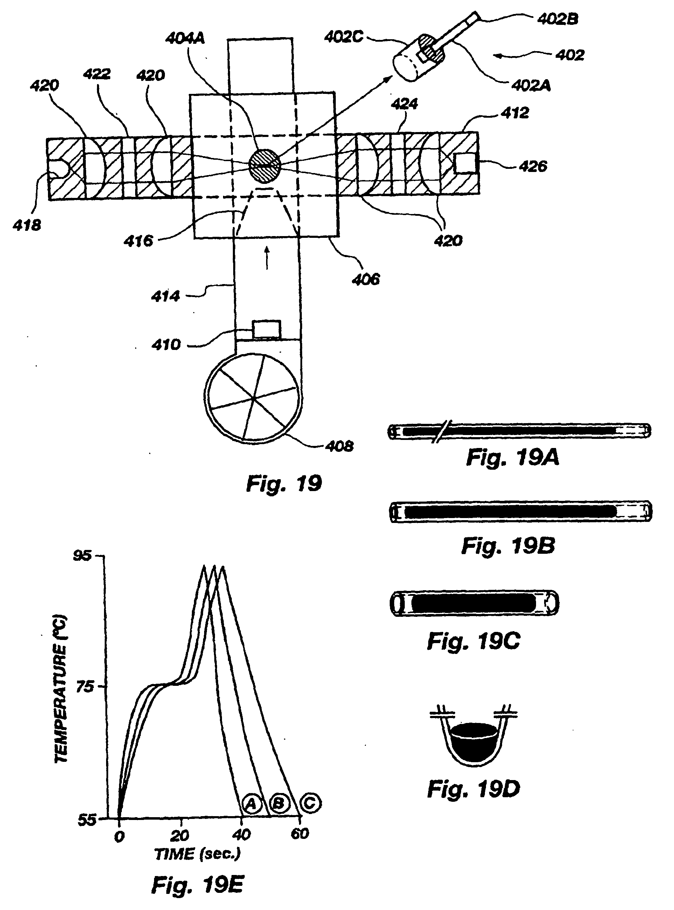

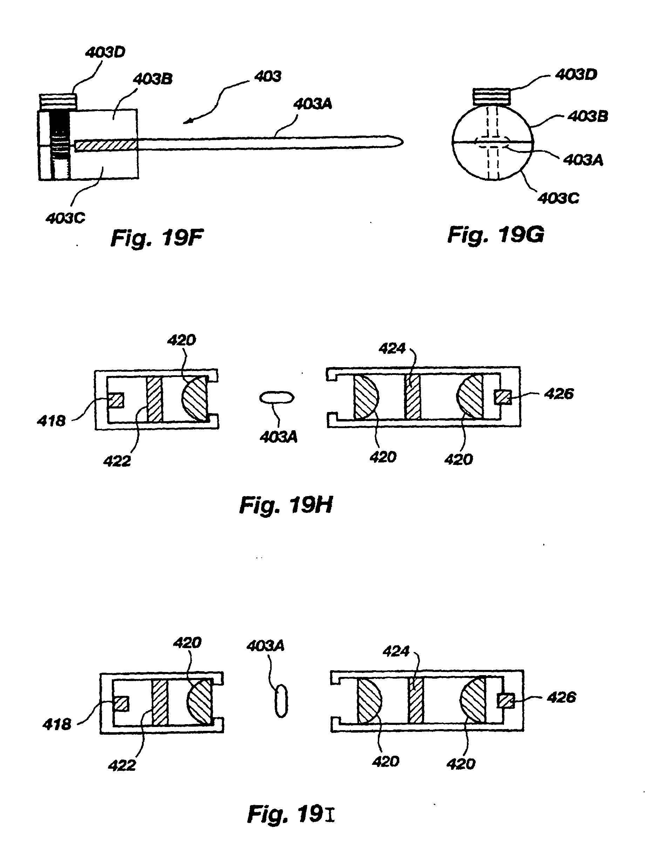

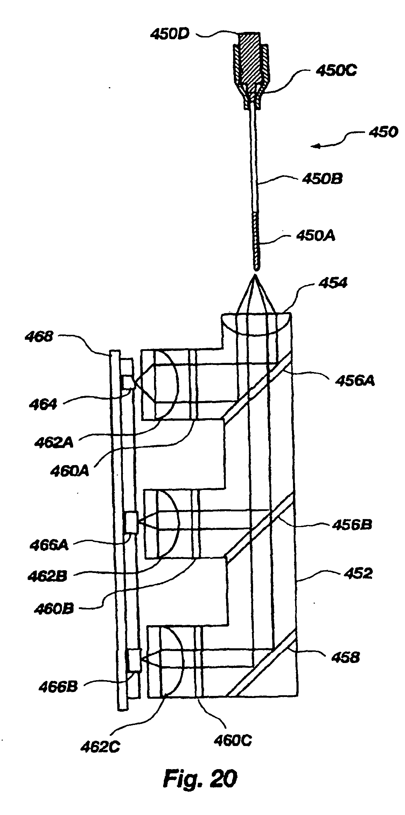

Reference will next be made to Figure 19, of a comparative example which provides a diagrammatic representation of another preferred embodiment of the present invention configured for continuous monitoring of a single sample. It will be understood, however, that the structures represented in Figures 19 and 20 can also be incorporated into a system which automatically processes multiple samples, such as the apparatus represented in figure 11 and as will be explained shortly herein. In the example of Figure 19, a single sample holder 402 is placed in a holding bracket 404 positioned at the intersection of a temperature-controlled air stream and a linear optical path. The sample holder 402 includes a tube 402A which has many of the desirable characteristics of a capillary tube. Different configurations of capillary tubes can be used and the tube 402A preferably has a rectangular cross section. The biological sample preferably is held at a bottom end of the tube 402A as indicated at 402B. A cap 402C is also preferably provided on the sample holder 402.

Reference will next be made to Figures 19A-19E which compare the effect of different configurations of sample containers on the temperature response of the sample itself. The temperature-time tracings shown in Figure 19B correspond to the response obtained using the sample container configurations represented in Figures 19A-C, respectively. Figure 19D represents a sample container which is less preferred for use in the present invention and is included for comparison. Using the information set forth herein, those skilled in the art can arrive at optimum sample container configurations for particular applications of the present invention. Further information regarding each of the sample container configurations represented in Figures 19A-D are set forth below.

| Figure | Surface Area (mm$^2$/10$\mu\ell$) | Fluid Column Length (mm) | Sample Volume | Source |
|--------|-----------------------------------|--------------------------|---------------|--------|
| 19A | 77 | 47 | 10$\mu\ell$ | Kimble KIMAX #46485-1 |
| 19B | 42 | 13.8 | 34 $\mu\ell$ | Kimble KIMAX #46485-15 |
| 19C | 32 | 8 | 59$\mu\ell$ | Kimble KIMAX #34500-99 |
| 19D | 18 | N/A | 10$\mu\ell$ | MICROAMP™ tube of Perkin-Elmer Cetus GeneAmp PCR System 9600 |

In the apparatus of Figure 19, an excitation radiation source 418, preferably an LED and most preferably a blue LED, is provided to illuminate the sample holder 402. The radiation emitted by the excitation radiation source 418 passes through aspheric focusing lenses 420 and an excitation bandpass filter 422 and the radiation is focused onto the sample holder 402.

The optical components illustrated in Figure 19 are preferably held in an optical housing 412. A housing 406 is also provided. A fan 408 is provided to move air through an air duct 414 and over the sample holder 402 held in the sample bracket 404. A temperature unit 410 is placed in the air flow path to provide heating or heating and cooling for the air passing over the sample holder 404. A nozzle 416 effectively directs the air over the sample holder 404.

The emissions which are given off by the sample pass through two more aspheric lenses 420 and an emission bandpass filter 424 and are received by a photo detector 426, which preferably is a photo diode. Those skilled in the art can readily provide the control components needed to advantageously operate the apparatus represented in Figure 19 using the information set forth herein.

Figures 19F and 19G are side and end views, respectively, of one preferred sample container 403 which utilizes a rectangular capillary tube 403A. The capillary tube 403A is preferably one available from Vitro Dynamics Inc. having dimensions of 1mm X 3mm X 50mm. A first cap member 403B and a second cap member 403C are held together by a screw 403D, the screw 403D also functioning as a holder for the capillary tube 403A.

Figures 19H and 191, respectively, show two possible orientations of a rectangular capillary tube 403A when detecting fluorescence of the sample contained therein. Figure 19H shows the rectangular capillary tube 403A oriented so that its edges are in line with the optical axis of the excitation and detection optics ("edge excitation and detection"). Figure 191 shows the rectangular capillary tube 403A oriented so that its faces are in line with the optical axis of the excitation and detection optics ("face excitation and detection"). Surprisingly, the fluorescence signal obtained from the edge detection orientation shown in Figure 19H is about three-fold to about five-fold higher than obtained with the face detection orientation shown in Figure 19I. The desirable characteristics of using the edge detection orientation shown in Figure 19H is at least partially due to total internal reflection which takes place in the capillary tube 403A which concentrates the fluorescence signal to the extremities of the capillary tube 403A.

Figure 20 shows the optical components of another preferred example embodiment. The optical components represented in Figure 20 are preferably incorporated into the thermal cycling and sample handling structures represented in Figure 21, which will be more fully described shortly, but which can also be used with many different arrangements to provide monitoring (most preferably continuous monitoring) of a sample undergoing the polymerase chain reaction.

In contrast to the arrangements previously disclosed herein, the optical excitation and detection paths are combined in the example of Figures 20 and 21, referred to herein as an epifluorescent path, rather than a linear path. In the example of Figures 20 and 21, the excitation and emission radiation follow the same optical path between the capillary tube and the dichroic element used in the excitation path. A capillary tube is particularly adapted for use in the embodiment of Figures 20 and 21 due to the total internal reflection (also referred to as "light piping") along the length of the capillary sample tube which is exploited to increase both excitation and emission intensities.

In the embodiment of Figures 20 and 21, to accommodate maximal light piping, the optical axis is parallel to the length of the capillary tube (paraxial) with the tip of the capillary tube positioned at the focal point. Assuming a refractive index of about 1.33 for the sample being detected, about 12.3% of emitted light is guided to the tip. It is understood that centrifuge action can be used to move the sample to the tip of the capillary tube.

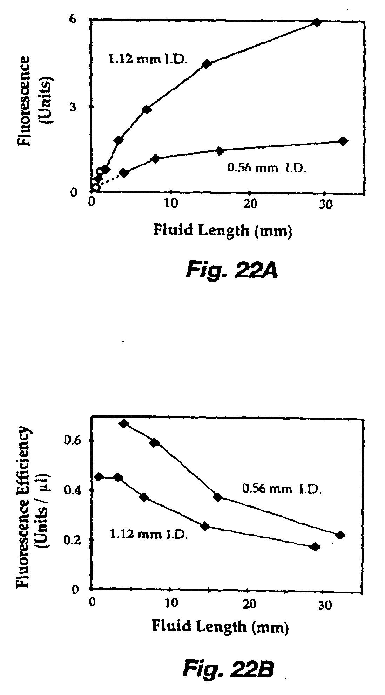

Figure 22A charts the effectiveness of light piping when detecting fluorescence at the tip of the capillary tube and shows a 10-fold increase in signal intensity by viewing the tip (closed diamonds) rather than the side (open circles) of the capillary sample container. Also, as indicated in Figure 22B, the results obtained using capillary sample tubes of two different sizes and which were filled to different lengths with dsDNA stained with SYHR® Green I are plotted. As can be surmised from Figures 22A and 22B, the observed epifluorescence increases as more sample is added to the tube, although the fluorescence efficiency decreases.

The optical properties of the emission from a capillary were investigated by stimulating fluorescence in a capillary filled with a fluorescein solution at 470 nm. The emission from a blunt end of the capillary was seen to be homogenous across the face of the capillary as opposed to concentrated in the glass as would be exepcted if the emission were the result of evanescent wave fluorescence.

The optical components represented in Figure 20 carry out paraxial epifluorescent illumination of the capillary tip, which provides advantageous results not otherwise obtainable. In Figure 20, an excitation radiation source 468 is preferably a blue LED, such as one known in the industry as a super bright LED and available from LEDtronics. The emitted fluorescence signals are acquired by photo detectors 466A and 466B. The excitation radiation source 468 and the photo detectors 466A and 466B are supported on a mounting board 468 which also includes necessary circuitry and which integrates filters with the photo detectors 466A and 466B. A preferred mounting board is available from Baling Electrooptics which includes 0.5 inch interference filters with high performance silicon photodiodes in TO5 packages. The excitation and detection components are supported directly on the mounting board 468 with associated electronics. It is preferred that the optical components are preferably ≤1.0 inches in diameter. A collimating lens 454, two dichroic filters 456A and 456B, a mirror 458, interference filters 460A-C, and aspheric focusing lenses 462A-C direct the radiation to and from the sample.

While Figure 20 utilizes only two colors/wavelengths when performing an analysis, those skilled in the art can readily adapt the embodiment to provide three, or more, color analysis. To provide three or more color analysis, the apparatus represented in Figure 20 can accommodate additional dichroic filters and photo detectors. Moreover, it is within the scope of the present invention to allow simultaneous separation of wavelengths onto a linear photo detector array, as is available in the industry, for multicolor acquisition. When a linear photo detector array is used, it is preferred that a prism or diffraction grating be utilized in cooperation with a lens and a photo detector array or CCD for detection of multiple wavelengths. One preferred linear photo detector array available in the industry collects 15-30 wavelength bins of 10-20 nm each between 500 and 800 nm. Various configurations of optical components, for example the Littrow autocollimating configuration for gratings used in most monochrometers, can be arrived at using the information set forth herein to arrive at the best accommodation between collection efficiency, spectral resolution and spatial requirements. The apparatus of Figure 20 will now be further described incorporated into an automated thermal cycling apparatus represented in Figure 21.

Figure 21 provides a schematic representation of another apparatus 400 which includes rapid temperature cycling components, sample handling components, and the optical components represented in Figure 20, all working together to provide fluorescence detection at the tip of the sample containers (epifluorescence). The rapid temperature cycler with epifluorescence detection 400 represented in Figure 21 provides particular advantages. It is to be understood that this described embodiment is merely exemplary of the present invention and that those skilled in the art can arrive at many different arrangements for carrying out the invention claimed herein.

In the example represented in Figure 21, air is taken in through an aperture 470 and generally follows the flow path indicated by the lines 472. The temperature of the air, and thus the temperature of the plastic/glass capillary tube 450, is preferably adjusted using a 400 watt heating cartridge 474 which is preferably one available from Reheat, Inc. The heating cartridge 474 is positioned within a central duct 476. A fan 498 is provided to move the air in the indicated path 472. The fan is driven via a shaft 496 and a motor 494. The motor 494 is preferably a DC rare earth brush motor which is preferably available from Escap AG. and having a maximum rpm of 15,000. When heating the plastic/glass tubes 450, the heating cartridge is proportionally controlled and the fan is run at a relatively low speed (12 volts, 0.5 amp) to provide temperature homogeneity for all of the plastic/glass sample containers 450. When cooling the plastic/glass tubes 450, the heating cartridge 474 is disabled and the motor 494 is run at a fast speed (for example with the above-mentioned preferred motor maximum speed is obtained by applying 27 volts, 1.4 amps). The fan 498 forces air into the aperture 470 and out via exhaust ports 471.

In the rapid temperature cycler with epifluorescence detection 400, it is preferred that twenty-four plastic/glass capillary tubes 450 (two of which are represented in Figure 21) be symmetrically arranged around the heating cartridge 474 and the central duct 476. The plastic/glass tubes 450 are received by sleeves 451 which (due to their offset structure) allow for precise adjusting of the position of the individual plastic/glass capillary tubes 450 in a circular carousel 480. The sleeves 451 are preferably fabricated from brass. The off-axis structure of the sleeve 451 allows each sleeve 451 to be aligned so that the tip of the glass/plastic tube 450 can be precisely adjusted to be at the optical focal point represented in Figure 21, both laterally and longitudinally, at the time that the rapid temperature cycler with epifluorescence detection 400 is fabricated.

The carousel 480 is supported on a bearing 482 above a housing 490. The carousel 480 is positioned by a stepper motor 488 provided with a drive gear 484 connected to the motor 488 via a shaft 486. The stepper motor 488 is microstepped (using a controller (not explicitly represented in Figure 21) from New England Affiliated Technologies) to provide over 10,000 steps per revolution of the carousel 480, providing precise positioning of each plastic/glass tube 450. The interior of the housing 490 is provided with an insulative material 492, preferably in accordance with the previously described insulative material. Baffles 476 function to form the exhaust port 471 and to block ambient light.

Figures 21A-D provide additional detailed views of the plastic/glass tubes 450 and will be referred to for an explanation of the preferred method of using the same. The plastic/glass tube 450 includes a capillary tube portion 450B which is closed at one end. The capillary tube portion 450B can take many different configurations and is not limited to only a capillary tube type structure. It is, however, preferred that the volume of fluid held by the plastic/glass tube 450 be not more than 1 milliliter in order to promote sample temperature homogeneity and rapid thermal cycling. For example, it is preferred that the material from which the capillary tube portion 450B is fabricated have a thermal conductively in the range from about 20 to about 35 in accordance with the formula $(\dfrac{cal\ cm}{cm^2 s\ degree\ C})\ X\ 10$ . Further information

regarding the thermal conductivity of different glasses can be obtained from R.C. weast & M.J. Astle, HANDBOOK OF CHEMISTRY AND PHYSICS, page E-6 (1982) (CRC Press). The plastic/glass tubes 450 are also provided with a reservoir portion 450C which is preferably fabricated from an appropriate plastic and joined to the open end of the capillary tube portion 450B. While many different materials can be used for the reservoir portion 450C, it is preferred that a plastic material be formed in a funnel-like shape and attached to the capillary tube portion 450B.

A sample S is loaded into the composite plastic/glass tube 450 using a pipette P, or some other appropriate instrument, either manually or using an automated process. It is preferred that the volume of the sample be in the range from about .01 $\mu\ell$ to about 10,000 $\mu\ell$, more preferably in the range from about .01 $\mu\ell$ to about 100 $\mu\ell$, and most preferably in the range from about .01 $\mu\ell$ to about 10 $\mu\ell$, with about 5 $\mu\ell$ being the most preferred volume. Once a sample has been added to each plastic/glass tube 450, the plastic/glass tubes 450 are centrifuged at low speed to place the samples at the tips of the closed end of the capillary portion 450B, so that the sample forms a 0.2 - 2.0 cm column of fluid 450A as represented best in Figure 21B. A stopper 450D (which is preferably configured as a plastic plug) is then placed in the reservoir portion 450C to seal the plastic/glass tube 450 as shown best in Figure 21C and the plastic/glass tube 450 is placed in the sleeve 451 in the rapid temperature cycler with epifluorescence detection 400.

Different structures to seal the capillary tube portion 450B may be used.

The capillary tube portion 450B of the glass/plastic tube 450 is preferably a capillary tube made of glass available in the industry having 0.8 mm inner diameter and a 1.0 mm outer diameter, and which is closed/sealed on one end and flared at the other end for receiving the plastic reservoir 450C. The glass/plastic sample containers 450 can be readily and economically fabricated. The shape of the tip 450E of the capillary tube portion 450B is optimized for optical efficiency. Flat tips as well as tips with various outside curvatures and inside curvature are all contemplated within the scope of the present invention. Those skilled in the art can select the most efficient configuration for the tip.

As can be discerned from Figures 21A-D, the addition of plastic loading and sealing structures to a capillary tube provides great advantages and allows efficient use of glass capillary tubes while retaining their desirable thermal characteristics.

Advantageously, the composite plastic/glass tube 450 provide a convenient, inexpensive sample holder. With the example of Figure 21, it is preferred that fluorescence is acquired from single samples one to ten times each second. When acquiring fluorescence from multiple samples at the preferred rate, the samples need to be moved into position by rotation of the carousel 480 relatively rapidly. With the preferred stepper motor 488 and appropriate control devices (which can be selected using the information contained herein) each of the twenty-four samples can be rapidly and accurately moved to the monitoring position represented in Figure 21.

when the flourescent signal from each sample is acquired for 100 msec., the signal variation (with repositioning) is <1%. It will be appreciated that it is within the scope of the present invention to decrease the signal acquisition time, increase the transit speeds, and also observe the coefficient of variation from repeated sampling. When twenty-four samples are processed, and the carousel is rotated without stopping at a rate between one and ten revolutions per second, each sample has 0.37-3.7 msec of excitation and detection.

Using the information set forth herein, one skilled the art can select whether the flourescent signal is integrated via software or hardware. In one preferred embodiment, a graphical programming language is used in connection with the rapid temperature cycler with epifluorescence detection 400, such as one known in the industry as LabView (available from National Instruments), which has subprograms for peak detection and integration. In another preferred embodiment, integration is done in hardware with variable integration time (user adjustable sensitivity control) so that the signals reach a level optimal for analog-to-digital conversion.

Using the rapid temperature cycler with epifluorescence detection 400 represented in Figure 21, continuous monitoring of the sample as the reaction is ongoing allows determination of temperature cycling requirements during amplification, based on continuous observation of annealing, extension, and denaturation. This is in contrast to the prior art where all cycling parameters are determined and programmed before amplification begins. In accordance with the prior art, using complementary oligonucleotides equivalent to the lowest melting primer, the annealing efficiency is controlled even during early cycles. In many cases, extension and denaturation can only be monitored with dsDNA dyes during later cycles when enough product has been made. Significantly, such a requirement is not usually a problem because denaturation and extension conditions are made permissive enough to amplify most products, and data from the first amplification can be used to optimize subsequent runs.

Still referring to Figure 21, a user interface and instrument control 500 can be fabricated using the information set forth herein in connection with the embodiment of Figure 11. As one preferred example of a user interface and instrument control 500, a PENTIUM™ microcomputer running the Labview programming language with a 12-bit multifunction input/output card (available from National Instruments) provides data acquisition and control. It is preferred that the analog output signals be used to adjust the amplifiers associated with the photo detectors 466A and 466B. Analog input channels also measure the temperature of the samples via a thermocouple 499 as well as the flourescent detected from the sample by the photodiodes. The user interface and instrument control 500 represented in Figure 21 also provides digital I/O control of the excitation radiation source 468, the direction of the stepper motor 488, the heating cartridge 474, and the fan 498.

When continuous fluorescence monitoring of PCR samples containing the dsDNA dye SYBR Green I or fluorescently labeled oligonucleotide probes can be used to monitor hybridization and melting during individual amplification cycles. This information can be used by preferred arrangements for the user interface and instrument control 500 to provide improved and customized thermal cycling conditions. The benefits of using hybridization information for temperature cycling include:

(A) Ensuring that complete denaturation of the PCR product occurs with each cycle while:

Minimizing exposure to excessively high denaturation temperatures, thus, avoiding heat induced damage to the amplification products and polymerase.
Increasing reaction specificity by minimizing the denaturation temperature which selects against products with a $T_m$ higher than the intended amplification product.

(B) Maximizing the amplification efficiency by ensuring adequate time for product extension with each cycle while:

Minimizing the amount of time required for amplification by allowing no longer than needed to complete product extension.
Enhancing reaction specificity by selecting against products longer than the intended amplification product.

(C) Maximizing the amplification efficiency by ensuring adequate time for product extension each cycle while:

Minimizing the amount of time required for amplification by allowing no longer than needed to complete product extension.
Enhancing reaction specificity by selecting against products longer than the intended amplification product.
These would require longer than the allotted time to complete product extension.

(D) Initiating thermal cycling changes dependent on the level of fluorescence obtained or the current efficiency of amplification. For example, over-amplification and nonspecific reaction products can be minimized by terminating thermal cycling when the efficiency drops to a certain level. As another example, temperature cycling can be modified to initiate slower temperature ramps for melting curve acquisition when the fluorescence becomes detectable. This saves time because the slower ramps need not be used on earlier cycles. Other desirable changes may become evident on continued practice of the invention.
(E) Minimizing over-amplification damage to PCR product and/or initiation of melting curve acquisition before over-amplification has increased the background of nonspecific reaction products.

The user interface and instrument control 500 can follow preprogrammed time/temperature set points and/or, advantageously, can acquire detected fluorescence values and then use the acquired detected fluorescence values to alter or adjust one or more reaction parameters in real time to optimize the results obtained. As used herein, the term "reaction parameter" includes, but is not limited to, any parameter which is used as a basis for controlling a reaction. Such reaction parameters include, but are not limited to, denaturation temperature and time, primer annealing temperature and time, probe annealing temperature and time, enzyme extension temperature and time, and number of cycles. In general, control of the reaction is initally based on an estimate of reaction parameters from the fluorescence data. The original fluorescence data is either acquired as a change in fluorescence over time (temperature specific rates of denaturation, annealing, and extension), a change in fluorescence over temperature (product or probe $T_m$), or a change in extent of amplification (amplification yield and efficiency). These rates, $T_m$s, and their first and second derivatives, are used to determine optimal reaction parameters such as denaturation temperature and time, primer annealing temperature and time, probe annealing temperature and time, enzyme extension temperature and time, and number of cycles.

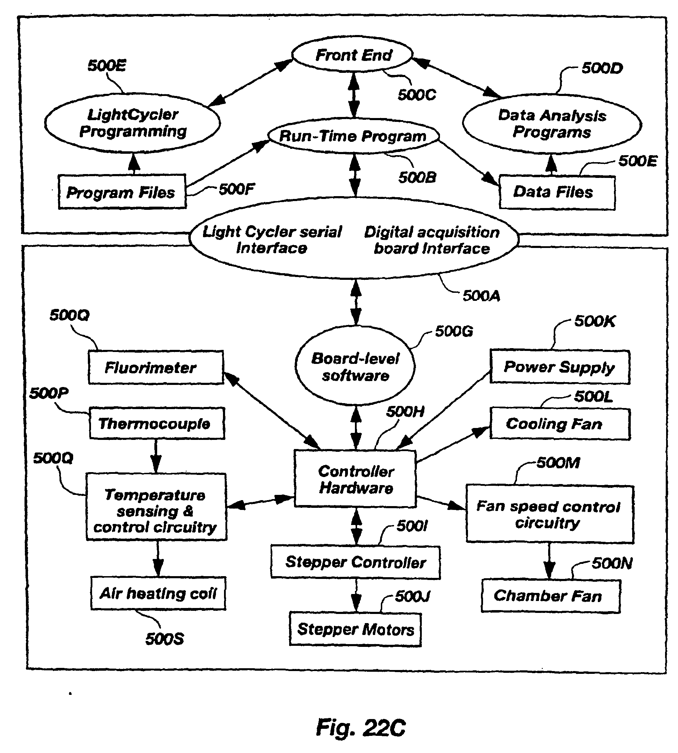

As depicted in the high level block of Figure 22C, tasks are divided between those carried out by a portion of the user interface and instrument control 500 (which preferably can be an IBM compatible computer using programing based upon the teachings set forth herein) (Blocks 500A-500E in Figure 22C) and those carried out by the remaining components (Blocks 500A. and 500G-500S in Figure 22C) of the rapid temperature cycler with epifluorescence detection 400. It is to be understood that the block diagram of Figure 22C is merely exemplary.

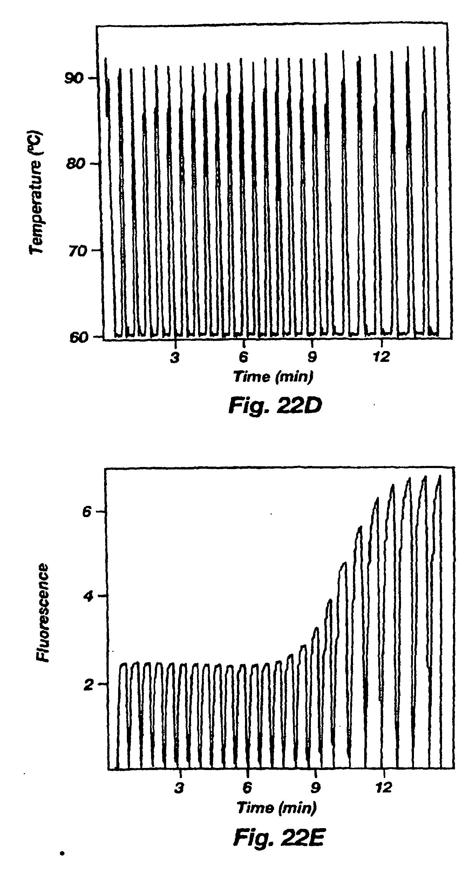

As an example of the advantages of the arrangement shown in Figure 22C, product melting control will be discussed. A melting peak fluorescence value is acquired for the intended PCR product and a baseline fluorescence is acquired for the sample containing the reaction mixture at the temperature at which the product is seen to have completely melted. Each cycle of the reaction uses this fluorescence value as a target. The approach being described in this example uses two stages in to provide a time lag to accommodate the requirement of sending the fluorescence values to a separate PC computer. With each product melting step, the temperature is increased until the fluorescence reaches an intermediate value, then the power applied to the heating device is reduced so that a temperature ramp of approximately 3°C per second is imposed so that the PC computer has adequate time to analyze the fluorescence and convey to other components that product denaturation has occurred. The resulting time/temperature plot is shown in Figure 22D. Figure 22D shows a characteristic increase in the melting temperature after twenty cycles as the concentration of amplification product grows. This is due to the fact that product $T_m$ is a function of product concentration.

As an example of the further advantages of the arrangement shown in Figure 22C, product annealing/extension will be discussed. During an extended hold at a combined annealing/extension temperature, the fluorescence of the sample is monitored and this information is used to ensure that adequate, but not excessive, time had been allowed for product extension. Fluorescence is monitored at ten second intervals, and if the fluoresce increased more than a preset ratio (typically 1.00 to 1.05), then the annealing/extension step is continued. Otherwise, the next product melting step is initiated. The interval of ten seconds is chosen to give a minimum of twenty seconds at the combined annealing/extension temperature.

Figure 22E shows a fluorescence/time plot which exhibits a characteristic increase in the dwell time at the combined annealing/extension temperature as the concentration of amplification product grows. This is due to the fact that as the primer concentration and polymerase become limiting more time is needed to complete product extension with each cycle.

As a yet another example of the advantages of the arrangement shown in Figure 22C, amplification plateau will be discussed. At the end of each annealing/extension step, the fluorescence value is acquired and stored. When this value increases to 1.2 times the lowest end-cycle fluorescence value and had subsequently stopped increasing below a user settable ratio (typically 1.00 - 1.02) the thermal cycle is terminated. Alternatively, a melting curve accusation step is initiated by entering a slow 0.1 °C to 0.2°C/second temperature ramp through the product $T_m$ and monitoring the fluorescence of the sample continuously. The resulting fluorescence/time plot shown in Figure 22D shows that after twenty-five cycles of amplification the ratio of cycle-by-cycle fluorescence growth fell below 1.00 and the reaction terminated. It will be appreciated that this approach can be used to acquire a high resolution melting curve for each sample. As a sample reaches its amplification plateau, a melting curve can be acquired for that sample, then regular temperature cycling can resume until another reaction reaches its amplification plateau.

Figure 22E illustrates useful temperature vs. time segments for fluorescence hybridization monitoring. Product melting curves are obtained during a slow temperature increase to denaturation. By quickly lowering the temperature after denaturation to a constant temperature, product, probe, or primer annealing can be detected. Probe melting curves are obtained by slowly heating through temperatures around the probe $T_m$. Those skilled in the art can readily utilize the system represented in Figure 21 to provide the necessary analysis, in real time if desired, during temperature cycling to provide heretofore unavailable information on the characteristics of the product, probe, and primer using the hardware and software described herein.

Absolute quantification of product is also advantageously carried out in accordance with the present invention. Continuous monitoring of double stranded DNA formation allows direct, absolute DNA quantification by reannealing kinetics. The sample temperature is quickly dropped from the denaturation temperature and held constant at a lower temperature that is still high enough to prevent primer annealing. The rate of product reannealing then follows second order kinetics. When different concentrations of DNA are tested, the shape of the reannealing curve is characteristic of the DNA concentration (see Figure 26). For any given PCR product and temperature, a second order rate constant can be measured. Once the rate constant is known, any unknown DNA concentration can be determined from experimental reannealing data. The curves can be fit by non-linear least squares regression during temperature cycling in real time using the LabView programming environment (explained previously). Cooling is not instantaneous, and some reannealing occurs before a constant temperature is reached, but regression analysis allow for this in accordance with the present invention. The technique requires pure PCR product, but this can be verified by melting curves also obtained during temperature cycling. Quantification by reannealing kinetics is independent of signal level and not affected by sample volume differences.

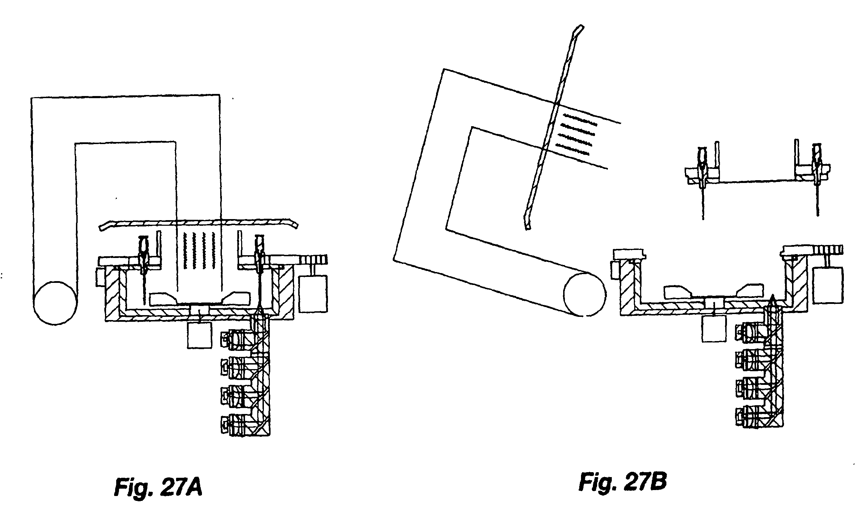

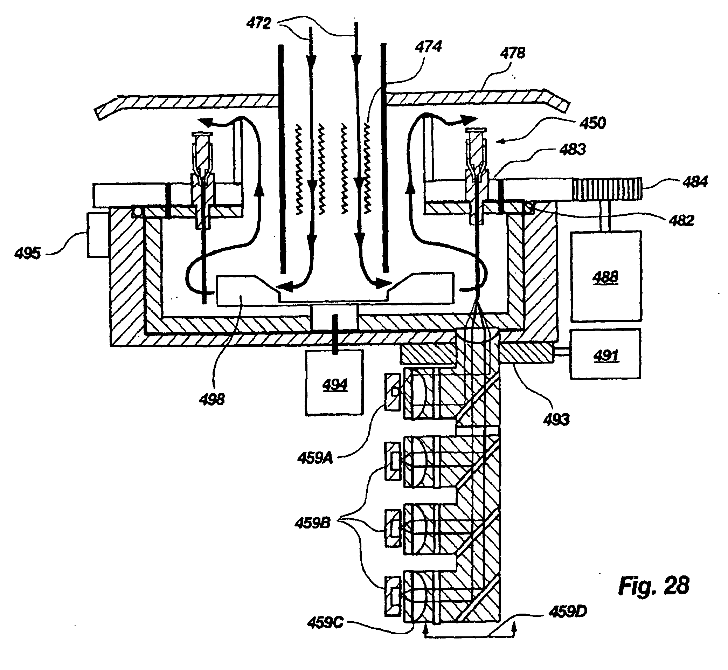

Figure 28 is a schematic representation of another apparatus which includes many of the structures included in the embodiment of Figure 21. In order to provide a succinct description of the embodiment of Figure 28, only those significant differences between those components represented in Figure 21 and those components represented in Figure 28 will be explained with the understanding that one skilled in the art can readily use the information contained herein to fabricate embodiments of the present invention. Figures 27A and 27B are cross sectional schematic views of the embodiment represented in Figure 28 in a run mode and a load mode, respectively.

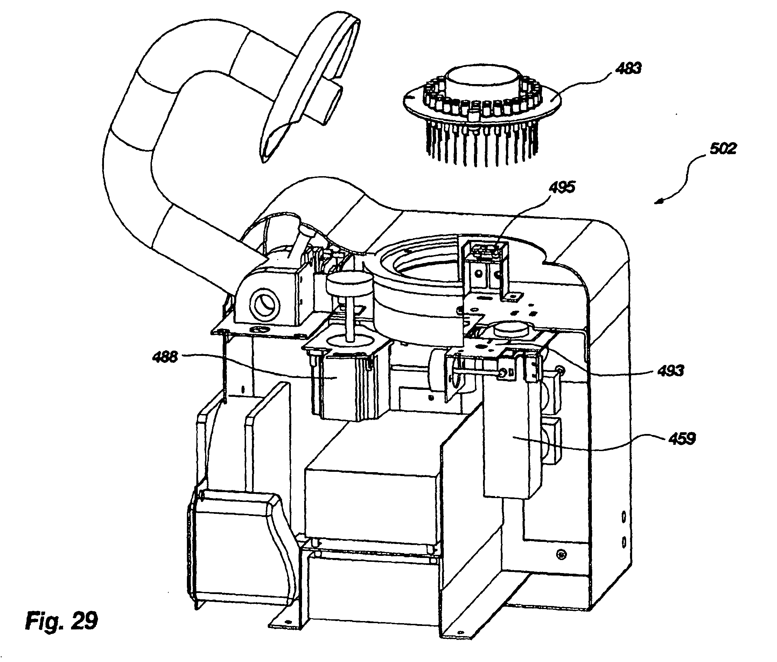

The embodiment of Figure 28 is a rapid temperature cycler, generally designed at 502, with fluorescence detection at the tip of the capillary tubes with automatic positioning of the sample containers in two dimensions which improves the fluoresce signal which is obtained from the sample. Figure 29 is a perspective view of the exterior of an apparatus including the components illustrated in the schematic representation of Figure 28.

As seen in both Figures 28 and 29, a removable circular sample tray 483 holds thirty-two samples. The removable circular sample tray 483 is placed into the rapid temperature cycler 502 so that it engages a carousel 481 which is driven by a motor 488. As the carousel 481 rotates, a hall effect position locator is used to precisely position the carousel 481 so that the each sample is precisely positioned over a flourimeter assembly 459. The flourimeter assembly 459 preferably includes a LED source 459A, three photodiodes 459B, focusing lenses 459C, and a filter assembly 459D. The flourimeter assembly 459 is similar in structure and function to that represented in Figure 20.

Most advantageously, the fluorimeter is mounted on a slider bearing 493 which is moved by a lateral stepper motor 491. As the carousel 481 rotates, the composite plastic/glass tubes 450 are precisely positioned over the fluorimeter assembly 459 in the direction of the carousel and the position is noted by the apparatus via the hall effect position locator 495 while the lateral stepper motor 491 adjusts the position of the fluorimeter assembly 459 is adjusted in a second dimension, and the position noted. Thus, the rapid temperature cycler 502 provides for improved placement of a plurality of samples into the apparatus using a removable sample tray 483 and provides for improved detection of a fluorescence signal from a sample.

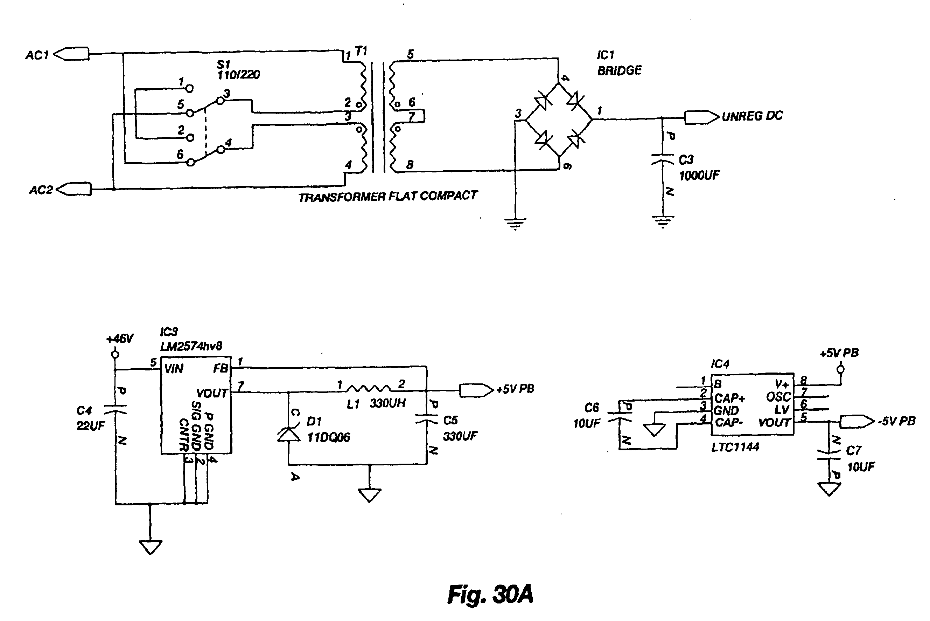

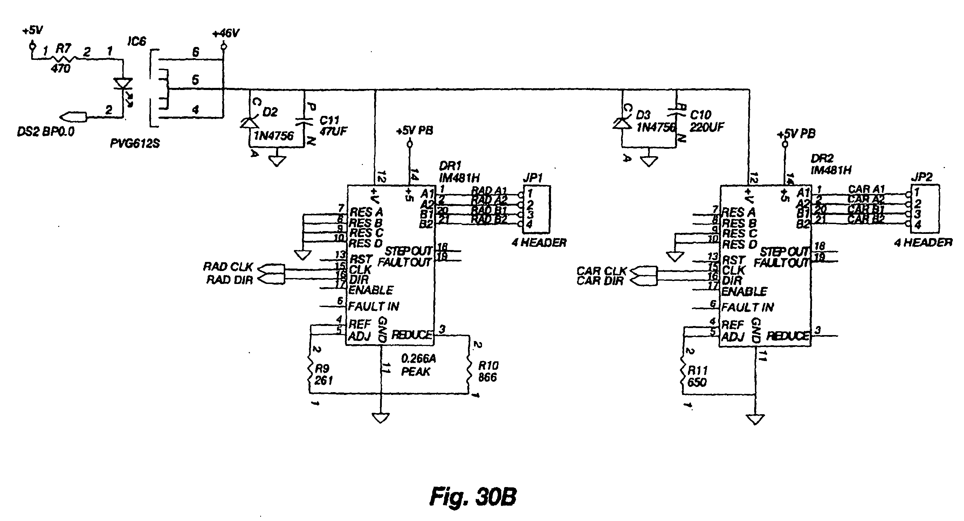

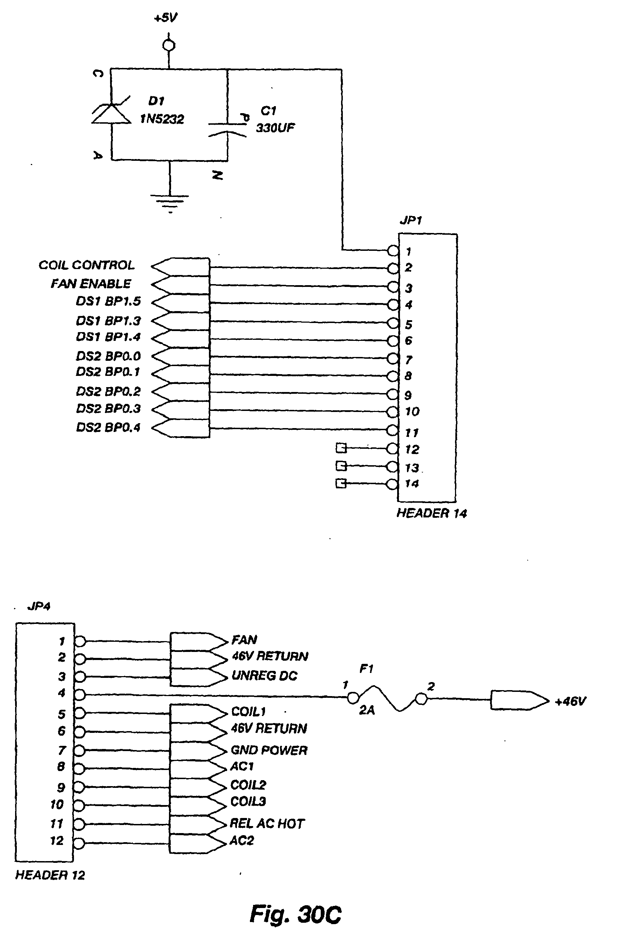

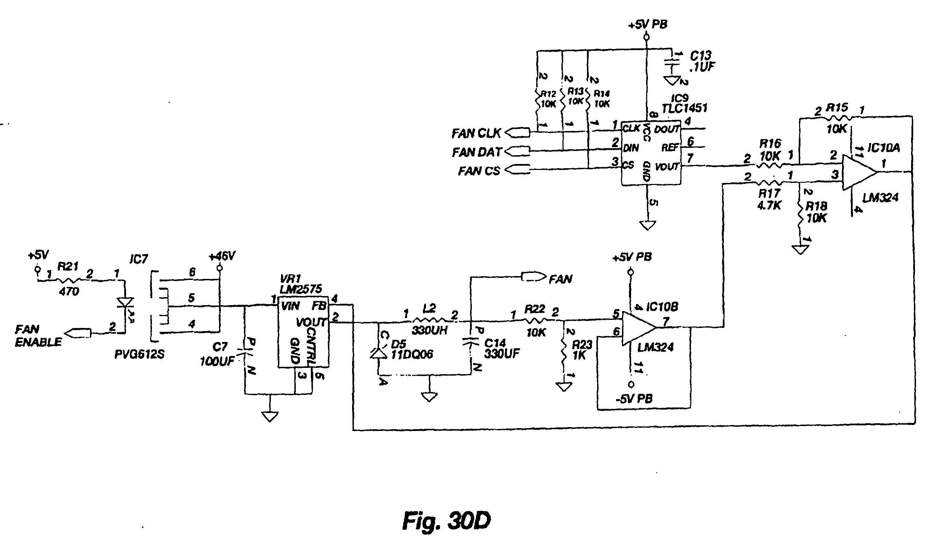

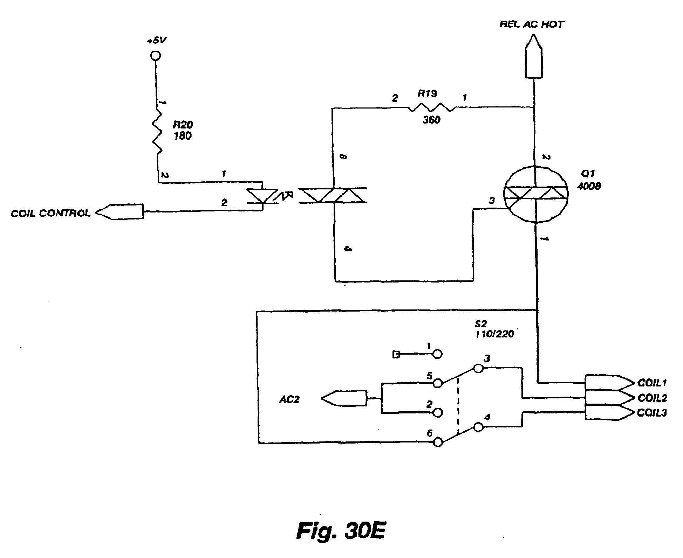

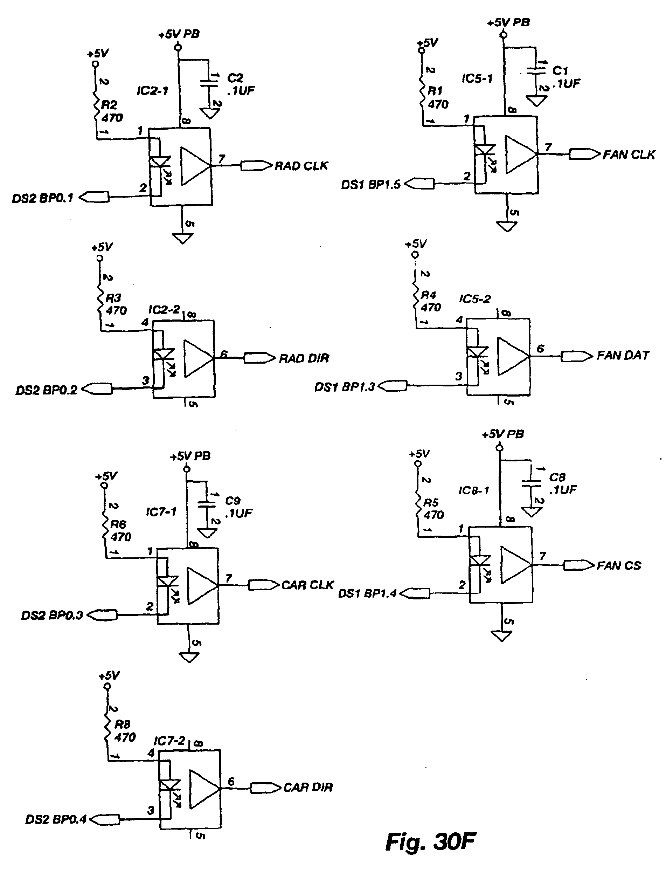

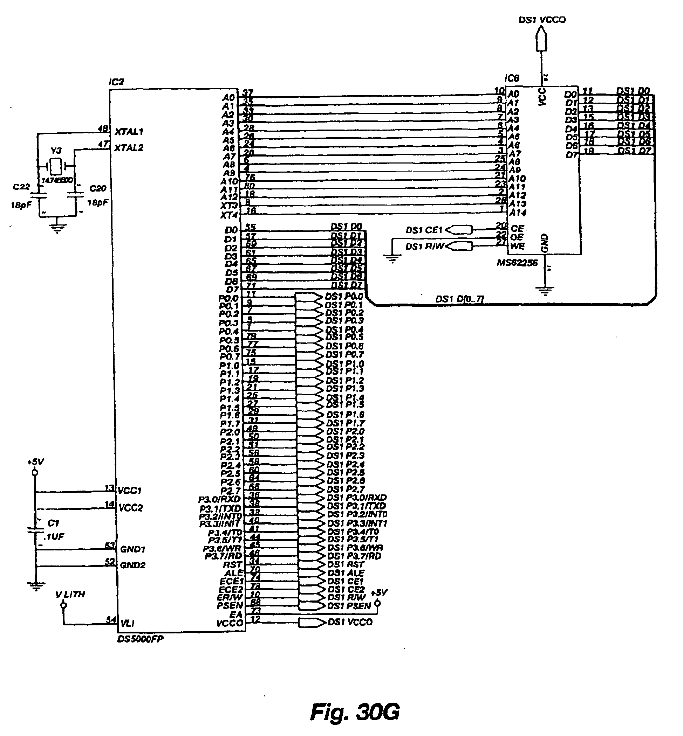

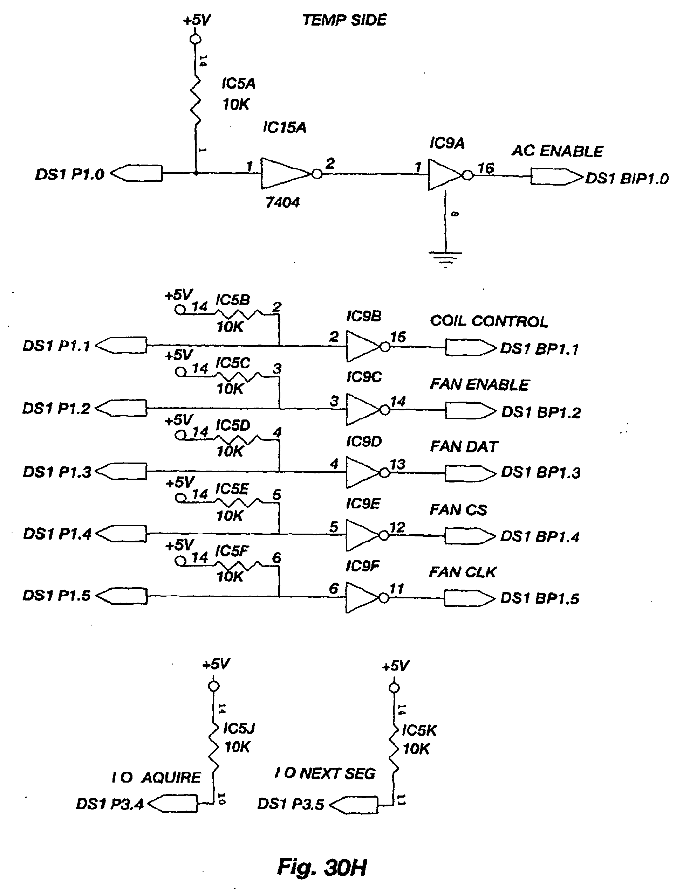

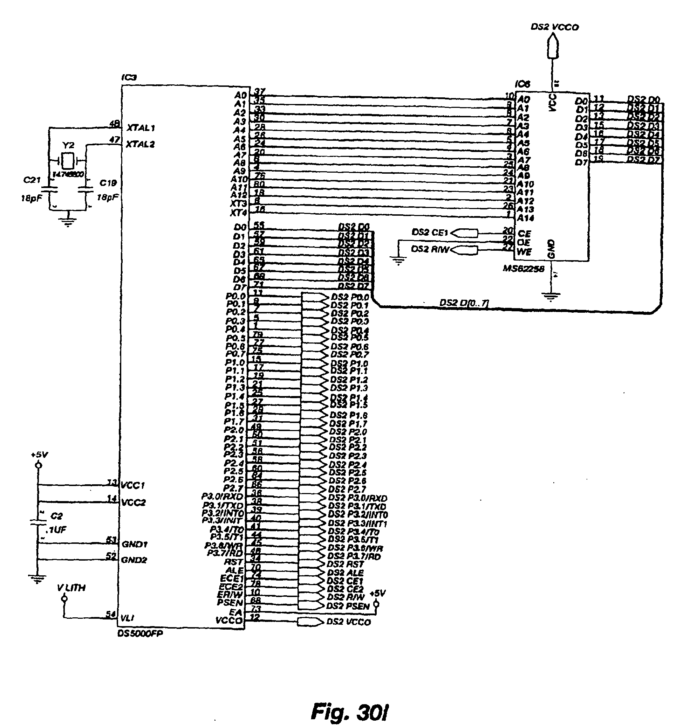

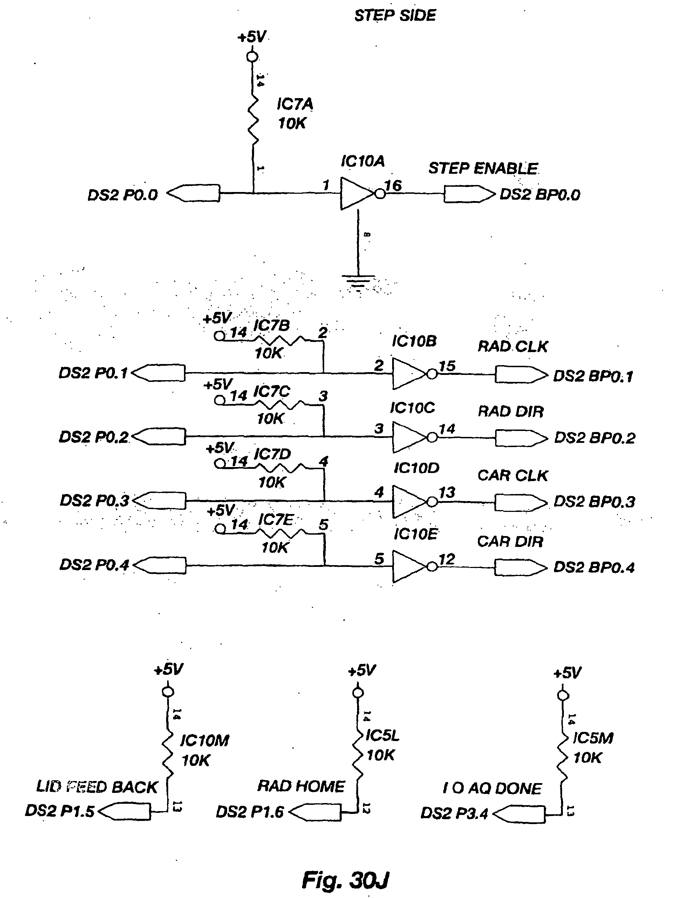

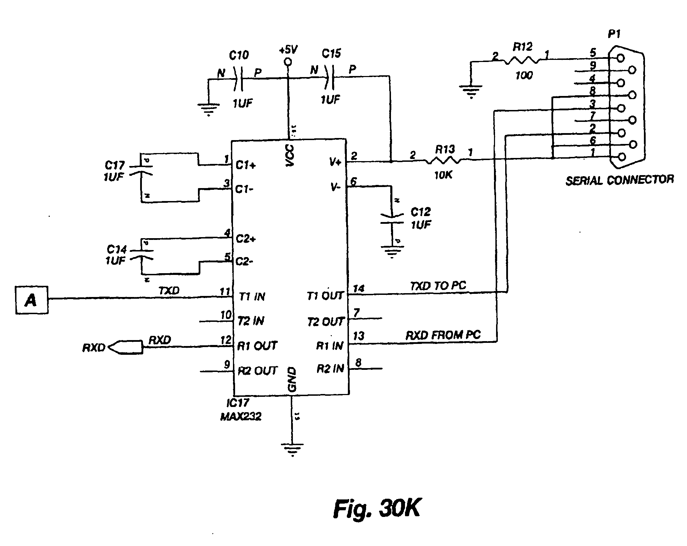

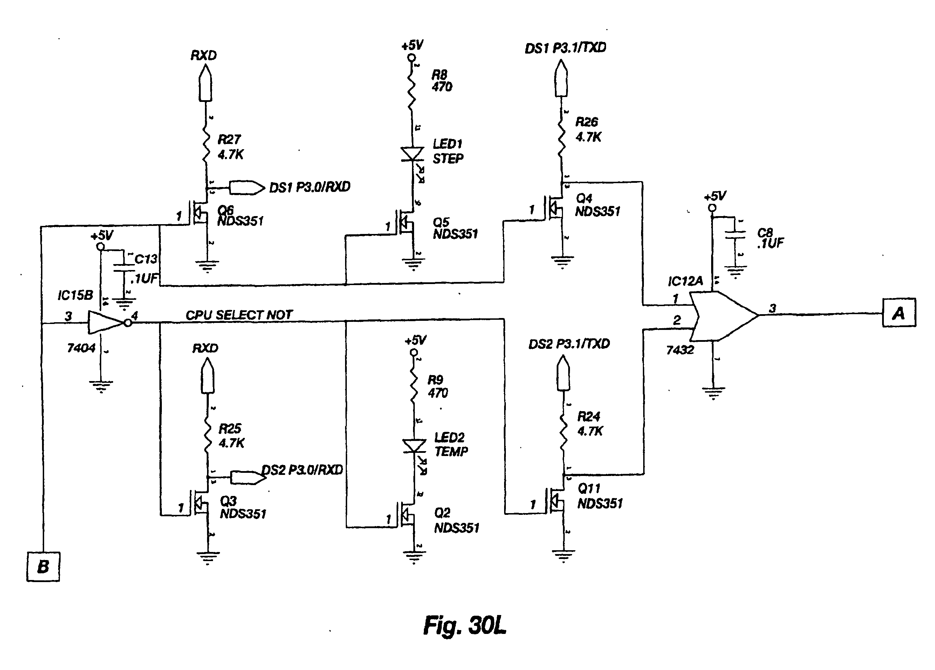

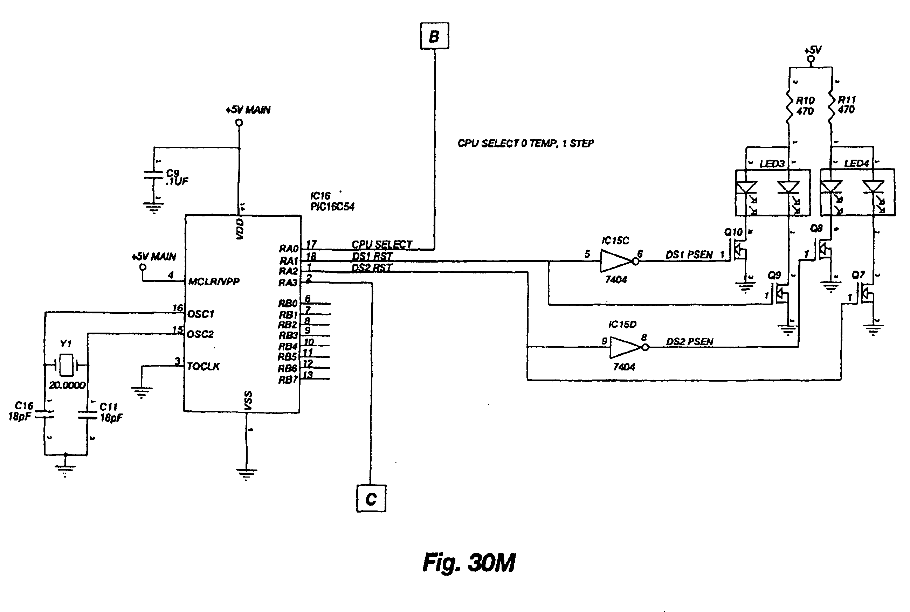

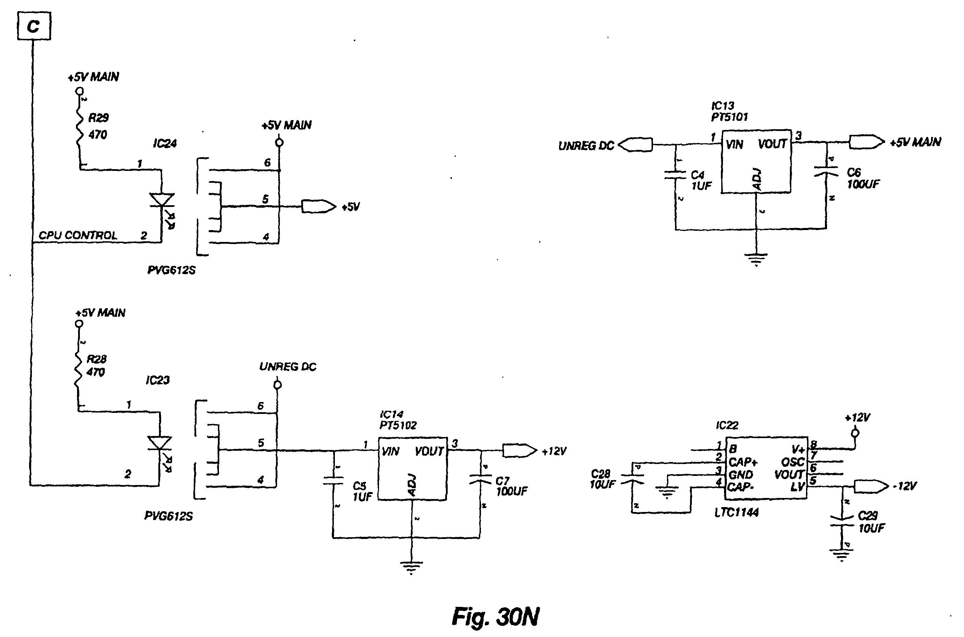

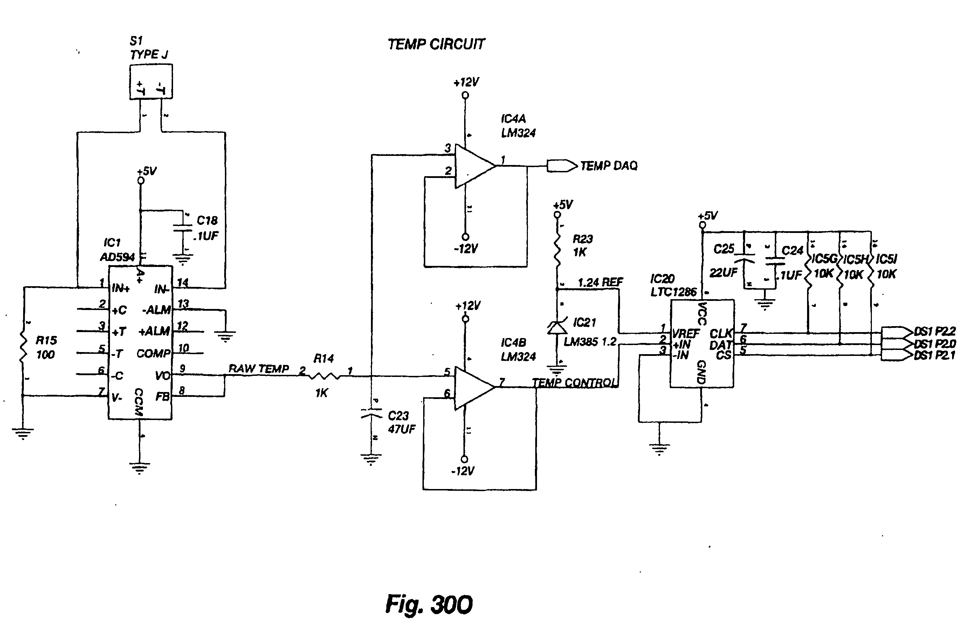

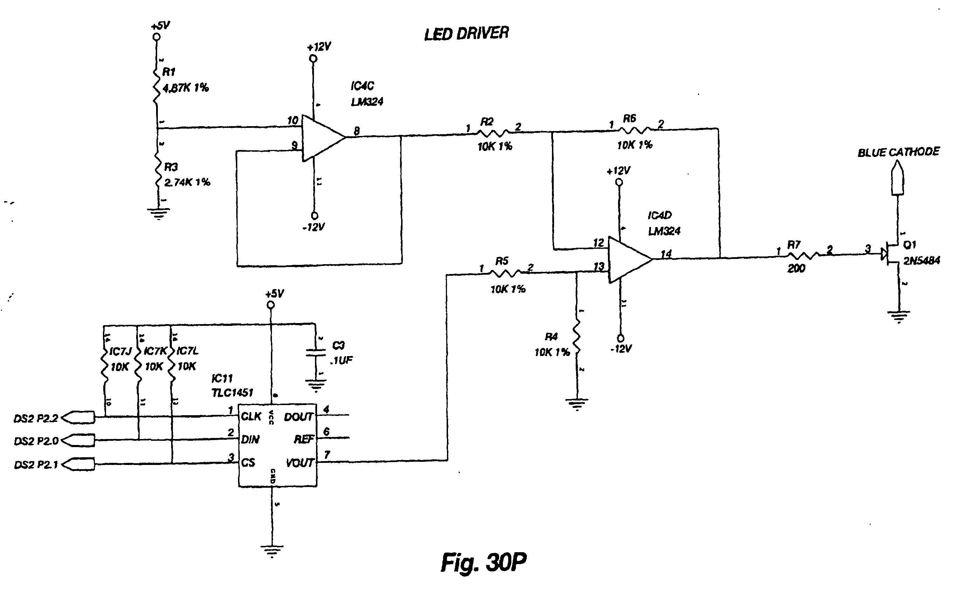

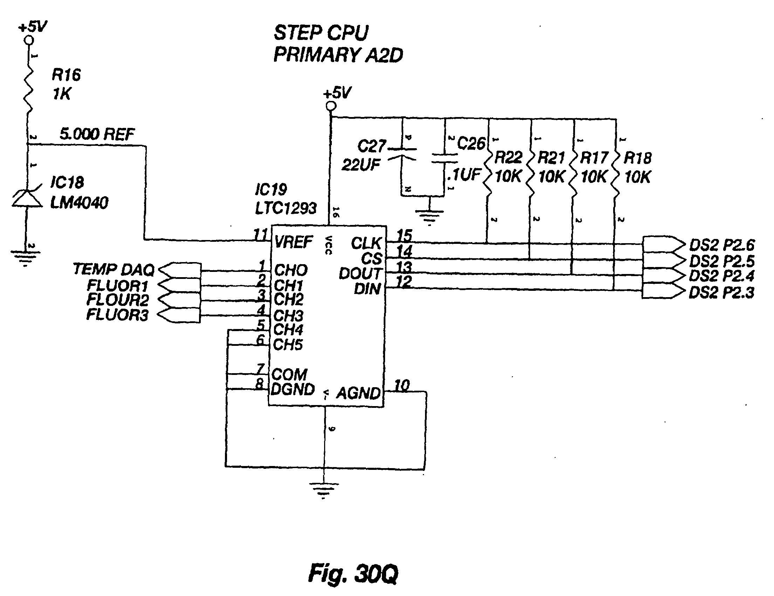

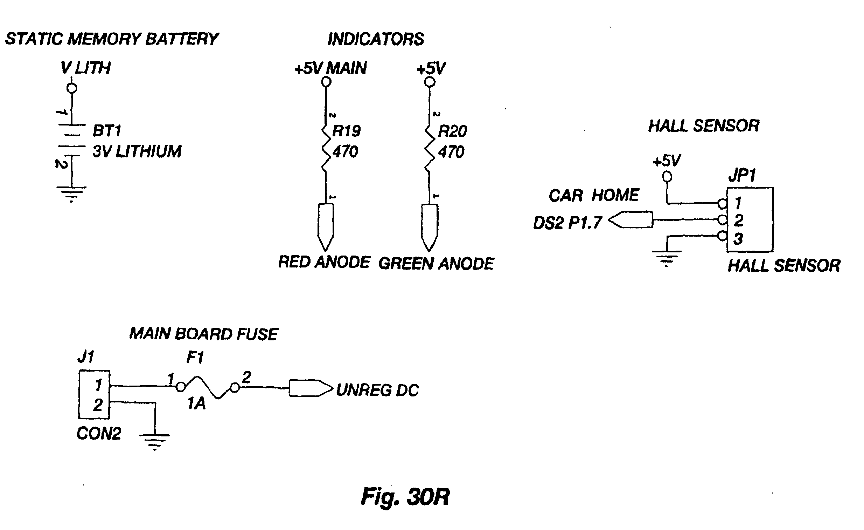

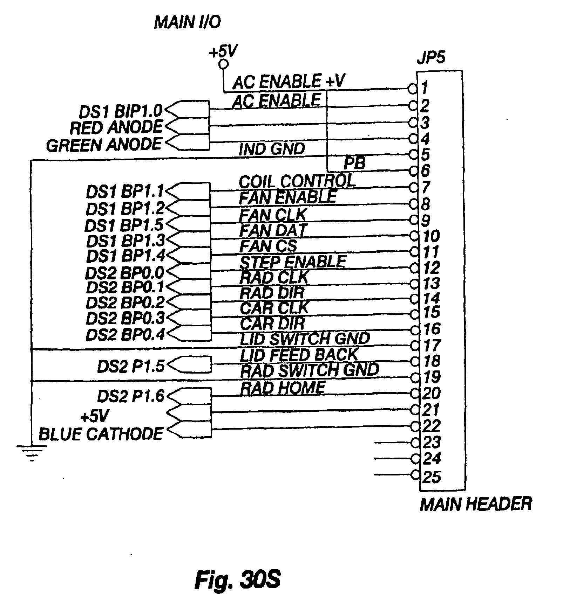

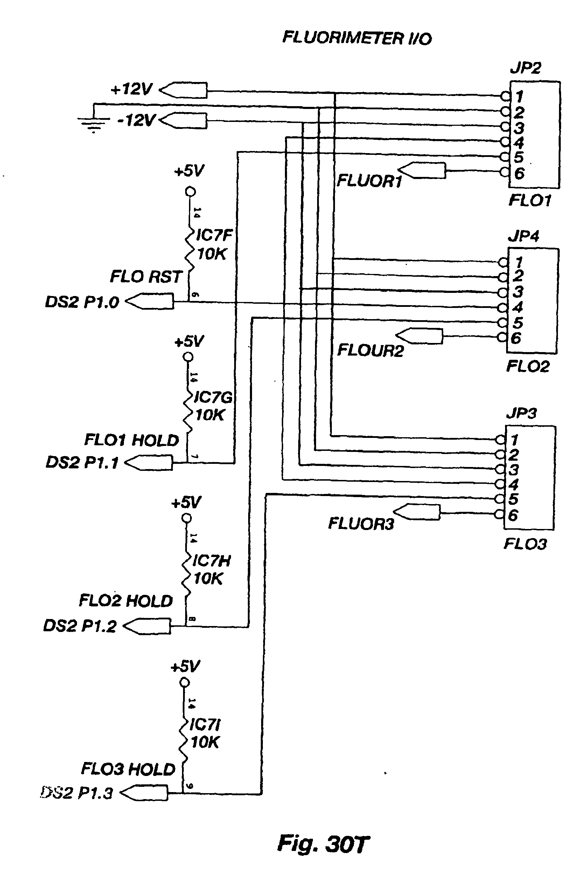

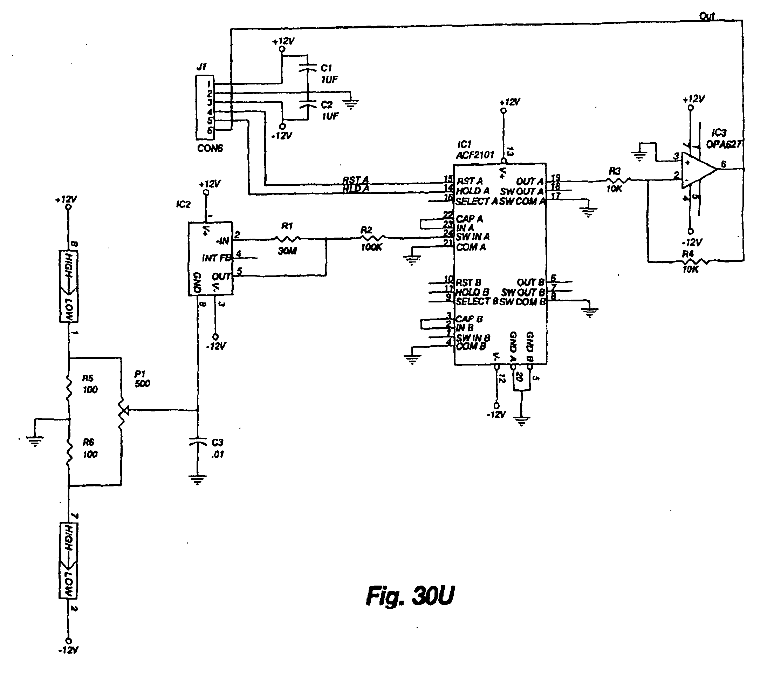

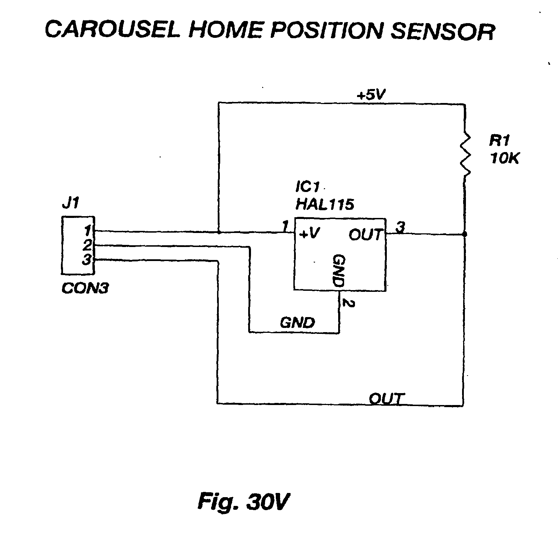

Provided in Figures 30A-30V are detailed schematic diagrams showing the preferred configuration of the electrical components of the rapid temperature cycler 502 represented in Figures 28 and 29. It is to be understood that the diagrams of Figures 30A-30V are merely one preferred arrangement for carrying out particular aspects of the present invention and these diagrams are not intended to be limiting of the scope of the present invention. In order to improve the clarity of the diagrams, the notations which are commonly used in the industry are maintained on these diagrams and are referenced in the corresponding parts list provided below.

| Parts List - MAIN | | | |
|---|---|---|---|
| Item | Quantity | Reference | part |
| 1 | 1 | BT1 | 3V LITHIUM |
| 2 | 9 | C1,C2,C3,C8,C9,C13,C18,C24,C26 | .1UF |
| 3 | 7 | C4,C5,C10,C12,C14,C15,C17 | 1UF |
| 4 | 2 | C7,C6 | 100UP |
| 5 | 6 | C11,C16,C19,C20,C21,C22 | 18pF |
| 6 | 1 | C23 | 47UF |
| 7 | 2 | C25,C27 | 22UF |
| 8 | 2 | C28,C29 | 10UF |
| 9 | 1 | F1 | 1A |
| 10 | 1 | IC1 | AD594 |
| 11 | 2 | IC2,IC3 | DS5000FP |
| 12 | 1 | IC4 | LM324 |
| 13 | 8 | IC5,IC7,IC10,R13,R17,R18,R21,R22 | 10K |
| 14 | 2 | IC6,IC8 | MS62256 |
| 15 | 2 | IC9,IC10 | DS2003 |
| 16 | 1 | IC11 | TLC1451 |
| 17 | 1 | IC12 | 7432 |
| 18 | 1 | IC13 | PT5101 |
| 19 | 1 | IC14 | PT5102 |
| 20 | 1 | IC15 | 7404 |
| 21 | 1 | IC16 | PIC16C54 |
| 22 | 1 | IC17 | MAX232 |
| 23 | 1 | IC18 | LM4040 |
| 24 | 1 | IC19 | LTC1293 |
| 25 | 1 | IC20 | LTC1286 |
| 26 | 1 | IC21 | LM385 1.2 |
| 27 | 1 | IC22 | LTC1144 |

Table continued

| Parts List - MAIN | | | |
|---|---|---|---|
| Item | Quantity | Reference | part |
| 28 | 2 | IC23,IC24 | PVG612S |
| 29 | 1 | JP1 | HALL SENSOR |
| 30 | 1 | JP2 | FLO1 |
| 31 | 1 | JP3 | FLO3 |
| 32 | 1 | JP4 | FLO2 |
| 33 | 1 | JP5 | MAIN HEADER |
| 34 | 1 | J1 | CON2 |
| 35 | 1 | LED1 | STEP |
| 36 | 1 | LED2 | TEMP |
| 37 | 2 | LED3, LED4 | RED/GREEN LED |
| 38 | 1 | P1 | SERIAL CONNECTOR |
| 39 | 1 | Q1 | 2N54B4 |
| 40 | 10 | Q2,Q3,Q4,Q5,Q6,Q7,QB,Q9.Q10,Q11 | NDS351 |
| 41 | 1 | R1 | 4.87K 1% |
| 42 | 4 | R2,R4,R5,R6 | 10K 1% |
| 43 | 1 | R3 | 2.74K 1% |
| 44 | 1 | R7 | 200 |
| 45 | 8 | R8,R9,R10,R11,R19,R20,R28,R29 | 470 |
| 46 | 2 | R15,R12 | 100 |
| 47 | 3 | R14,R16,R23 | 1K |
| 48 | 4 | R24,R25,R26,R27 | 4.7K |
| 49 | 1 | S1 | TYPE J |
| 50 | 1 | Y1 | 20.0000 |
| 51 | 2 | Y3,Y2 | 14.745600 |

| Parts List - POWER BOARD | | | |
|---|---|---|---|
| Item | Quantity | Reference | Part |
| 1 | 3 | C1,C5,C6 | 330UF |
| 2 | 1 | C2 | 47UF |
| 3 | 1 | C3 | 1000UF |
| 4 | 1 | C4 | 22UF |
| 5 | 1 | C7 | 100UF |
| 6 | 1 | C8 | 220UF |
| 7 | 5 | C9,C10,C11,C12,C13 | .1UF |
| 8 | 2 | C15, C14 | 10UF |

Table continued

| Parts List - POWER BOARD | | | |
|---|---|---|---|
| Item | Quantity | Reference | Part |
| 9 | 2 | DR1, DR2 | IM481H |
| 10 | 1 | D1 | 1N5232 |
| 11 | 2 | D2,D4 | 1N4756 |
| 12 | 2 | D5, D3 | 11DQ06 |
| 13 | 1 | F1 | 2A |
| 14 | 4 | IC1, IC2,IC3,IC4 | HCPL2630 |
| 15 | 1 | IC5 | LM2574hv8 |
| 16 | 2 | IC7, IC6 | PVG612S |
| 17 | 1 | IC8 | MOC 3020 |
| 18 | 1 | IC9 | TLC1451 |
| 19 | 1 | IC10 | LM324 |
| 20 | 1 | IC11 | BRIDGE |
| 21 | 1 | IC12 | LTC1144 |
| 22 | 1 | JP1 | HEADER 14 |
| 23 | 2 | JP2,JP3 | 4 HEADER |
| 24 | 1 | JP4 | HEADER 12 |
| 25 | 2 | L2, L1 | 330UH |
| 26 | 1 | Q1 | 4008 |
| 27 | 9 | R1,R2,R4,R5,R6,R7,R8,R9,R10 | 470 |
| 28 | 1 | R3 | 360 |
| 29 | 7 | R11,R13,R14,R16,R17,R18,R19 | 10K |
| 30 | 1 | R12 | 4.7K |
| 31 | 1 | R15 | 1K |
| 32 | 1 | R20 | 261 |
| 33 | 1 | R21 | 866 |
| 34 | 1 | R22 | 650 |
| 35 | 1 | R23 | 180 |
| 36 | 2 | S1,S2 | 110/220 |
| 37 | 1 | T1 | TRANSFORMER FLAT COMPACT |
| 38 | 1 | VR1 | LM2575 |

| Parts List - INTTEGRATOR | | | |
|---|---|---|---|
| Item | Quantity | Reference | Part |
| 1 | 2 | C1,C2 | 1UF |
| 2 | 1 | C3 | .01 |

Table continued

| Parts List - INTTEGRATOR | | | |
|---|---|---|---|
| Item | Quantity | Reference | Part |
| 3 | 1 | IC1 | ACF2101 |
| 4 | 1 | IC2 | OPT301 |
| 5 | 1 | IC3 | OPA627 |
| 6 | 1 | IC4 | REF200 |
| 7 | 1 | J1 | CON6 |
| 8 | 1 | P1 | 500 |
| 9 | 1 | R1 | 30M |
| 10 | 1 | R2 | 100K |
| 11 | 2 | R3,R4 | 10K |
| Parts List - INTEGRATOR | | | |
| Item | Quantity | Reference | Part |
| 12 | 2 | R5,R6 | 100 |
|  |  |  |  |

| Parts List - HALL EFFECT | | | |
|---|---|---|---|
| Item | Quantity | Reference | Part |
| 1 | 1 | IC1 | HAL115 |
| 2 | 1 | J1 | CON3 |
| 3 | 1 | R1 | 10K |
|  |  |  |  |

Exemplary programming code used in connection with the components of Figures 28, 29, and 30A-30V is included in the Programming Code Appendix B attached to the description. A handling system is provided for loading small volume sample vessels with liquid samples, particularly samples to analyzed by detection of emitted fluorescence. The sample vessel typically has a volume of less than 1ml, and is in the form of a tube (i.e. a capillary tube). The sample vessel typically has a volume to external surface area ratio of about 1mm, more typically less than about 0.5. Capillary tubes having an inner diameter of less than 1mm have a volume to surface area ratio of less than 0.25mm. The vessel used is preferably formed from an optically transparent material. Preferred materials are optically transmissible for light having a wavelength ranging from about 400 to about 800 nm. The use of such material will allow the detection of a fluorescent signal generated in a liquid sample held by the vessel. Moreover the use of vessels with a low volume to surface area ratio for analyzing fluorescence from a fluorescent sample enables more efficient detection of the fluorescence due to enhanced total internal reflection.

Vessels having a high surface area to volume ratio (or conversely, a low volume to surface area ratio) can be difficult to load with liquid samples. Advantageously, the sample handling system helps to overcome such difficulties. A vessel having a high surface area to volume ratio and an open end is provided with a funnel cap that fits onto the open end of the vessel to facilitate loading of liquid samples into the vessel. The funnel cap includes a first sample receiving port and a second sample transfer port and means for releasably fixing the funnel cap on the vessel so that the sample transfer port of the funnel cap and the open end of the vessel are in alignment. The funnel cap may be a plastic or rubber construction and is formed so that the inner diameter of the sample transfer port frictionally engages the outer diameter of the vessel proximal to its open end. However, other means of coupling the funnel cap to the vessel are know to those skilled in the art, including the use of adhesives, clamps, clasps and the like. The sample handling system may further comprise a plug for frictional fit sealing engagement with the sample receiving port of the funnel cap. However any device or material that effectively seals the opening of the funnel to prevent contamination or evaporation of the loaded sample is suitable for use.

Advantageously the vessels can be used in a method for enhancing detection and efficiency of acquisition of fluorescence in a sample comprising a fluorophore. The method comprises the steps of placing a sample in a vessel having walls composed of an optically transparent material and defining a volume having at least first and second dimensions. The first dimension is less than the second dimension and the ratio of volume to external surface area of the vessel is less than 1mm. Enhanced detection and efficiency of acquisition of fluorescence generated from the sample is achieved by detecting fluorescence along an axis substantially parallel to a wall along the second dimension of the vessel. In one embodiment, sample fluorescence is induced by fluorophore-excitatory illumination of the sample wherein the sample is illuminated along an axis substantially parallel to a wall along the second dimension of the vessel. In a preferred embodiment, optimum efficiency of fluorescence acquisition is achieved by fluorophore-excitatory illumination of the sample along the fluorescence detection axis (epifluorescent detection), and fluorescence is detected along an axis through a wall of the vessel having the smallest surface area, preferably along an axis through the bottom of the vessel.

The fluorescence of the biological sample may be temperature dependent. For example the vessel may contain a sample comprising nucleic acid sequences and the fluorescent entity may comprise a double strand specific dye. As the temperature of the sample is raised to the denaturation temperature, fluorescence intensity decreases. Alternatively the fluorescent entity may comprise a pair of oligonucleotide probes that hybridize to adjacent regions of a target nucleic acid sequence, wherein one of said probes is labeled with an acceptor fluorophore and the other probe is labeled with a donor fluorophore of a fluorescence energy transfer pair. In this embodiment the vessel and the sample can be heated while monitoring the fluorescence of at least one fluorophore of the fluorescence energy transfer pair.

The vessel is in the form of a capillary tube that can be used with advantage in procedures that require thermal cycling of a sample, for example, amplification of a target nucleic acid sequence by the polymerase chain reaction. In one example, the capillary vessel is formed to be inserted into a sample holder of a device used for thermal cycling or a device used to detect fluorescence. The sample holder of the device may hold only a single vessel, or the sample holder may be in the form of a carousel for holding a plurality of sample vessels.

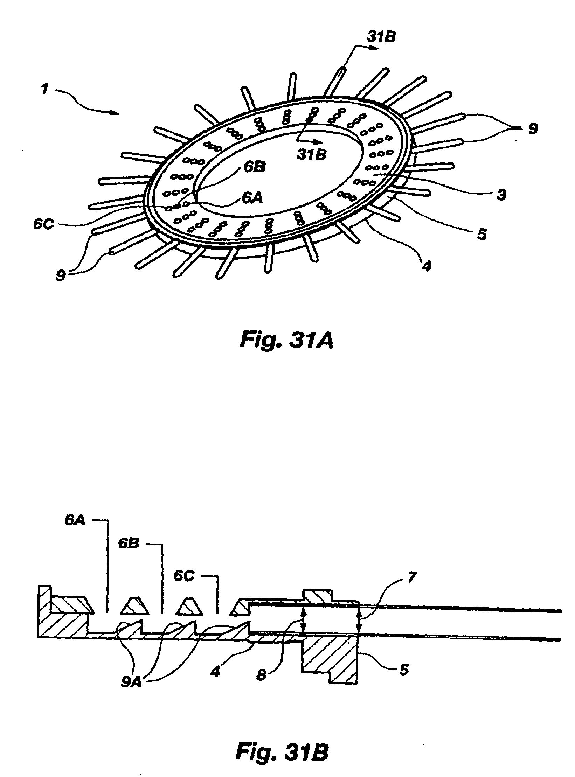

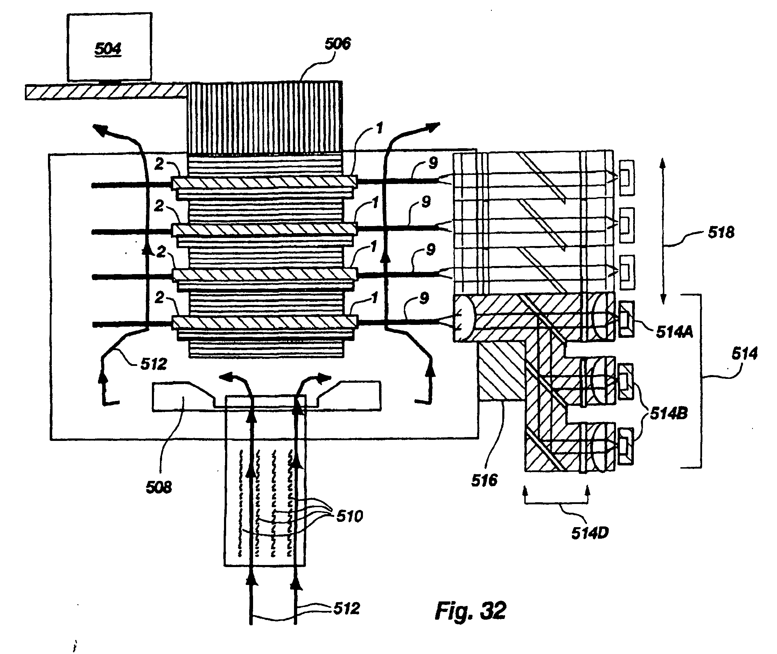

A carousel suitable for use is shown in Figures 31A&B. The carousel 1 is generally in the form of a disc 2 having a top surface 3, a bottom surface 4 and an outer edge 5 extending therebetween. The disc 2 has a plurality of sets of radially aligned sample receiving ports 6A, 68, and 6C in the top surface 3, a sample vessel port 7 in outer edge 5 and a sample passageway 8 communicating with the sample receiving ports 6A, 6B, and 6C and the respective sample vessel port 7. The carousel 1 is shown with fixed sample vessels, some of which are indicated at 9. The sample vessel port 7 and sample passageway 8 are formed for receiving and fixing sample vessel 9 to the disc 2. In one embodiment the sample vessel 9 is releasably fixed to the carousel 1 to allow the removal of the sample vessel and its replacement with another sample vessel to allow for multiple use of the carousel 1. In an alternative embodiment the sample vessels 9 are permanently fixed to, or formed as an integral component of, the disc 2. In one embodiment the sample vessel 9 is fixed to the disc 2 by frictional contact between the sample vessel 9 and at least a portion of the sample passageway 8 proximal to said sample vessel port 7. Other conventional means for fixing the sample vessel in communication with the sample vessel can be used. For example, complementary screw threads can be formed on the surface of the sample passageway 8 and on the exterior surface of the sample vessel 9. In addition adhesives or any other fixation means known to those skilled in the art can be used in accordance with the present invention to fix the sample vessel 9 to the disc 2. The top and bottom surfaces of the carousel of the present invention are preferably formed to allow multiple carousels to be stacked one on top of another so that a stack of multiple carousels can be releasably engaged with a motor drive shaft and rotated simultaneously as a unit as shown in Figure 32.

The embodiment shown in Figure 32 includes a stepper motor 504 and a drive shaft 506 which functions to hold and rotate the carousels generally indicated at 1. A chamber fan 508 is used to generate the air flow indicated by the arrows 512. A heating device 510 functions to heat the air which passes by the sample vessels 9. A fluorimeter assembly 514 includes an LED source 514A, photodiodes 514B, focusing lenses 514C, and a filter assembly 514D. A fluorimeter stepper motor 516 functions to move the fluorimeter assembly 514 in the direction of arrow 518. Those skilled in the art can readily fabricate the arrangements represented in Figure 32 using the information set forth herein.

In another embodiment (not shown) the carousel comprises a disc having a top surface, a bottom surface, an outer edge extending therebetween, a sample receiving port in the top surface, a sample vessel port in the bottom surface and a sample passageway communicating with said sample receiving port and the sample vessel port. The sample vessel port and sample passageway are formed for receiving and fixing a sample vessel to the disc. Preferably the sample vessels are held at a radially extending acute angle to the bottom surface of the disc.

In one embodiment the sample passageway of the disc comprises a first portion having a central axis substantively parallel to the top and bottom surfaces of the disc and a second portion having a central axis forming an acute angle with the top and bottom surfaces of the disc. In this embodiment the sample vessel port and sample passageway are formed for receiving and fixing a sample vessel to the disc such that the sample vessel extends from the disc at an acute angle relative to the bottom surface of the disc.

Carousel 1 is further provided with means for closing the sample receiving ports 6A, 6B, and 6C. The closure means can be a plug (not shown) that fits into the sample receiving port 6 and frictionally engages the adjacent walls of the sample passageway, or for example, adhesive backed tape, for application to the top surface to effectively seal the opening of the sample receiving port to prevent contamination or evaporation of a loaded sample. Carousel 1 is releasably engaged with a drive shaft for rotation. Any suitable engagement means well known to those of ordinary skill in the art can be used including frictional engagement, or the use of screws, bolts, locking pins or clamps. In one embodiment, the disc 2 is formed as ring having a center hole formed for receiving a drive shaft (see 506 in Figure 32). The end of the drive shaft is preferably provided with structures for holding the discs 2 in place.

The carousel 1 can be used to deliver a liquid sample to a sample vessel 9. In one embodiment the sample vessel 9 is a capillary vessel containing a predetermined mixture (for example a reagent mixture) that interacts with one or more components of the introduced sample. In accordance with one embodiment the predetermined mixture is added to the sample vessel before positioning a capillary sample vessel into the sample vessel port. Alternatively the sample vessel is prepackaged with a predetermined mixture. The predetermined mixture may comprise reagents that react or interact with the sample to produce a detectable signal or to produce a derivative product.

The sample passageway 8 of the carousel 1 are optionally provided with one or more barriers 10 that prevent a liquid sample delivered through sample receiving ports 6A, 6B, and 6C from flowing to the sample vessel port 7 absent a biasing force on said liquid sample. The term "barrier" is used herein to include any structure that impedes the free flow of a liquid sample delivered into a sample receiving port to the sample vessel port. Examples of suitable barriers for use in the sample passageway of the carousel include depressions or wells formed in the sample passageway, sample passageway narrowing projections or annular rims that extend from the surface of the sample passageway, porous membranes, directional valves, or flaps that are biased in a closed position.

The barriers are formed so that the liquid sample can overcome the barrier by application of a biasing force on a liquid sample present in the sample passageway and blocked by the barrier. The application of biasing force on the sample is preferably provided by the centripetal force generated by rotation of the carousel. Therefore, in a carousel having a plurality of sets of sample receiving ports 6A, 6B, and 6C in the top surface, each set with a corresponding sample passageway and sample vessel port, samples can be added individually to the various sample receiving ports and the barrier will localize the liquid sample and prevent the samples from flowing to the respective sample vessel ports. After all of the samples are delivered into the respective receiving ports, the carousel is rotated to deliver the samples to the respective sample vessel port and into an attached sample vessel.

Each sample passageway of the carousel communicates with a single sample vessel port and a plurality of sample receiving ports. In accordance with that embodiment, the sample passageway can optionally include a central passageway that branches to communicate with multiple sample receiving ports, or alternatively, as illustrated in Figures 31A&B multiple sample receiving ports 6A, 6B, and 6C are aligned along a common axis that extends radially from the center of the disc, each of said ports communicating through one passageway with a sample vessel received in the sample vessel port. The sample passageway can be provided with one or more barriers 9A that prevent a sample added to any one of the plurality of sample receiving ports from flowing to the sample vessel port absent a biasing force on said liquid sample. Furthermore, each sample passageway can be provided with multiple barriers, each of which require a different amount of biasing force to transfer a sample over the barrier. In accordance with this embodiment, after delivery of the samples to the respective sample receiving ports, individual samples can be selectively transferred to the sample vessel port and into the sample vessel by controlling the rate of rotation of the carousel.

For example, a first sample can be delivered into a first sample receiving port and a second sample can be delivered to a second sample receiving port wherein the first and second sample receiving ports communicate with a common passageway and the first and second sample receiving ports are each provided with a barrier that prevents flow of the respective first and second sample. The barriers allow the disc to be provided as part of a kit with predetermined amounts of selected reagents, catalysts, enzymes, oils, etc. being preloaded into the sample passageway via one or more of the sample receiving ports.

In one embodiment the barrier for the second sample receiving port is formed so that a greater biasing force must be applied to the sample delivered to the second sample receiving port to pass its associated barrier than is required for a sample delivered to the first sample receiving port to pass its associated barrier. In accordance with this embodiment, rotation of the carousel at a first rate will deliver the first sample to the sample vessel port and into the sample vessel, while the second sample is prevented from flowing to the sample vessel port and into the sample vessel. Rotation at a increased second rate will then enhance the centripetal force on the second sample and result in the delivery of the second sample to the sample vessel port and into the sample vessel. Based on this principle, different samples can be delivered to multiple sample vessel ports that communicate with a common passageway and after all the samples have been loaded, the individual samples can be delivered to the sample vessel port and into the sample vessel one at a time or simultaneously by controlling the rate of rotation of the carousel. In one embodiment a first sample, comprising a fluorophore is added to a first sample vessel port and a second sample comprising oil is delivered to the second vessel port. The carousel is rotated to deliver the first sample into the sample vessel followed by the oil. The oil (or another liquid that effectively seals the first sample within the sample vessel) functions both to decrease evaporation of the first sample and to reduce the risk of contamination of the first sample.

In one example a multiple sample carousel is used to handle multiple samples simultaneously. The carousel is a disc-like structure having a multiplicity of sample receiving ports in the top surface of the disc structure and in fluid communication with corresponding sample vessels attached to the disc. Samples added to the sample receiving ports are transferred to their corresponding sample vessels by rotation of the carousel. The carousel can also have multiple sample receiving ports communicating with each individual sample vessel. Reagents can be placed by the user into a second sample receiving port that communicates with the sample vessel for delivery to the vessel with another sample that was added to the first sample receiving port, or alternatively, predetermined reagents may be located in a second sample receiving port by the manufacturer; i.e. where the carousel, the sample vessels and the predetermined reagent are in a prepackaged form. The reagents, with the sample, are delivered to the sample vessel by rotation of the carousel. An oil for overlay of an aqueous sample may be placed in a third sample receiving port that is in liquid communication with the sample vessel (and the first and second sample receiving ports), or the oil may be added to the carousel by the manufacturer.

Alternatively, a sample, reagents and oil for sample overlays can be delivered to a single sample receiving port. The carousel can be rotated to deliver each composition or sample to the respective vessel before a second or subsequent sample or other composition is delivered to the sample receiving port.

One preferred sample vessel carousel includes three sample receiving ports preferably, but optionally, arranged in radial alignment and in fluid communication with a common sample vessel. In accordance with this embodiment, about 1 to about 5 $\mu\ell$ of an oil overlay, preferably dyed black, is present in prepackaged form, or delivered to the radially innermost sample receiving port. The oil overlay comprises mineral oil and about 0.01% to about 1% organic black dye such as waxoline® Black OBP available from Zenica, Inc. of Wilmington, DE. About 1 to abut 9 $\mu\ell$ of a reagent master mix is present in prepackaged form or is delivered to the radially outer most sample receiving port. The reagent master mix comprises a portion of, or all the necessary reaction components. A liquid sample containing the template nucleic acid to be tested is delivered manually or robotically into the radially intermediate sample receiving port. The disc is then rotated at a rate that transfers the sample to the reagent compartment, but at a rotated rate insufficient to deliver the mixture into the sample vessel. The sample and reagent can optionally be mixed by rapid changes in the rate of the rotation of the disc. The disc is then rotated at a higher rate that causes the sample and reagent mixture, but not the oil, to move into the sample vessel. The disc is then rotated at still a higher rotation rate to deliver the oil overlay to the sample vessel. The oil will overlay the aqueous sample because of its lower density and will block light passage because of its dye content. The selective transfer of oil, reagents and sample by altering the rate of carousel rotation is achieved by a combination of: 1) varying the diameter of the fluid communication passageways; 2) varying the size or shape of the physical barriers present in the fluid communication passageways; and 3) by using the dependence of centrifugal force on the varying distance (radius) of each sample receiving port from the center of the disc.

The carousel can be releasably engaged with the drive shaft and a motor (506 and 504, respectively in Figure 32) for rotating the carousel. Furthermore, individual carousels of this invention can be stacked upon one another and engaged with a drive shaft for simultaneous rotation (as shown in Figure 32). A device may be provided for monitoring the fluorescence of a sample held within a sample vessel (see 514 in Figure 32). The sample vessel comprises an optically transparent material and has walls defining a volume having at least first and second dimensions wherein the first dimension is less than the second dimension and wherein the ratio of volume to external surface area of the vessel is less than 1mm. In one embodiment the device comprises a chamber, a sample vessel holder, a light emitting source mounted in said chamber and positioned to illuminate the sample vessel along an axis substantially parallel to a wall along the second dimension of the vessel and a light detector mounted in said chamber and positioned to measure fluorescence from the sample vessel along an axis substantially parallel to a wall along the second dimension of the vessel. The light emitting source and the light detector in accordance with one embodiment are mounted on a platform that can be raised and lowered (as indicated by arrow 518 in Figure 32). In this embodiment, the light emitting source and the light detector can be positioned to measure fluorescence from the sample vessels (along an axis substantially parallel to a wall along the second dimension of the vessel) of multiple carousels when individual carousels are stacked upon one another and engaged with a drive shaft for simultaneous rotation (see Figure 32).

In one embodiment the sample vessel holder comprises a carousel for holding a plurality of capillary tubes, and the carousel is rotatably mounted in said chamber. The light emitting source is positioned to illuminate the capillary tube through the bottom of the tube and the light detector is mounted to detect fluorescence through the bottom of the capillary tube. In addition the device is provided with a stepper motor for rotating said carousel and means for coupling the carousel to the motor.

In accordance with one preferred embodiment, the chamber of the fluorescence detecting device is further provided with a heater (see 510 in Figure 32) and a fan (see 508 in Figure 32) mounted in said device and in air flow communication with the chamber, and a controller therefor, for rapidly cycling the temperature of the chamber using, at least initially, predetermined time and temperature parameters. The device is capable of conducting polymerase chain reactions in the sample vessels held by the carousel. In particular the device allows for an improved method of conducting PCR reactions because the progress of the reaction can be monitored in real time, and thus allow the adjustment of temperature and time parameters during the course of the reaction to optimize the yield and purity of the amplified target nucleic acid sequence.

Further, there is described a method of amplifying a targeted nucleic acid sequence of a biological sample comprising the steps of adding to the biological sample an effective amount of two nucleic acid probes that hybridize to adjacent regions of the target sequence, one of said probes being labeled with an acceptor fluorophore and the other probe labeled with a donor fluorophore of a fluorescence energy transfer pair such that upon hybridization of the two probes with the target sequence, the donor and acceptor fluorophores are within 0 to 15 nucleotides, and more preferably within 1-5 nucleotides of one another, amplifying the targeted nucleic acid sequence using polymerase chain reaction, illuminating the biological sample with a selected wavelength of light that is absorbed by said donor fluorophore during the polymerase chain reaction monitoring fluorescent emissions from said sample, and adjusting the temperature and time parameters in accordance with the data generated from the monitoring step.

Thus, device is described for conducting PCR reactions. The device comprises a chamber, a heater and a fan mounted in said device and in air flow communication with the chamber, carousel for holding a plurality of sample vessels. The sample vessels used in conjunction with this device comprise an optically transparent material and walls defining a volume having at least first and second dimensions wherein the first dimension is less than the second dimension and wherein the ratio of volume to external surface area of the vessel is less than 1mm. The carousel is rotatably mounted in the chamber. The device further comprises a light emitting source mounted in said chamber and positioned to illuminate at least one of the sample vessels along an axis substantially parallel to a wall along the second dimension of the vessel and a light detector mounted in said chamber and positioned to measure fluorescence from at least one of the sample vessels along an axis substantially parallel to a wall along the second dimension of the vessel. Furthermore, the device can be equipped with a stepper motor for rotating the carousel to position the respective capillary tubes held by said carousel for illumination and fluorescence detection. Monitoring the PCR reaction in real time and determining at least one reaction parameter in accordance with the detected fluorescence allows for the adjustment of the reaction conditions to optimize the reaction. In a preferred embodiment one or more values representative of the status of the reaction are displayed in a visually perceptible manner in real time.

The carousel can also be used for delivering a liquid sample to a capillary sample vessel. The carousel comprises a disc having a top surface, a bottom surface and an outer edge extending therebetween, a sample receiving port in the top surface, a sample vessel port in the outer edge and a sample passageway communicating with the sample receiving port and the sample vessel port. The sample vessel port and the sample passageway are formed for receiving and fixing a sample vessel to the disc. The method of using the carousel to deliver a liquid sample to a capillary sample vessel comprises the steps of selecting a carousel for receiving a liquid sample and holding a sample vessel, delivering the liquid sample into the sample receiving port of the carousel, positioning a capillary sample vessel into the sample vessel port, and rotating the carousel to deliver the sample into the capillary sample vessel.

In view of the foregoing, it will be appreciated that the present invention provides an apparatus for accurately submitting biological samples to thermal cycling and for quickly and accurately varying the temperature of biological samples.

Moreover, the present invention provides an apparatus which can subject a biological sample to rapid thermal cycling using air as a thermal transfer medium and which provides a system and method for performing PCR rapidly and for simultaneously monitoring the reaction. Still further, the present invention also provides a system and method for performing PCR rapidly and also continuously monitoring the reaction while it is ongoing and for adjusting the reaction parameters while the reaction is ongoing.

PROGRAMMING CODE APPENDIX A FOLLOWS

```
320   WS=(DS*10+WS*9)/10:TX=INT(WS+0.5):GOSUB 15
325   XBY(65508)=TX:GOSUB 50:IF TP>TG GOTO 340
330   IF TX<>DS*10 GOTO 320
333   GOSUB 30
335   GOSUB 50:IF TP<TG GOTO 335
340   TG=DTEMP:EB=TIME
345   WS=(6*WS+10*(TG-TP))/7 :TX=INT(WS+0.5)
350   IF TX>=0 THEN GOSUB 25:XBY(65508)=TX:XBY(65509)=4
355   IF TX<0 THEN GOSUB 20:XBY(65508)=0:XBY(65509)=4-TX
360    IF V=1 THEN IF DTIM+5>(TIME-EB) GOTO 380
370    IF DTIM<(TIME-EB) GOTO 500
380    GOSUB 50 :  GOTO 345

500    PRINT 'A',:XBY(65508)=0:TG=ATEMP
845     $(0)='sp1 200;gol nd;hf1 2;wfo =.x..;s1':GOSUB 3:         rem open
855   XBY(65509)=AS*10
860.  GOSUB 30
890   EB=TIME:WS=-AS*10
913   WS=(4*WS+20*(TG-TP))/5:TX=INT(WS+0.5)
915   IF TX>=0 THEN GOSUB 25:XBY(65508)=TX:XBY(65509)=4
918   IF TX<0 THEN GOSUB 20:XBY(65508)=0:XBY(65509)=4-TX
920    IF ATIM+1<(TIME-EB) GOTO 925
924   GOSUB 50 :  GOTO 913

925    PRINT:PRINT 'B ',:XBY(65509)=4:TG=BTEMP+1
930    $(0)='hf1 1;sp1 50;gol;wfo =x...;s1':GOSUB 3:WS=0:   REM CLOSE
935   WS=(ES*10+WS*9)/10:TX=INT(WS+0.5):GOSUB 15:GOSUB 25:XBY(65508)=TX
937   GOSUB 50:IF TP>TG-1 GOTO 970
940   IF TX<>ES*10 GOTO 935
950    GOSUB 50 :  IF TP<TG-1 GOTO 950
970   EB=TIME
980   WS=(WS+5*(TG-TP))/2 :TX=INT(WS+0.5)
983   IF TX>=0 THEN GOSUB 25:XBY(65508)=TX:XBY(65509)=4
987   IF TX<0 THEN GOSUB 20:XBY(65508)=0:XBY(65509)=4-TX
990    IF ETIM<(TIME-EB) GOTO 1000
995   GOSUB 50 :  GOTO 980
1000  CTIM=TIME-TMC : TMC=TIME :   PRINT 'CYCLE=',V,'TIME=',CTIM
1050   NEXT V

1055  XBY(65508)=0:XBY(65509)=255
1060   INPUT 'STRAND REANNEALING (Y/N) ?',$(1)
1070   IF ASC($(1),1)=78 GOTO 1290
1100  PRINT 'STRAND REANNEALING IN PROCESS...'
1110   TG=DTEMP:XBY(65509)=4
1120   WS=(DS*10+WS*9)/10:TX=INT(WS+0.5):GOSUB 15
1130  XBY(65508)=TX:GOSUB 50:IF TP>TG GOTO 1170
1140  IF TX<>DS*10 GOTO 1120
1150  GOSUB 30
1160  GOSUB 50:IF TP<TG GOTO 1160
1170  EB=TIME
1180  WS=(11*WS+10*(TG-TP))/12 :TX=INT(WS+0.5)
1185  IF TX>=0 THEN GOSUB 25:XBY(65508)=TX:XBY(65509)=4
1190  IF TX<0 THEN GOSUB 20:XBY(65508)=0:XBY(65509)=4-TX
1193    IF 5<(TIME-EB) GOTO 1200
1196   GOSUB 50 :  GOTO 1180
1200  XBY(65508)=0:XBY(65509)=255:WS=0
1210  DO:GOSUB 50:UNTIL TP<75

1220   EB=TIME
1230  WS=(WS+5*(75-TP))/3 :TX=INT(WS+0.5)
1240  IF TX>=0 THEN GOSUB 25:XBY(65508)=TX:XBY(65509)=4
1250  IF TX<0 THEN GOSUB 20:XBY(65508)=0:XBY(65509)=4-TX
1260    IF 60<(TIME-EB) GOTO 1280
1270    GOSUB 50 :  GOTO 1230
```

```
NEW
1       GOTO 60:REM                         ***I/O stepper (I=$(0), O=z(72))***
3       IF (XBY(65507).AND.64)=0 THEN Z(0)=XBY(65506) :  GOTO 3
4       DO  :  WHILE (XBY(65507).AND.128)=128
5       I=0 :  DO  : I=I+1 :  XBY(65506)=ASC($(0),I)
6       WHILE ASC(6(0),I)<>13  :  RETURN
7       DO  :  WHILE (XBY(65507).AND.64)=64
8       I=0 :  DO  : Z(I)=XBY(65506)
9       I=I+1 :  UNTIL Z(I-1)=32 :  RETURN

10      REM     ***error check***
15      IF TX<0 THEN TX=0
16      RETURN
20      IF TX<-251 THEN TX=-251
21      RETURN
25      IF TX>251 THEN TX=251
26      RETURN

30      GOSUB 50:TP1=TP:T1=TIME
32      DO:UNTIL TIME>T1+0.2
34      GOSUB 50:TP2=TP:T2=TIME
36      SL=(TP2-TP1)/(T2-T1) :IF SL<>0 THEN IT=(TG-TP2)/SL
38      PRINT USING(###.##),'SL=',SL,'IT=',IT,'AT TEMP=',TP2
40      IF IT>1 GOTO 30
42      RETURN

49      REM        ***temperature measurement***
50      E=XBY(65504):F=XBY(65505):G=XBY(65504):TP=(F*256+G+300)/38.3:   RETURN

60      CLEAR  :  STRING 150,72 :  DIM Z(72)
72      DTEMP=95:DTIM=1:DS=10
74      ATEMP=60:ATIM=1:AS=20
76      ETEMP=74:ETIM=15:ES=1:CYCLE=100
80      PRINT USING(###),'#',TAB (20),'TEMP',TAB (30),'TIME',TAB (40),'SLOPE'
82      PRINT '1) DENATURATION', TAB(20),DTEMP,TAB(30),DTIM,TAB(40),DS
84      PRINT '2) ANNEALING',TAB(20),ATEMP,TAB(30),ATIM,TAB(40),AS
86      PRINT '3) EXTENSION',TAB(20),ETEMP,TAB(30),ETIM,TAB(40),ES
88      PRINT '4) CYCLES      ',CYCLE:PRINT
110     INPUT 'TYPE # TO CHANGE OR 0 IF OK (CHOICES ARE 0,1,2,3, OR 4)?',J
114     IF J=0 THEN GOTO 170
116     ON J GOSUB ,120,130,150,160
118     PRINT:GOTO 80
120     INPUT 'ENTER DENATURATION Temp, Time, Slope: ',DTEMP,DTIM,DS
125     IF DTEMP>98 GOTO 120
126     RETURN
130     INPUT 'ENTER ANNEALING Temp, Time, Slope: ',ATEMP,ATIM,AS
140     IF AS<0 GOTO 130
142     IF AS>21 GOTO 130
145     RETURN
150     INPUT 'ENTER EXTENSION Temp, Time, Slope: ',ETEMP,ETIM,ES:RETURN
160     INPUT 'ENTER Number of Cycles:',CYCLE:RETURN
170     $(0)='msrl 500;spl 200;hfl 2;rt2 1;esr' :  GOSUB 3 :   GOSUB 7
175     FOR I=0 TO 10 :  PRINT CHR(Z(I)), :  NEXT I :  PRINT

200     $(0)='gol nd;hfl 2;wfo =.x..;sl':GOSUB 3:      rem open
210     PRINT 'WAIT FOR COOLING':XBY(65504)=3
220     DO  :  GOSUB 50 :  UNTIL TP<45
230     PRINT 'TEMP=',TP,' LOAD SAMPLES AND THEN PRESS A KEY'
240     W=GET :  IF W=0 GOTO 240

300     TMC=0 :WS=0:  CLOCK 0 :  TIME=0 :  DBY(71)=0 :  CLOCK 1
305     $(0)='hfl 1;spl 100;gol;wfo =x...;sl':GOSUB 3:       REM CLOSE
310     FOR V=1 TO CYCLE
315     PRINT 'D', : TG=DTEMP:XBY(65509)=4:IF V=1 THEN TG=DTEMP+(DS/4)
```

```
1280    XBY(65508)=0:XBY(65509)=255
1290     INPUT "ANOTHER RUN (Y/N) ?",$(1)
1300     IF ASC($(1),1)=78 GOTO 2000
1400     PRINT:$(0)="gol nd;hfl 9;wfo =.x..;el":GOSUB 3:GOTO 80
2000    $(0)="gol nd;hfl 9;wfo =.x..;el":GOSUB 3:END
```

## PROGRAMMING CODE APPENDIX B FOLLOWS

Temperature processor(IC 2) hex file (Temp24a.hex):

```
:03000000020033C8
:05000B00C0D002312508
:05001300C0D00230D74F
:05002300C0D00232BA5A
:05002B00C0D0023042CC
:20003300C2AFC2D4C2D375210075220075230D215D28AAF8175813085811 5C0D0C0F0C098
:20005300E0C08753C6FB1230897527405388CF5389F012317D78FC12305D12324912365439
:20007300C20EC20FC208759850D2ACD2AFE587D0E0D000D001D0D030E20530E4028012
206A
:20009300E602800DBF070280089038A974FA02373975C7AA75C755438740907471C082C03F
:2000B3008378187A00C000C0029037A6C082C083D003D001D005D004D002D00012309590D6
:2000D3007084C082C08378007A00D083D082E8F0907060C082C08378007A00D083D082E8C1
:2000F300F0A3EAF002013BD281D29090705CC2D57400C4C0A8C2AFC282C2907908C282
A28C
:20011300813392800D282D9F5F5F07904C282A28133D282D9F7C282D290D0A8F0E5F0C454C9
:200133000FA3F030D501222202015BD2A3C2A122D2A3D2A122C2A3D2A122C2A222D2A
22248
:20015300D2A022C2A022222221201561201481201501201 5A907055C082C08378017A00D0FF
:20017300083D082E8F078007A001230D278007A0012319D123216A8887A00C000C00278F7F2
:20019300 7A00C000C002D003D001D002D00012322D8888A8887A00C000C00278407A00C06B
:2001B30000C002D003D001D002D000123231888890232FA882AA83123235D217907471127D
:2001D30033931233D8123416D2979070 87C082C08378007A00D083D082E8F01223281 22357
:2001F3002890705AC082C08390705CE0F8A3E0FAD083D082E8F0A3EAF01225FF907087
E09D
:20021300F87A00C000C00278007A00C000C002D003D001D002D00012342FE84A6003020110
:20023300F11223281201531201439070 7EC082C08378007A00D083D082E8F0A3EAF09070B2
:20025300CC082C08378007A00D083D082E8F0A3EAF0907088C082C08378017A00D083D07B
:20027300 82E8F078007A001230D29070 74C082C08378017A00D083D082E8F0A3EAF0907025
:2002930062C082C083907060E0F8A3E0FAD083D082E8F0A3EAF0907060C082C08390708947
:2002B300C082C083907074E0F8A3E0FAC000C00278087A00C000C002D003D001D002D00099
:2002D30012348EC000C002C000C00278E87A03C000C002D003D001D002D000123508E84A0D
:2002F300700303230C278017A00C000C002D003D001D002D0001234CFC000C002D003D0018E
:20031300D002D00012350EC000C002D003D00189828B83123528D083D082E8F0A3EAF0909B
:200333007080C082C083907089C082C083907074E0F8A3E0FAC000C00278087A00C000C062
:20035300 02D003D001D002D0001234CFC000C00278017A00C000C002D003D001D002D000F0
```

:20037300123 50EC000C002C000C00278E87A03C000C002D003D001D002D000123508E84AEB
:2003930070030230C278017A00C000C002D003D001D002D0001234CFC000C002D003D001ED
:2003B300D002D000123 50EC000C002D003D00189828B83123528C000C00278D27A00C0007F
:2003D300C002D003D001D002D0001234CFC000C002907089C082C083907074E0F8A3E0FA94
:2003F300C000C00278087A00C000C002D003D001D002D0001234CFC000C00278027A00C05B
:200413 0000C002D003D001D002D000123 50EC000C002C000C00278E87A03C000C002D00336
:20043300D001D002D000123508E84A70030230C278017A00C000C002D003D001D002D00093
:200453001234CFC000C002D003D001D002D000123 50EC000C002D003D00189828B831235D1
:2004730028C000C002D003D001D002D00012350ED083D082E8F0A3EAF0907085C082C08320
:20049300907089C082C083907074E0F8A3E0FAC000C00278087A00C000C002D003D001D000
:2004B30002D00012348EC000C00278037A00C000C002D003D001D002D000123512C000C06B
:2004D30002C000C00278E87A03C000C002D003D001D002D000123508E84A70030230C27880
:2004F300017A00C000C002D003D001D002D0001234CFC000C002D003D001D002D000123582
:200513 000EC000C002D003D00189828B83123528D083D082E8F0907089C082C0839070740D
:20053300E0F8A3E0FAC000C00278087A00C000C002D003D001D002D00012348EC000C002B9
:2005530078057A00C000C002D003D001D002D000123512C000C002C000C00278E87A03C0CF
:2005730000C002D003D001D002D000123508E84A70030230C278017A00C000C002D003D060
:2005930001D002D0001234CFC000C002D003D001D002D000123 50EC000C002D003D00189C4
:2005B300828B83123528C000C00278C87A00C000C002D003D001D002D000123 52DE84A700F
:2005D300030205DA020A16907068C082C083907088E0F87A00C000C00278017A00C000C046
:2005F30002D003D001D002D000123512D083D082E8F0A3EAF0907068C082C083907068E018
:20061300F8A3E0FAC000C002907089C082C083907074E0F8A3E0FAC000C00278087A00C
0BD
:2006330000C002D003D001D002D00012348EC000C00278047A00C000C002D003D001D0025B
:20065300D0001235 12C000C002C000C00278E87A03C000C002D003D001D002D000123 50866
:20067300E84A70030230C278017A00C000C002D003D001D002D0001234CFC000C002D003A9
:20069300D001D002D000123 50EC000C002D003D00189828B83123528C000C002D003D001AB
:2006B300D002D000123537D083D082E8F0A3EAF0907068E0F8A3E0FAC000C00278007A
00DC
:2006D300C000C002D003D001D002D000123 52DE84A7003020A16907052C082C083907089A4
:2006F300C082C083907074E0F8A3E0FAC000C00278087A00C000C002D003D001D002D00055
:200713001234CFC000C00278057A00C000C002D003D001D002D000123 50EC000C002C000D9
:20073300C00278E87A03C000C002D003D001D002D000123508E84A70030230C278017A0064
:20075300C000C002D003D001D002D0001234CFC000C002D003D001D002D000123 50EC00
0CC
:200773 00C002D003D00189828B83123528D083D082E8F0A3EAF0907060E0F8A3E0FAC00009
:20079300C002907052E0F8A3E0FAC000C002D003D001D002D000123 54DE84A70030207CA09

```
:2007B300907014C082C08378FF7AFFD083D082E8F0A3EAF00207DE907014C082C083780
1AA
:2007D3007A00D083D082E8F0A3EAF0907012C082C083907089C082C083907074E0FPES8A3E
:2007F300FAC000C00278087A00C000C002D003D001D002D00012348EC000C00278067A005A
:20081300C000C002D003D001D002D000123512C000C002C000C00278E87A03C000C002D071
:2008330003D001D002D000123508E84A70030230C278017A00C000C002D003D001D002D08C
:20085300001234CFC000C002D003D001D002D000123350EC000C002D003D00189828B831202
:200873003528D083D082E8F0A3EAF0907012C082C083907012E0F8A3E0FAC000C00290708E
:2008930014E0F8A3E0FAC000C002D003D001D002D00012348ED083D082E8F0A3EAF090
7046
:2008B30068C082C083907068E0F8A3E0FAC000C002907012E0F8A3E0FAC000C002D003
D06D
:2008D30001D002D00012348ED083D082E8F0A3EAF0907068C082C083907068E0F8A3E0F
AEA
:2008F300C000C002907060E0F8A3E0FAC000C002D003D001D002D000123512D083D082E800
:20091300F0A3EAF0907068E0F8A3E0FAC000C00278007A00C000C002D003D001D002D0
005E
:20093300123553E84A70030209519070688C082C08378007A00D083D082E8F0A3EAF0907030
:2009530014E0F8A3E0FAC000C00278017A00C000C002D003D001D002D00012342FE84A7
0C7
:20097300030209BC907068E0F8A3E0FAC000C002907052E0F8A3E0FAC000C002D003D0018E
:20099300D002D000123554DE84A70030209B9907068C082C083907052E0F8A3E0FAD083D0EE
:2009B30082E8F0A3EAF00209FE907068E0F8A3E0FAC000C002907052E0F8A3E0FAC000
C0DE
:2009D30002D003D001D002D000123508E84A70030209FE907068C082C083907052E0F8A305
:2009F300E0FAD083D082E8F0A3EAF0907060C082C083907068E0F8A3E0FAD083D082E8
F0F1
:200A1300A3EAF0907060C082C083907060E0F8A3E0FAC000C00278207A00C000C002D00
3C3
:200A3300D001D002D00012348ED083D082E8F0A3EAF0907082C082C08378017A00D083
D045
:200A530082E8F0907062E0F8A3E0FAC000C002907060E0F8A3E0FAC000C002D003D001
D045
:200A730002D000012342FC000C00290705CE0F8A3E0FAC000C002907060E0F8A3E0FAC0
00F2
:200A9300C002D003D001D002D000123537123559C000C00278207A00C000C002D003D00163
:200AB300D002D000123568C000C002D003D001D002D00012356EE84A7003020AE190708241
```

:200AD300C082C08378007A00D083D082E8F0907057C082C083907089C082C083907074E0D1
:200AF300F8A3E0FAC000C00278087A00C000C002D003D001D002D00012348EC000C002
785C
:200B1300077A00C000C002D003D001D002D000123512C000C002C000C00278E87A03C0007F
:200B3300C002D003D001D002D000123508E84A70030230C278017A00C000C002D003D00199
:200B5300D002D0001234CFC000C002D003D001D002D00012350EC000C002D003D001898
27D
:200B73008B83123528D083D082E8F0120DEF121231907074C082C083907074E0F8A3E0FA43
:200B9300C000C00278017A00C000C002D003D001D002D000123512D083D082E8F0A3EA
F0B2
:200BB300907074E0F8A3E0FAC000C0029070B9C082C08378027A00C000C002C000C0027859
:200BD300E87A03C000C002D003D001D002D000123508E84A70030230C278017A00C000C07A
:200BF30002D003D001D002D0001234CFC000C002D003D001D002D00012350EC000C002
D016
:200C130003D00189828B83123528C000C002D003D001D002D00012352DC000C00290708720
:200C3300E0F87A00C000C00278007A00C000C002D003D001D002D00012342FC000C002D04C
:200C53003D001D002D000123575E84A7003020291907088C082C083907088E0F87A00C06E
:200C730000C00278017A00C000C002D003D001D002D0001235127A00D083D082E8F0907094
:200C930088E0F87A00C000C0029070B9C082C08378007A00C000C002C000C00278E87A0304
:200CB300C000C002D003D001D002D000123508E84A70030230C278017A00C000C002D00329
:200CD300D001D002D0001234CFC000C002D003D001D002D00012350EC000C002D003D0
0136
:200CF30089828B83123528C000C002D003D001D002D000123570C000C0029070B7E0F87A73
:200D13000C000C00278007A00C000C002D003D001D002D00012342FC000C002D003D001E9
:200D3300D002D000123575E84A7003020270120150907087C082C08378007A00D083D08216
:200D5300E8F0D21778FF7A00E81233A41233D81234167B007A00123319D1225FF12318FC0EA
:200D730000C00278EE7A02C000C002D003D001D002D00012354DC000C0029070B7E0F87A05
:200D930000C000C00278017A00C000C002D003D001D002D00012342FC000C002D003D00168
:200DB300D002D000123575E84A7003020D6C90707BE0F87A00C000C00278017A00C000C0D4
:200DD3000D002D003D001D002D00012342FE84A600302023412014B1201560201DD90708E06D
:200DF300F87A00C000C00278007A00C000C002D003D001D002D00012352DC000C0029070B3C
:200E130085E0F87A00C000C00278007A00C000C002D003D001D002D000123553C000C00286
:200E3300D003D001D002D000123570E84A7003020E6D907054C082C08378017A00D083D093
:200E5300B2E8F090700CC082C08378047A00D083D082E8F0A3EAF0020E92907054C082C0AC
:200E73008378007A00D083D082E8F090700CC082C08378087A00D083D082E8F0A3EAF0905B
:200E93007080E0F8A3E0FAC000C00278007A00C000C002D003D001D002D0001235ADE84
AF8

:200EB30007003020ECC90700CC082C083780C7A00D083D082E8F0A3EAF0122328907085E085
:200ED300F87A00C000C00278007A00C000C002D003D001D002D00012342FE84A7003020F26
:200EF30005907085C082C08378C87A00D083D082E8F090705EC082C08390705CE0F8A3E0FF
:200F1300FAD083D082E8F0A3EAF090705CE0F8A3E0FAC000C002907060E0F8A3E0FAC00022
:200F3300C002D003D001D002D00012352DE84A7003020FC0907068C082C08390705CE0F88B
:200F5300A3E0FAC000C002907060E0F8A3E0FAC000C002D003D001D002D000123537D08331
:200F7300D082E8F0A3EAF090706AC082C08378FF7AFFD083D082E8F0A3EAF0907068E0F89E
:200F9300A3E0FAC000C00278407A00C000C002D003D001D002D00012352DE84A7003020F1B
:200FB300BA12013E020FBD120143020FD790706AC082C083780I7A00D083D082E8F0A3EA1B
:200FD300F0120143907057E0F87A00C000C00278037A00C000C002D003D001D002D00012BE
:200FF300342FE84A7003021038907085C082C08378C87A00D083D082E8F0907072C082C0D7
:2010130008378017A00D083D082E8F0A3EAF090700CC082C083780x7A00D083D082E8F0A3A2
:20103300EAF0021230907060E0F8A3E0FAC000C00278007A0AC000C002D003D001D002D084
:2010530000012352DE84A70030212C907060E0F8A3E0FAC000C00278807A0CC000C002D01E
:2010730003D001D002D00012352DE84A70030210D890706AE0F8A3E0FAC000C00278017AB0
:2010930000C000C002D003D001D002D00012342FE84A70030210C19070702C082C083780217
:2010B3007A00D083D082E8F0A3EAF00210D59070702C082C083780E7A00D083D082E8F0A3AB
:2010D300EAF0021129907060AE0F8A3E0FAC000C00278017A00C000C002D003D001D002D0EB
:2010F30000012342FE84A7003021159070702C082C08378067A00D083D082E8F0A3EAF002B0
:20111300112990702C082C083780C7A00D083D082E8F0A3EAF00211F79070060E0F8A3E0CE
:2011330FAC000C00278807A07C000C002D003D001D002D000123552DF84A70030211A6907D
:201153007060AE0F8A3E0FAC000C00278017A00C000C002D003D001D002D00012342FE84A69
:2011730070030211BF9070702C082C083780A7A00D083D082E8F0A3EAF00211A39070702C072
:20119300082C08378027A00D083D082E8F0A3EAF00211F79070706AE0F8A3E0FAC000C00278C6
:2011B300017A00C000C002D003D001D002D00012342FE84A700302111E39070702C082C083D2
:2011D300780A7A00D083D082E8F0A3EAF00211F79070702C082C083780I7A00D083D082E885

:2011F300F0A3EAF0907085E0F87A00C000C002780A7A00C000C002D003D001D002D000
1240

:201213003553E84A7003021230907072C082C08378017A00D083D082E8F0A3EAF022907044

:2012330054E0F87A00C000C00278017A00C000C002D003D001D002D00012342FE84A70039E

:201253000212591 2013E907057E0F87A00C000C00278027A00C000C002D003D001D002D0D6

:2012730000123582E84A70030212811225F4907057E0F87A00C000C00278037A00C000C08D

:2012930002D003D001D002D00012342FE84A70030212CF907062C082C08390705CE0F8A338

:2012B300E0FAD083D082E8F0A3EAF0907056C082C08378017A00D083D082E8F0D21790
374C

:2012D300B5123588907088E0F87A00C000C00278207A00C000C002D003D001D002D000122F

:2012F3003512E81233A4907074E0F8A3E0FAC000C00278207A00C000C002D003D001D0026E

:20131300D000123512E81233A41233D8123416907082E0F87A00C000C00278017A00C0003E

:201333000C002D003D001D002D00012342FE84A700302149412189A122328907068C082C043

:2013530083907060E0F8A3E0FAC000C00290705CE0F8A3E0FAC000C002D003D001D002
D047

:2013730000123537D083D082E8F0A3EAF090706AE0F8A3E0FAC000C00278FF7AFFC000
C031

:2013930002D003D001D002D00012342FE84A70030213D5907068C082C083907068E0F8A31E

:2013B300E0FAC000C00278FF7AFFC000C002D003D001D002D00012348ED083D082E8F0
A312

:2013D300EAF0907068E0F8A3E0FAC000C00290700CE0F8A3E0FAC000C002D003D001D
00288

:2013F300D000123 52DC000C002907087E0F87A00C000C00278017A00C000C002D003D001A0

:20141300D002D00012342FC000C002D003D001D002D00012356EC000C00290707EE0F8A
3AA

:20143300E0FAC000C00278007A00C000C002D003D001D002D000123596C000C002D003D081

:2014530001D002D00012356EC000C00290707CE0F8A3E0FAC000C00278007A00C000C002D8

:20147300D003D001D002D000123596C000C002D003D001D002D00012356EE84A600302130F

:20149300471201 50907054E0F87A00C000C00278017A00C000C002D003D001D002D000129A

:2014B300342FE84A70030214BF120143907072C082C08378017A00D083D082E8F0A3EAF002

:2014D300907085C082C08378007A00D083D082E8F0907054C082C08378007A00D083D08210

:2014F300E8F090706AC082C08378017A00D083D082E8F0A3EAF0907080E0F8A3E0FAC0
0090

:20151300C00278007A00C000C002D003D001D002D00012354DE84A7003021 5FD78007A00FD

:2015330012 30D278007A0012319D12232812189A12359CC000C002907080E0F8A3E0FAC097

:2015530000C002D003D001D002D000123508C000C002907087E0F87A00C000C00278017A51

:201 5730000C000C002D003D001D002D000123596C000C002D003D001D002D00012356EC076

:2015930000C00290707EE0F8A3E0FAC000C00278007A00C000C002D003D001D002D0001255
:2015B3003596C000C002D003D001D002D000123 56EC000C00290707CE0F8A3E0FAC000C
0FD
:2015D3000278007A00C000C002D003D001D002D000123596C000C002D003D001D002D00097
:2015F30012356EE84A600302153D907057E0F87A00C000C00278007A00C000C002D003D0F8
:2016130001D002D000123 52DC000C00290707EE0F8A3E0FAC000C00278007A00C000C00255
:20163300D003D001D002D000123596C000C002D003D001D002D00012356EC000C002907075
:2016530087E0F87A00C000C00278017A00C000C002D003D001D002D00012342FC000C0026A
:20167300D003D001D002D00012356EE84A7003021863907057E0F87A00C000C00278027A1B
:2016930000C000C002D003D001D002D00012342FC000C002907057E0F87A00C000C00278D5
:2016B300037A00C000C002D003D001D002D00012342FC000C002907056E0F87A00C000C0B3
:2016D3000278017A00C000C002D003D001D002D00012342FC000C002D003D001D002D00
0FD
:2016F30012356EC000C002D003D001D002D000123575E84A70030217C0907057C082C08344
:2017130078017A00D083D082E8F012232812189A907087E0F87A00C000C00278017A00C017
:2017330000C002D003D001D002D00012342FC000C00290707EE0F8A3E0FAC000C0027800CA
:201753007A00C000C002D003D001D002D000123596C000C002D003D001D002D00012356
EDA
:20177300C000C00290707CE0F8A3E0FAC000C00278007A00C000C002D003D001D002D00
0C7
:20179300123596C000C002D003D001D002D00012356EE84A600302171D90707CC082C08310
:2017B30078007A00D083D082E8F0A3EAF0907057C082C08378017A00D083D082E8F01225A7
:2017D300F412232812189A907087E0F87A00C000C00278017A00C000C002D003D001D0029B
:2017F300D00012342FC000C00290707EE0F8A3E0FAC000C00278007A00C000C002D003D
0A3
:2018130001D002D000123596C000C002D003D001D002D00012356EC000C00290707CE0F8E2
:20183300A3E0FAC000C00278007A00C000C002D003D001D002D000123596C000C002D00
3AA
:20185300D001D002D00012356EE84A600302 17D490707EC082C08378007A00D083D082E849
:20187300F0A3EAF090707CC082C08378007A00D083D082E8F0A3EAF078007A001230D27
87D
:20189300007A0012319D22907057E0F87A00C000C00278037A00C000C002D003D001D002A1
:2018B300D00012342FE84A7003021993907068C082C083907062E0F8A3E0FAC000C00290C7
:2018D300705CE0F8A3E0FAC000C002D003D001D002D000123537D083D082E8F0A3EAF
09004
:2018F3007068C082C083907068E0F8A3E0FAC000C00290706AE0F8A3E0FAC000C002D00325

:20191300D001D002D00012348ED083D082E8F0A3EAF0907068E0F8A3E0FAC000C00278
01BB

:201933007A00C000C002D003D001D002D000123553C000C002907056E0F87A00C000C0020C

:2019530078007A00C000C002D003D001D002D00012342FC000C002D003D001D002D000126B

:20197300356EE84A7003021990907056C082C08378017A00D083D082E8F01225F4021CBA13

:20199300907085E0F87A00C000C00278007A00C000C002D003D001D002D00012342FC000EC

:2019B300C002907085E0F87A00C000C00278C87A00C000C002D003D001D002D00012342F02

:2019D300C000C002D003D001D002D000123575E84A7003021A04907062C082C08390706064

:2019F300E0F8A3E0FAD083D082E8F0A3EAF0021CBA907058C082C08312359CC000C002
90DB

:201A13007085E0F87A00C000C002D003D001D002D0001234CFC000C00278207A00C000C07B

:201A330002D003D001D002D0001234CFD083D082E8F0A3EAF0907058C082C083907058E027

:201A5300F8A3E0FAC000C002780A7A00C000C002D003D001D002D0001231AAD083D08
2E83E

:201A7300F0A3EAF0907068C082C083907058E0F8A3E0FAD083D082E8F0A3EAF0907058
C03A

:201A930082C08312318FC000C00278647A00C000C002D003D001D002D00012350EC000C027

:201AB3000278197A00C000C002D003D001D002D0001231AAC000C002907085E0F87A00C038

:201AD30000C002D003D001D002D0001234CFC000C00278087A00C000C002D003D001D0
0262

:201AF300D0001234CFC000C00278647A00C000C002D003D001D002D0001231AAD083D0
82BC

:201B1300E8F0A3EAF0907068C082C083907068E0F8A3E0FAC000C002907058E0F8A3E0F
A84

:201B3300C000C002D003D001D002D000123550ED083D082E8F0A3EAF0907068E0F8A3E0
FABE

:201B5300C000C00278007A10C000C002D003D001D002D00012352DE84A7003021B8590706B

:201B730068C082C08378007A10D083D082E8F0A3EAF0907068C082C083907068E0F8A3E0B9

:201B9300FAC000C00290706AE0F8A3E0FAC000C002D003D001D002D00012348ED083D0
82B6

:201BB300E8F0A3EAF09907062C082C083907068E0F8A3E0FAC000C00290705EE0F8A3E0
FAE4

:201BD300C000C002D003D001D002D000123512D083D082E8F0A3EAF090706AE0F8A3E0
FA18

:201BF300C000C00278017A00C000C002D003D001D002D00012342FE84A7003021C679070F6

:201C13062E0F8A3E0FAC000C002907060E0F8A3E0FAC000C002D003D001D002D00012
35B4

:201C33002DE84A7003021C64907062C082C083907060E0F8A3E0FAD083D082E8F0A3EA
F0A7
:201C5300907085C082C08378C87A00D083D082E8F0021CBA907062E0F8A3E0FAC000C0
021F
:201C7300907060E0F8A3E0FAC000C002D003D001D002D000123553E84A7003021CBA907
0BD
:201C930062C082C083907060E0F8A3E0FAD083D082E8F0A3EAF0907085C082C08378C87
AD7
:201CB30000D083D082E8F0907016C082C083907084E0F87A00C000C002C000C002781E7A0F
:201CD30000C000C002D003D001D002D000123508E84A70030230C278027A00C000C002D0FB
:201CF30003D001D002D00001234CFC000C002D003D001D002D000123550EC000C00290705C4B
:201D1300E0F8A3E0FAC000C00290705AE0F8A3E0FAC000C002D003D001D002D000123
537E4
:201D3300D083D082E8F0A3EAF0907070C082C08378007A00D083D082E8F0A3EAF09070
763F
:201D5300C082C08378007A00D083D082E8F0A3EAF090700AC082C08378137A00D083D0
82C6
:201D7300E8F0A3EAF0907008C082C083780J7A00D083D082E8F0A3EAF0907070C082C0
838C
:201D9300907070E0F8A3E0FAC000C002907016C082C083907076E0F8A3E0FAC000C002C041
:201DB30000C002781E7A00C000C002D003D001D002D000123508E84A70030230C278027A9A
:201DD30000C000C002D003D001D002D00001234CFC000C002D003D001D002D000123550E
C036
:201DF30000C002D003D00189828B831235A5C000C002D003D001D002D00012351 2D083D021
:201E130082E8F0A3EAF0907076C082C083907076E0F8A3E0FAC000C002907008E0F8A3E
02D
:201E3300FAC000C002D003D001D002D000123512D083D082E8F0A3EAF0907076E0F8A3
E0A9
:201E5300FAC000C00290700AE0F8A3E0FAC000C002D003D001D002D0009070081235AB
E8EA
:201E73004A6003021D8C907070C082C083907070E0F8A3E0FAC000C002907072E0F8A3E0EE
:201E9300FAC000C002D003D001D002D00012348EC000C00278027A00C000C002D003D00
1FD
:201EB300D002D00012 35B7D083D082E8F0A3EAF0907080E0F8A3F0FAC000C00278007A
002C
:201ED300C000C002D003D001D002D000123596E84A7003021F18907070C082C08390707007

44

:201EF300E0F8A3E0FAC000C00278027A00C000C002D003D001D002D0001235B7D083D08299

:201F1300E8F0A3EAF0907054E0F87A00C000C00278017A00C000C002D003D001D002D00076

:201F330012342FC000C002907085E0F87A00C000C00278007A00C000C002D003D001D00254

:201F5300D00012342FC000C00212359CC000C00278017A00C000C002D003D001D002D00087

:201F7300123S4DC000C002D003D001D002D000123S6EC000C002D003D001D002D000123S2E

:201F930075E84A700302225B907054E0F87A00C000C00278017A00C000C002D003D001D084

:201FB30002D00012342FE84A7003021FEB907010C082C08378147A00D083D082E8F0A3EA71

:201FD300F090700EC082C08378F67AFFD083D082E8F0A3EAF0022013907010C082C0837848

:201FF300287A00D083D082E8F0A3EAF090700EC082C08378D87AFFD083D082E8F0A3EAF0DC

:2020130090706CC082C083907062E0F8A3E0FAC000C00290705CE0F8A3E0FAC000C002D080

:20203300D001D002D000123537D083D082E8F0A3EAF0907068C082C08390705CE0F8A3DB

:20205300E0FAC000C002907070E0F8A3E0FAC000C002D003D001D002D000123512C000C0AB

:2020730002907062E0F8A3E0FAC000C002D003D001D002D000123537C000C00278027A00D8

:20209300C000C002D003D001D002D0001235B7D083D082E8F0A3EAF090706CC082C08390EC

:2020B300706CE0F8A3E0FAC000C002907068E0F8A3E0FAC000C002D003D001D002D00012C3

:2020D3003537D083D082E8F0A3EAF090706CE0F8A3E0FAC000C002907010E0F8A3E0FAC01F

:2020F3000C002D003D001D002D00012352DE84A70030221209 0706CC082C083907010E088

:20211300F8A3E0FAD083D082E8F0A3EAF090706CE0F8A3E0FAC000C00290700EE0F8A3E091

:20213300FAC000C002D003D001D002D000123553E84A7003022162 90706CC082C08390701S

:2021S300EE0F8A3E0FAD083D082E8F0A3EAF090706EC082C083907062E0F8A3E0FAC000A5

:20217300C00278117A00C000C002D003D001D002D0001235B7C000C00278467A00C000C087

:2021930002D003D001D002D000123537C000C00290706CE0F8A3E0FAC000C002D003D001FD

:2021B300D002D000123512D083D082E8F0A3EAF0907083C082C08378007A00D083D082E890

:2021D300F0907083C082C083907083E0F87A00C000C00278017A00C000C002D003D001D0B4

:2021F30002D0001235127A00D083D082E8F0907083E0F87A00C000C00290706EE0F8A3E08A

:20221300FAC000C002D003D001D002D000123568E84A70030222301201 4D02223312015027

45

:20223300907083E0F87A00C000C00278C87A00C000C002D003D001D002D000123582E84AB7
:2022530070030221D40222A990705CE0F8A3E0FAC000C002907070E0F8A3E0FAC000C002BA
:20227300D003D001D002D000123512C000C002907062E0F8A3E0FAC000C002D003D001D07D
:2022930002D000123568E84A70030222A612014D0222A91201501225FF90705AC082C08396
:2022B30090705CE0F8A3E0FAD083D082E8F0A3EAF0907084C082C083907084E0F87A00C0C1
:2022D30000C00278017A00C000C002D003D001D002D0001235127A00D083D082E8F090701E
:2022F30084E0F87A00C000C00278137A00C000C002D003D001D002D000012352DE84A70038D
:20231300022327907084C082C08378007A00D083D082E8F0221200FA1200FA22907087E023
:20233300F87A00C000C00278017A00C000C002D003D001D002D000012342FE84A700302259A
:20235300F1907057E0F87A00C000C00278017A00C000C002D003D001D002D000012342FE836
:202373004A700302239090707CC082C08378017A00D083D082E8F0A3EAF00225F19070577B
:20239300E0F87A00C000C00278027A00C000C002D003D001D002D000012342FE84A7003027E
:2023B30023B81225F4907057E0F87A00C000C00278037A00C000C002D003D001D002D0001C
:2023D30012342FE84A7003022 5F1907056C082C08378007A00D083D082E8F0907064C082C8
:2023F300C083907089C082C083907074E0F8A3E0FAC000C00278087A00C000C002D003D00F
:20241300001D002D00012348EC000C00278037A00C000C002D003D001D002D0001235 12C0DA
:2024330000C002C000C00278E87A03C000C002D003D001D002D000123508E84A700302307A
:20245300C278017A00C000C002D003D001D002D0001234CFC000C002D003D001D002D0000F
:2024730001235 0EC000C002D003D00189828B83123528C000C00278207A00C000C002D0035D
:20249300D001D002D00012348EC000C002780A7A00C000C002D003D001D002D00012 35B79E
:2024B300D083D082E8F0A3EAF0907064C082C083907064E0F8A3E0FAC000C00290706A E0A1
:2024D300F8A3E0FAC000C002D003D001D002D00012348ED083D082E8F0A3EAF0907062 C0BC
:2024F30082C083907062E0F8A3E0FAC000C002907064E0F8A3E0FAC000C002D003D001 D01C
:20251300 02D000123512D083D082E8F0A3EAF090706AE0F8A3E0FAC000C00278017A00 C08F
:2025330000C002D003D001D002D000012342FE84A700302259E907062E0F8A3E0FAC000C06A
:2025530002907060E0F8A3E0FAC000C002D003D001D002D000012352DE84A700302259B907E
:20257300062C082C083907060E0F8A3E0FAD083D082E8F0A3EAF0907057C082C0837801ED
:202593007A00D083D082E8F00225F1907062E0F8A3E0FAC000C002907060E0F8A3E0FAC06B

46

EP 1 442 794 B1

:2025B30000C002D003D001D002D000123553E84A70030225F1907062C082C083907060E082

:2025D300F8A3E0FAD083D082E8F0A3EAF0907057C082C08378017A00D083D082E8F002

35F6

:2025F300EFC29775F005D5F0FDD29722907086C082C08312361A750200D083D082E8F090D8

:202613007086E0F87A00C000C00278087A00C000C002D003D001D002D00012342FE84A7004

:2026330003022D1E907068C082C08378007A00D083D082E8F0A3EAF090707AC082C08378E7

:20265300007A00D083D082E8F0A3EAF0907006C082C08378077A00D083D082E8F0A3EA

F075

:20267300907004C082C08378017A00D083D082E8F0A3EAF0907086C082C08312361A7502ED

:2026930000D083D082E8F0907086E0F87A00C000C00278007A00C000C002D003D001D00266

:2026B300D00012366CE84A7003022687907068C082C083907068E0F8A3E0FAC000C0029073

:2026D3007086E0F87A00C000C002D003D001D002D0001235 12D083D082E8F0A3EAF090

7084

:2026F30089C082C08390707AE0F8A3E0FAC000C002C000C00278E87A03C000C002D003

D0E4

:2027130001D002D000123508E84A70030230C278017A00C000C002D003D001D002D000124E

:2027330034CFC000C002D003D001D002D00012350EC000C002907086E0F87A00C000C0028A

:2027530078207A00C000C002D003D001D002D0001235377A00D083D082E8F090707AC0825B

:20277300C08390707AE0F8A3E0FAC000C002907004E0F8A3E0FAC000C002D003D001D0

0261

:20279300D000123512D083D082E8F0A3EAF090707AE0F8A3E0FAC000C002907006E0F8

A331

:2027B300E0FAC000C002D003D001D002D0009070041235ABE84A6003022687907066C08282

:2027D300C083907068E0F8A3E0FAC000C00278D27A00C000C002D003D001D002D00012

3591

:2027F300B7D083D082E8F0A3EAF0907066C082C083907066E0F8A3E0FAC000C00278D2

7A29

:2028130000C000C002D003D001D002D00012348ED083D082E8F0A3EAF0907068C082C08322

:20283300907068E0F8A3E0FAC000C002907066E0F8A3E0FAC000C002D003D001D002D0

00C3

:20285300123537D083D082E8F0A3EAF0907086C082C08312361A750200D083D082E8F090FC

:202873007086E0F87A00C000C00278007A00C000C002D003D001D002D00012366CE84A706B

:202893000302285F907086C082C083907086E0F87A00C000C00278207A00C000C002D003CD

:2028B300D001D002D0001235377A00D083D082E8F0907086E0F87A00C000C002907068E07B

:2028D300F8A3E0FAC000C002D003D001D002D00012366CE84A70030228FBD2179037B7

12B1

:2028F30035881233D8123416907068E0F8A3E0FAC000C002907086E0F87A00C000C002D026

```
:2029130003D001D002D00012342FE84A7003022637D2179037B91235881233D81234169074
:20293300707&C082C08378017A00D083D082E8F0A3EAF0907068C082C08378007A00D083F8
:20295300D082E8F0A3EAF090707AC082C08378007A00D083D082E8F0A3EAF0907002C0
828E
:20297300C08378077A00D083D082E8F0A3EAF0907000C082C08378017A00D083D082E8F019
:20299300A3EAF0907086C082C08312361A750200D083D082E8F0907086E0F87A00C000C0EE
:2029B3000278007A00C000C002D003D001D002D00012366CE84A70030229969070688C08284
:2029D300C083907068E0F8A3E0FAC000C002907086E0F87A00C000C002D003D001D002
D0C2
:2029F30000012351 2D083D082E8F0A3EAF0907089C082C083907078E0F8A3E0FAC000C00214
:202A130078087A00C000C002D003D001D002D00012348EC000C00290707AE0F8A3E0FA
C0FC
:202A330000C002D003D001D002D000123512C000C002C000C00278E87A03C000C002D003EC
:202A5300D001D002D000123508E84A70030230C278017A00C000C002D003D001D002D0004D
:202A73001234CFC000C002D003D001D002D00012350EC000C002907086E0F87A00C000C037
:202A93000278207A00C000C002D003D001D002D0001235377A00D083D082E8F090707AC098
:202AB30082C08390707AE0F8A3E0FAC000C002907000E0F8A3E0FAC000C002D003D001
D0A2
:202AD30002D0001235 12D083D082E8F0A3EAF090707AE0F8A3E0FAC000C002907002E0
F893
:202AF300A3E0FAC000C002D003D001D002D00090700012 35ABE84A6003022996907066C010
:202B130082C083907068E0F8A3E0FAC000C00278D27A00C000C002D003D001D002D0001200
:202B330035B7D083D082E8F0A3EAF0907066C082C083907066E0F8A3E0FAC000C00278
D22A
:202B53007A00C000C002D003D001D002D00012348ED083D082E8F0A3EAF0907068C082
C0E8
:202B730083907068E0F8A3E0FAC000C002907066E0F8A3E0FAC000C002D003D001D002
D0FD
:202B930000012353 7D083D082E8F0A3EAF0907086C082C08312361A750200D083D082E8F049
:202BB300907086E0F87A00C000C00278007A00C000C002D003D001D002D00012366CE84A08
:202BD3007003022BA0907086C082C083907086E0F87A00C000C00278207A00C000C002D0D9
:202BF30003D001D002D0001235377A00D083D082E8F0907068E0F8A3E0FAC000C002907098
:202C130086E0F87A00C000C002D003D001D002D00012342FE84A7003022C6ED2179037BBE0
:202C33001235881233D8123416907078C082C083907078E0F8A3E0FAC000C00278017A00FA
:202C5300C000C002D003D001D002D000123512D083D082E8F0A3EAF0022C7CD2179037
BD2F
:202C73001235881233D8123416907078E0F8A3E0FAC000C002907089C082C08378027A00A8
```

```
:202C9300C000C002C000C00278E87A03C000C002D003D001D002D000123508E84A70030282
:202CB30030C278017A00C000C002D003D001D002D0001234CFC000C002D003D001D002
D077
:202CD3000012350EC000C002D003D00189828B83123528C000C002D003D001D002D0001204
:202CF300352DE84A7003022946907087C082C08378017A00D083D082E8F0907086C082C045
:202D13008378007A00D083D082E8F0907086E0F87A00C000C00278097A00C000C002D00304
:202D3300D001D002D000012342FE84A7003022D5790707EC082C08378017A00D083D082E8EA
:202D5300F0A3EAF0907086E0F87A00C000C002780A7A00C000C002D003D001D002D000
12C3
:202D7300342FE84A7003022DDF907087C082C083907087E0F87A00C000C00278017A00C010
:202D930000C002D003D001D002D0001235127A00D083D082E8F0907087E0F87A00C000C06F
:202DB3000278017A00C000C002D003D001D002D000012352DE84A7003022DDF907087C08253
:202DD300C08378007A00D083D082E8F0907086E0F87A00C000C002780B7A00C000C002D085
:202DF30003D001D002D000012342FE84A7003022E15907086C082C08378007A00D083D08249
:202E1300E8F0907086E0F87A00C000C002780C7A00C000C002D003D001D002D00012342F32
:202E3300E84A7003022F03907086C082C08312361A750200D083D082E8F0907086E0F87A6D
:202E530000C000C00278007A00C000C002D003D001D002D00012366CE84A7003022E3A90D0
:202E73007086C082C083907086E0F87A00C000C00278207A00C000C002D003D001D002D090
:202E930000012353377A00D083D082E8F0907087E0F87A00C000C00278007A00C000C002D00B
:202EB30003D001D002D00012342FE84A7003022EF2907086E0F87A00C000C00278017A0000
:202ED300C000C002D003D001D002D00012342FE84A7003022EEF120153022EF2120156905D
:202EF3007086C082C08378007A00D083D082E8F0907086E0F87A00C000C002780D7A00C0BC
:202F130000C002D003D001D002D00012342FE84A7003022FF7907086C082C08312361A7572
:202F33000200D083D082E8F0907086E0F87A00C000C00278007A00C000C002D003D001D0BD
:202F530002D00012366CE84A7003022F28907087E0F87A00C000C00278007A00C000C0020B
:202F7300D003D001D002D00012342FE84A7003022FE6907055C082C083907086E0F87A0015
:202F9300C000C00278207A00C000C002D003D001D002D00012353377A00D083D082E8F090BD
:202FB3007055E0F87A00C000C00278007A00C000C002D003D001D002D00012342FE84A7094
:202FD30003022FDD120159022FE612015A1201481201569070860C82C08378007A00D083C9
:202FF300D082E8F0907086E0F87A00C000C002780E7A00C000C002D003D001D002D0001260
:2030130034 2FE84A7003023022D907086C082C08378007A00D083D082E8F0907086C082C034
:20303300837 8007A00D083D082E8F0220236728587E020E5FAD0D0D0E0D0E0D2D574AA
759A
:203053 00F055900033C082C0833253883F53892F438920438780888D758B00D28E2275C770
:20307300AA75C755D2BF2212307175C7AA75C755438704D21D2275C7AA75C7555387FB
C238
:2030930 01D2289828B83E493C082C08388828A83F06017DC09E4F0158115810230CAA3A81F
```

49

:2030B30082AA83D083D082A380DC15811581229037BF74460237399037D374500237398852

:2030D300298A2A2230191C301705D21AD0D0222008FDC082C08390748BC2D5D21BC21A
C026

:2030F300D0800ED0D032C082C083907489D2D5C0D0C0E0C0F0C000C001C002C003C004
C06A

:2031130005C006C007D215E0F8A3E0F583850082E473301052C0E085278CE52804B4C808F3

:20313300E40529B52902052AF528301138201335201 20EE52BB52A07E52CB5290280025064

:2031530024201 71F20081CE5C620E217D0E0D213C212C211C082C083903173C082C083322E

:203173000230FDD212D0E0D0D032E4F529F52AF528F52CF52BC211C212C213228528E07588

:20319300F005A485E00085F0022275010575030012 31AA85002822C3E94B7005D30232065D

:2031B300227F007E007C007D0075F0101231EFEE33FEEF33FFC3EF9B40117005C3EE994060

:2031D3000AC3EE99FEEF9BFFD38001C31231F7D5F0DA8C008D028F038E01C322C3E83
3F81A

:2031F300EA33FA22EC33FCED33FD229037F374140237399037E4740A0237399038 10742866

:20321300023739538 9F0D21043A882D28C22907489E8F0A3EAF043A88222E859F822E84991

:20323300F82290748BE8F0A3EAF0D219D2BCC2BAD2B943A884229074F87475F0A37400
F090

:203253009074F67475F0A37400F0751000751101C208C21722C2ACC082C083F89074F6E0EB

:20327300F9A3E0F5828983E8F0A3E582B45003907500A882E5829074F7F088828983A3E534

:2032930082B45003907500A88290 74F8A3E0B50008D2AC0000C2AC80F4200804D208D29956

:2032B300D083D082D2AC22209846C299300E03023390C0E0C082C083C000C001C002C0032C

:2032D3009074F7E0FA9074F8E0F9A3E0FBB50205C208023382850382850183E0F599A3E56D

:2032F30082B45003907500E5829074F9F0023382C0E0E599C298201 50302338EB4030620D2

:20331300F030233EEB41304D20E806FB41109300E69D299C20E8063C082C083C000C00132

:20333300C002C003FA90749FE0F890749DAB829074A0E0F9A3E0F5828983A3E582B503036A

:2033530090748DEAF0E583A9829074A0F0E9A3F0B5001C90749EE0F5838882A3E582B503B5

:203373000390748DE583A98290749EF0E9A3F0D003D002D001D000D083D082D0E0D0D0
3298

:20339300E060061233A4A380F72008FD222008FD221233AB12326822791 1B42000400207DE

:2033B30022B40D03770122B408021722B40A07D0E0D0E00233DD22C000C0E0123268D0E06E

:2033D300D000D8F322740D1233A4740A123268E5106005F8E41233CA22C2AF9033F6C082B6

:2033F300C08332C3721B721A5008903405C082C083323008033099FD851581301D04C2AFB3

:2034130080FE222008FDC21712341F22301A0C90342EC082C083C0D00230D722123435026F

:203433003462C2D5EB33EA12345990344 17380 12D3222200EACBFAE8C9F812347620D5017A

:20345300B3221 2347622333333C3540622B2D5A2D58008B3B0D58003B372D5400302348595

:20347300023489C2D5EAB50308C2D5E8B50102D2D522E4F8FA2278FF7AFF22EB33EA12
34E1

:20349300059C0E090349A73800F8027800312 34F61234FFEA5B33401F1234CFEA30E70A33EB
:2034B300487014D0E030D01122D0E030D0031234F6221234F680E115810231FEC3E889F0B1
:2034D300A4FCADF0E960088AF0A420D2122DFDEB600688F0A420D2072D4004FAA804
22D38D
:2034F3000231FEE4C398F8E49AFAC322E4C399F9E49BFBC322123476023467D2D58002C218
:2035130005C3E829F8EA3BFA10D504A2D2C2D250030231FE22E0F87A0022123435023466B6
:20353300D2D58002C2D5C3E899F8EA9BFA10D504A2D2C2D250030231FE221234760234
660E
:2035530012343502346 7EA30E70A1234F6EA30E70302320E2212343502346CE859F8EA5BF6
:20357300FA22E849F8EA4BFA2212347C023466123435023466BE49360061233A4A380F62029
:2035930008FD22123476023462E52AA829B52AF9FA22E0F8A3E0FA22C3A3E033400302355F
:2035B30068023582EB33EA1234599035C17380118013801E1234F61234FF1231AA1234FFC2
:2035D300221231AA221234F61231AA1234F61234FF221234FF1231AA1234F622D007D0069E
:2035F300D005D004D003D002D001D000D0F0D0E0D0D020D509D083D082D0D0C21B32
D08314
:20361300D082D0D0C21322C0A853A8EB9074A1E0F890749DAB8290749EE0F9A3E0B500
045E
:20363300078008 01A8983F582A3E582B5030390748DE0F8AB82E58390749EF0EBA3F0D0A897
:20365300022907 48DAA83A98290749EEAF0A3E9F09074A0EAF0A3E9F0221234760234 60C224
:2036730011 1233A080FE7433C3301B02940EF5F0E581B51500400C747FF42581400525F022
:20369300400122851 58102369C903871746402373 9C082C0831233D89038971235881 2362A
:2036B300BFD083D0821235881233D82290389E12358890755B1235A5907551A982AB831243
:2036D30036E2907551123393 9038A512358822C3E84A7006C0E07C01800E7C00790A1237D5
:2036F30007C0010CE84A70F47930D0E029F0A3DCF9E4F022AF02AE00AB0178077902EF
8BEE
:2037130 0F084AAF0FF18E6D7C48BF084AAF0C4C6C4D7C48BF084AAF0D6A806AA07A
9F022E5
:20373300E8F0A3EAF022B42800500122C0E0C000C002C0E0C082C0839075591235A590751A
:203753005B12373 3D083D082D0E0B4280040061236A42008FDD002D000D0E090755DF0B49F
:20377300500122B42800500122B49601223 01B0B301A0890378EC082C08332C2AF80FEC2A2
:20379300AF301B0B301A089037A2C082C08332C2AF80FE5045533A54454D5020322E34356F
:2037B30061006100420047004700420041727261792049 6E64657820546F6F204269670087
:2037D300537472696E67205472756E6361746564004469 6964657206279205A65726F0085
:2037F3004D617468204F7665 72666C6F77004D617468205 56E6465726 66C6F77004D617436
:2038130068204172677 756D656E7420426164 00496E707574205661 6C756520546F6F2042C2
:2038330069670 0496E 70757420566 16C7565204572726F 200496E70757420566 16C7565 21
:2038530020 4E65676174697665 004973744163 6B62F42353220436F6E666C696374004973 BD

:2038730074616368204572726F7200566563746F722072656C6F636174696F6E20464149B5
:20389300C4544004552524F522000284C696E652000293A200053544152545502F52454A
:0438B3005345540025
:00003301CC

Stepper processor(IC 3) hex file(Step23.hex):

:03000000020033C8
:05000B00C0D0021EB68A
:05001300C0D0021E68D0
:05002300C0D0021FDF48
:05002B00C0D0021C3CE6
:20003300C2AFC2D4C2D37521007522007523000D215D28AAF817581308581 15C0D0C0F0C098
:20005300E0C08753C6FB121C837527405388CF5389F0121F0E78FC121C57121F6E1221AC99
:20007300C20EC20FC208759850D2ACD2AFE587D0E0D000D001D0D030E20530E4028012
206A
:20009300E602800DBF07028008902453 74FA0222E475C7AA75C755438740907058C082C030
:2000B3008378177A00C000C002902351C082C083D003D001D005D004D002D000121C8F905A
:2000D3007024C082C0837819 7A00C000C002C000C002781A7A00C000C002D003D001D002E1
:2000F300D000121CCCE84A7003021CBC78027A00C000C002D003D001D002D000121D00
C0F9
:20011300000C002D003D001D002D000121D3FC000C00278547A3DC000C002D002D000D0
83DA
:20013300D082E8F0A3EAF0907009C082C083783C7A00D083D082E8F002068D907004C082F1
:20015300C08378007A00D083D082E8F0907010E0F8A3E0FAC000C00278807A3EC000C002C1
:20017300D003D001D002D000121D59E84A70030201B49070 10C082C083907010E0F8A3E042
:20019300FAC000C00278807A3EC000C002D003D001D002D000121D8ED083D082E8F0A3
EA91
:2001B300F0907010E0F8A3E0FAC000C00278007A00C000C002D003D001D002D000121D
A4C8
:2001D300E84A70030202099907010C082C083907010E0F8A3E0FAC000C00278807A3EC0006E
:2001F300C002D003D001D002D000121D43D083D082E8F0A3EAF0907010E0F8A3E0FAC
0053
:20021300C00278407A1FC000C002D003D001D002D000121D59E84A7003020272907004C089
:20023300082C08378017A00D083D082E8F0120639 19070 10C082C08378807A3EC000C00290D6

52

:200253007010E0F8A3E0FAC000C002D003D001D002D000121D8ED083D082E8F0A3EAF
090A7

:200273007012C082C083907010E0F8A3E0FAC000C00290700EE0F8A3E0FAC000C002D003C5

:20029300D001D002D000121DAAD083D082E8F0A3EAF090700CC082C08390700CE0F8A
3E00D

:2002B300FAC000C0029070 12E0F8A3E0FAC000C002D003D001D002D000121D43D083D0
8269

:2002D300E8F0A3EAF090700CE0F8A3E0FAC000C00278807A3EC000C002D003D001D00
2D05B

:2002F30000121DFDE84A700302032D90700CC082C08390700CE0F8A3E0FAC000C00278807C

:200313007A3EC000C002D003D001D002D000121D8ED083D082E8F0A3EAF0907010E0F8
A308

:20033300E0FAC000C00278017A00C000C002D003D001D002D000121D59E84A70030205D
28D

:20035300907015E0F87A00C000C00278017A00C000C002D003D001D002D000121DA4E84AE1

:200373007003020389907015C082C083780 17A00D083D082E8F0907015E0F87A00C000C078

:2003930000 2780B7A00C000C002D003D001D002D000121D59E84A70030203BF907015C0823B

:2003B300C083780B7A00D083D082E8F090701EC082C083907010E0F8A3E0FAC000C002786B

:2003D300027A00C000C002D003D001D002D000121E03D083D082E8F0A3EAF090701EE0
F8A3

:2003F300A3E0FAC000C0029070 15E0F87A00C000C002D003D001D002D000121E57E84A7093

:200413000030 2042E90701EC082C083907015E0F87A00D083D082E8F0A3EAF090701CC08230

:20043300C083780 17A00D083D082E8F0A3EAF090701AC082C08378157A00D083D082E8F056

:20045300A3EAF0907012C082C083907010E0F8A3E0FAC000C00290701EE0F8A3E0FAC0
005B

:20047300C00290701CE0F8A3E0FAC000C002D003D001D002D000121D00C000C00278027
AC9

:20049300 00C000C002D003D001D002D000121D00C000C002D003D001D002D000121D8A
D001

:2004B30083D082E8F0A3EAF0907020C082C083907012E0F8A3E0FAC000C00278C87A00
C0F7

:2004D3000 00C002D003D001D002D000121E03D083D082E8F0A3EAF0907022C082C083907
02D

:2004F30012E0F8A3E0FAC000C00278C87A00C000C0029070200E0F8A3E0FAC000C002D0
03FA

:20051300D001D002D000121D00C000C002D003D001D002D000121D8AD083D082E8F0A3
EA9B

```
:20053300F0907022E0FE907020E0FF90701EE0F890701CE0F990701AE0FA74FF88F0C0F03F
:200553009A89F0C0F0D2A0F5F0D5F0FDC2A0F5F0D5F0FDD0F0D5F0EBD0F0D5F0E1F
BEF24BF
:2005730001FFDF0302059475F0C8C0F0D2A08BF0D5F0FDC2A08BF0D5F0FDD0F0D5F0
EBDF71
:20059300E6EE2401FEDE030205ADD2A08BF0D5F0FDC2A08BF0D5F0FDDEF0EB88F0C
0F02A63
:2005B30089F0C0F0D2A0F5F0D5F0FDC2A0F5F0D5F0FDD0F0D5F0EBD0F0D5F0E10206
089067
:2005D3007010E0F8A3E0FAC000C00278017A00C000C002D003D001D002D000121E5DE8
4A37
:2005F3007003020608D2A075F0FFD5F0FDC2A075F0FFD5F0FD907004E0F87A00C000C0
026D
:20061300780I7A00C000C002D003D001D002D000121E5DE84A7003020630120631229O7O37
:200633000EE0F8A3E0FAC000C00278FF7AFFC000C002D003D001D002D000121E5DE84
A70DB
:200653000302067090700EC082C08378017A00D083D082E8F0A3EAF0D2A202068690700E7C
:20067300C082C08378FF7AFFD083D082E8F0A3EAF0C2A222D2A122C2A12278007A0012
1E36
:200693006378007A00121F2E121F3BA8887A00C000C00278F77A00C000C002D003D001D01C
:2006B30002D000121F528888A8887A00C000C00278407A00C000C002D003D001D002D0009C
:2006D300121F5688889019BAA882AA83121F5AD297C2A3D2A2C2A4C2A1D21790705812
20AD
:2006F300B81220FD12213B90700EC082C08378017A00C000C002D002D000D083D082E8F06B
:20071300A3EAF0907016C082C08378007A00D083D082E8F0907008C082C08378007A00D0F0
:2007330083D082E8F0907018C082C08378007A00D083D082E8F0907019C082C08378197A44
:2007530000D083D082E8F078007A00121E6378007A00121F2E121F20C000C00278FA7A0074
:20077300C000C002D003D001D002D000121E57E84A700302078C121AFE120C9E907016E001
:20079300F87A00C000C00278017A00C000C002D003D001D002D000121E5DE84A700302075C
:2007B3006890701 8E0F87A00C000C00278017A00C000C002D003D001D002D000121D59E8A7
:2007D3004A7003020B2590700AC082C08378017A00D083D082E8F0A3EAF0907002C082C097
:2007F30083907018E0F87A00D083D082E8F0A3EAF0907000C082C08378017A00D083D082E2
:2008130 0E8F0A3EAF090700AE0F8A3E0FAC000C002907019E0F87A00C000C002D003D0
01FE
:20083300D002D000122154E84A700302096E907010C082C083907024C082C08390700AE036
:20085300F8A3E0FAC000C002C000C002781A7A00C000C002D003D001D002D000121CCC
E856
```

:200873004A7003021CBC78027A00C000C002D003D001D002D000121D00C000C002D003D
0BE
:2008930001D002D000121D3FC000C002D003D00189828B8312215AC000C002907024C08280
:2008B300C083907019E0F87A00C000C002C000C002781A7A00C000C002D003D001D002D09F
:2008D30000121CCCE84A7003021CBC78027A00C000C002D003D001D002D000121D00C0
00E1
:2008F300C002D003D001D002D000121D3FC000C002D003D00189828B8312215AC000C00221
:20091300D003D001D002D000121D8AD083D082E8F0A3EAF0907010C082C083907010E0
F84E
:20093300A3E0FAC000C00290700EE0F8A3E0FAC000C002D003D001D002D000121DAAD
0834E
:20095300D082E8F0A3EAF090701SC082C083780A7A00D083D082E8F012014E907019C0820E
:20097300C08390700AE0F8A3E0FA7A00D083D082E8F0121A2090700AC082C08390700AE006
:20099300F8A3E0FAC000C002907000E0F8A3E0FAC000C002D003D001D002D000121D43
D0EE
:2009B30083D082E8F0A3EAF090700AE0F8A3E0FAC000C002907002E0F8A3E0FAC000C
00240
:2009D300D003D001D002D0009070001221 60E84A600302081 89070 10C082C083907024C0FB
:2009F30082C08378017A00C000C002C000C002781A7A00C000C002D003D001D002D0001242
:200A13001CCCE84A7003021CBC78027A00C000C002D003D001D002D000121D00C000C0
02EF
:200A3300D003D001D002D000121D3FC000C002D003D00189828B8312215AC000C0029070A1
:200A530024C082C083907019E0F87A00C000C002C000C002781A7A00C000C002D003D00139
:200A7300D002D000121CCCE84A7003021CBC78027A00C000C002D003D001D002D00012
1D5D
:200A930000C000C002D003D001D002D000121D3FC000C002D003D00189828B8312215AC081
:200AB30000C002D003D001D002D000121D8AD083D082E8F0A3EAF0907010C082C08390
70D3
:200AD30010E0F8A3E0FAC000C00290700EE0F8A3E0FAC000C002D003D001D002D0001
21DC2
:200AF300AAD083D082E8F0A3EAF0907015C082C083780A7A00D083D082E8F012014E90
70CB
:200B13019C082C08378017A00D083D082E8F0020C87907019E0F87A00C000C002780 17A3F
:200B330000C000C002D003D001D002D000122154E84A7003020C73907010C082C083907098
:200B530024C082C08378017A00C000C002C000C002781A7A00C000C002D003D001D002D00E
:200B730000121CCCE84A7003021CBC78027A00C000C002D003D001D002D000121D00C0
003E

EP 1 442 794 B1

:200B9300C002D003D001D002D000121D3FC000C002D003D00189828B8312215AC000C0027E
:200BB300907024C082C083907019E0F87A00C000C002C000C002781A7A00C000C002D003A9
:200BD300D001D002D000121CCCE84A7003021C8C78027A00C000C002D003D001D002D0005A
:200BF300121D00C000C002D003D001D002D000121D3FC000C002D003D00189828B8312210B
:200C13005AC000C002D003D001D002D000121D8AD083D082E8F0A3EAF0907010C082C08357
:200C3300907010E0F8A3E0FAC000C00290700EE0F8A3E0FAC000C002D003D001D002D0008F
:200C5300121DAAD083D082E8F0A3EAF0907015C082C083780A7A00D083D082E8F012014E3A
:200C7300907019C082C08378017A00D083D082E8F0121A20907016C082C08378007A00D0AA
:200C930083D082E8F0121AF302075A907017C082C083122172750200D083D082E8F09070DD
:200CB30017E0F87A00C000C00278097A00C000C002D003D001D002D000121E5DE84A700341
:200CD3000218341206879070080C082C08378007A00D083D082E8F090700EE0F8A3E0FAC0F5
:200CF30000C00278FF7AFFC000C002D003D001D002D000121E5DE84A7003020D13120631CA
:200D1300907000CC082C08378007A00D083D082E8F0A3EAF0907015C082C08378057A00D0E2
:200D330083D082E8F0907005C082C08378007A00D083D082E8F0907017C082C083122172B9
:200D53007502000D083D082E8F0907017E0F87A00C000C00278007A00C000C002D003D00189
:200D7300D002D000122154E84A7003020D499070170E0F87A00C000C00278527A00C000C08B
:200D930002D003D001D002D000121E5DE84A7003020DA912063190700170E0F87A00C000C0DC
:200DB3000278507A00C000C002D003D001D002D000121E5DE84A7003020DE5907010C0829C
:200DD300C08378017A00D083D082E8F0A3EAF012014E907017E0F87A00C000C00278447A4E
:200DF30000C000C002D003D001D002D000121E5DE84A7003020E58907010C082C083780A67
:200E13007A00D083D082E8F0A3EAF012014E90700CE0F8A3E0FAC000C002786B7A3EC000AC
:200E3300C002D003D001D002D000121D59E84A7003020E58907005C082C083780170A00D0B5
:200E530083D082E8F0907017E0F87A00C000C00278467A00C000C002D003D001D002D000E7
:200E7300121E5DE84A7003020ECB907010C082C0837814 7A00D083D082E8F0A3EAF01201AA
:200E93004E90700CE0F8A3E0FAC000C002786B7A3EC000C002D003D001D002D000121D5923
:200EB300E84A7003020ECB907005C082C083780170A00D083D082E8F0907017E0F87A00C07C

56

EP 1 442 794 B1

:200ED30000C00278477A00C000C002D003D001D002D000121E5DC000C002907005E0F87
AD6

:200EF30000C000C00278007A00C000C002D003D001D002D000122154C000C002D003D001F6

:200F1300D002D0001221D2E84A7003020D49907005E0F87A00C000C00278007A00C000C0CF

:200F33000 2D003D001D002D000121E5DE84A70030218 1 590700EE0F8A3E0FAC000C0027898

:200F5300017A00C000C002D003D001D002D000121E5DE84A7003020F6F1206319070I7C069

:200F730082C083122 172750200D083D082E8F0907017E0F87A00C000C00278007A00C00063

:200F9300C002D003D001D002D000122154E84A7003020F6F907017C082C083907017E0F8FF

:200FB3007A00C000C00278207A00C000C002D003D001D002D000121D8E7A00D083D082
E884

:200FD300F0907010C082C08378967A00C000C002907017E0F87A00C000C002D003D001D010

:200FF30002D000121D8ED083D082E8F0A3EAF0907015C082C083780A7A00D083D082E8
F042

:201013001201 4E907024C082C08378017A00C000C002C000C002781A7A00C000C002D0035B

:201033 00D001D002D000121CCCE84A7003021CBC78027A00C000C002D003D001D002D0
00F5

:20105300121D00C000C002D003D001D002D000121D3FC000C00278007A00D083D082E8F027

:20107300A3EAF0907007C082C08378017A00D083D082E8F09070I0C082C08378367A05D052

:2010930083D082E8F0A3EAF0907015C082C083780A7A00D083D082E8F012014E90700CC0D3

:2010B30082C08378CA7AFAD083D082E8F0A3EAF012063190701 7C082C083122172750200A7

:2010D300D083D082E8F0907017E0F87A00C000C00278007A00C000C002D003D001D002D
0DB

:2010F300001221 54E84A70030210C6907017E0F87A00C000C002784E7A00C000C002D00359

:20111300D001D002D000121E5DE84A7003021178907007C082C083907007E0F87A00C000E7

:20113300C00278017A00C000C002D003D001D002D000121D437A00D083D082E8F0907010A6

:20115300C082C083789B7A02D083D082E8F0A3EAF0907015C082C083780A7A00D083D08233

:20117300E8F012014E907017E0F87A00C000C00278477A00C000C002D003D001D002D00037

:20119300121E5DE84A70030210C6907017C082C083122172750200D083D082E8F0907017E6

:2011B300E0F87A00C000C00278007A00C000C002D003D001D002D000122154E84A70030260

:2011D300119D907007C082C083907017E0F87A00C000C00278207A00C000C002D003D0019F

:2011F300D002D000121D8E7A00D083D082E8F0907010C082C08378807A3EC000C0029070BF

:20121300CE0F8A3E0FAC000C002D003D001D002D000121D8ED083D082E8F0A3EAF09
070DB

:20123300015C082C083780A7A00D083D082E8F012014E90700EE0F8A3E0FAC000C00278F
FCB

:201253007AFFC000C002D003D001D002D000121E5DE84A700302126E120631D21790235F42

:201273001221C41220FD12213B907017C082C083122172750200D083D082E8F0907017E09B

57

:20129300F87A00C000C00278007A00C000C002D003D001D002D000122154E84A700302124D
:2012B3007C907007E0F87A00C000C00278017A00C000C002D003D001D002D000121E5DE894
:2012D3004A700302130C907018C082C08378017A00D083D082E8F0D21790236 11221C4120A
:2012F30020FD12213B907019C082C08378017A00D083D082E8F0021812907014C082C0837D
:2013130078007A00D083D082E8F0907018C082C08378017A00D083D082E8F0907017C082E5
:20133300C083122172750200D083D082E8F0907017E0F87A00C000C00278007A00C000C061
:2013530002D003D001D002D000122154E84A700302132E907017E0F87A00C000C00278520E
:201373007A00C000C002D003D001D002D000121E5DE84A700302138E1206319 07017E0F80B
:201393007A00C000C00278507A00C000C002D003D001D002D000121E5DE84A70030213CA23
:2013B30090 07010C082C08378017A00D083D082E8F0A3EAF012014E907017E0F87A00C0000E
:2013D300C00278447A00C000C002D003D001D002D000121E5DE84A7003021417907010C00B
:2013F30082C083780A7A00D083D082E8F0A3EAF0907015C082C08378047A00D083D082E802
:20141300F012014E907017E0F87A00C000C00278467A00C000C002D003D001D002D000126B
:201433001E5DE84A70030214E0907010C082C08378147A00D083D082E8F0A3EAF0907015D9
:20145300C082C08378087A00D083D082E8F090700CE0F8A3E0FAC000C00278287A3CC00084
:201473000C002D003D001D002D000121DA4C000C0029 07018E0F87A00C000C002907007E029
:20149300F87A00C000C002D003D001D002D0001221D9C000C002D003D001D002D0001221F8
:2014B300DFE84A70030214C112014E0214E0D21790236 31221C41220FD12213B907014C000
:2014D30082C08378017A00D083D082E8F0907017E0F87A00C000C00278547A00C000C00211
:2014F300D003D001D002D000121E5DE84A700302152D907010C082C083782C7A01D083D046
:2015130082E8F0A3EAF0907015C082C083780A7A00D083D082E8F012014E907017E0F87A04
:2015330000C000C00278477A00C000C002D003D001D002D000121E5DE84A70030216C39078
:20155300 7017C082C083122172750200D083D082E8F0907017E0F87A00C000C00278007AF6
:20157300 00C000C002D003D001D002D000122154E84A7003021552907017C082C08390705F
:2015930017E0F87A00C000C00278207A00C000C002D003D001D002D000121D8E7A00D083E9
:2015B300D082E8F0907018C082C083907018E0F87A00C000C00278017A00C000C002D0031D
:2015D300D001D002D000121D437A00D083D082E8F0907024C082C083907018E0F87A00C049
:2015F30000C002C000C002781A7A00C000C002D003D001D002D000121CCCE84A7003021
C03
:20161300BC78027A00C000C002D003D001D002D000121D00C000C002D003D001D002D0
0048
:20163300121D3FC000C00290700CE0F8A3E0FAC000C00278957A00C000C002D003D001
D047
:2016530002D000121D8AC000C0029 07017E0F87A00C000C002D003D001D002D000121D3
FCB
:20167300D083D082E8F0A3EAF0907018E0F87A00C000C002907007E0F87A00C000C002D0C6
:2016930003D001D002D000122 1E6E84A70030216C3D21790236 51221C41220FD12213B9003

58

EP 1 442 794 B1

```
:2016B3007014C082C08378017A00D083D082E8F09070I4E0F87A00C000C00278017A00C003
:2016D30000C002D003D001D002D000121E5DE84A700302132E907010C082C08378807A3E35
:2016F300C000C00290700CE0F8A3E0FAC000C002D003D001D002D000121D8ED083D082
E8E2
:20171300F0A3EAF09070I0E0F8A3E0FAC000C00278007A00C000C002D003D001D002D0
00A8
:20173300121DA4E84A700302176C9070I0E0F8A3E0FAC000C00278807AAB
:201753003EC000C002D003D001D002D000121D43D083D082E8F0A3EAF09070I0E0F8A3
E099
:20177300FAC000C00278407A1FC000C002D003D001D002D000121D59E84A70030217C412A5
:2017930006319070I0C082C08378807A3EC000C002907010E0F8A3E0FAC000C002D003D0AE
:2017B30001D002D000121D8ED083D082E8F0A3EAF090701 5C082C083780A7A00D083D0
8281
:2017D300E8F012014E90700EE0F8A3E0FAC000C00278FF7AFFC000C002D003D001D002
D020
:2017F30000121E5DE84A7003021801120631907019C082C08378017A00D083D082E8F00230
:2018130018823D2179023671221C41220FD12213B907017C082C08378007A00D083D082E8C8
:20183300F0907017E0F87A00C000C00278087A00C000C002D003D001D002D000121E5DE883
:201853004A700302186A907017C082C08378007A00D083D082E8F0907017E0F87A00C00000
:20187300C002780A7A00C000C002D003D001D002D000121E5DE84A70030218A0907017C00C
:2018930082C08378007A00D083D082E8F0907017E0F87A00C000C002780C7A00C000C00296
:2018B300D003D001D002D000121E5DE84A70030218D6907017C082C08378007A00D083D
0FC
:2018D30082E8F0907017E0F87A00C000C002780D7A00C000C002D003D001D002D000121EB9
:2018F3005DE84A700302197290701 7C082C0831221 72750200D083D082E8F0907017E0F822
:20191300 7A00C000C00278007A00C000C002D003D001D002D000122154E84A700302218FBBD
:201933009070I7E0F87A00C000C00278217A00C000C002D003D001D002D000121E5DE84A0F
:20195300700302195E12068702196112068A907017C082C08378007A00D083D082E8F09030
:201973007017E0F87A00C000C002780E7A00C000C002D003D001D002D000121E5DE84A7002
:201993000030219A8907017C082C08378007A00D083D082E8F0907017C082C08378007A00D5
:2019B300D083D082E8F022907016E0F87A00C000C00278007A00C000C002D003D001D002A1
:2019D300D000121E5DE84A7003021A1D907018E0F87A00C000C00278007A00C000C002D089
:2019F30003D001D002D000121D59E84A7003021A1AD2A490701 6C082C08378017A00D083A4
:201A1300D082E8F0021A1D121AF30221EC78007A00121F2E121F20C000C002907009E0F81D
:201A33007A00C000C002D003D001D002D000122217E84A7003021A27D2A378007A00121F86
:201A53002E907006C082C083A8A07A00C000C00278207A00C000C002D003D001D002D00
09C
```

EP 1 442 794 B1

```
:201A7300121F52D083D082E8F0907006E0F87A00C000C00278007A00C000C002D003D00161
:201A9300D002D000121E5DE84A7003021A54C2A3C2A4907006C082C083A8A07A00C000
C057
:201AB3000278207A00C000C002D003D001D002D000121F52D083D082E8F0907006E0F87
ADF
:201AD30000C000C00278007A00C000C002D003D001D002D000122154E84A7003021AA522A8
:201AF300C29775F005D5F0FDD29722907010C082C083907024C082C08378197A00C000C0FA
:201B130002C000C002781A7A00C000C002D003D001D002D000121CCCE84A7003021CBC
7869
:201B3300027A00C000C002D003D001D002D000121D00C000C002D003D001D002D000121
D28
:201B53003FC000C002D003D00189828B83122I5AC000C002907024C082C083907019E0F84B
:201B73007A00C000C002C000C002781A7A00C000C002D003D001D002D000121CCCE84A
7064
:201B930003021CBC78027A00C000C002D003D001D002D000121D00C000C002D003D001
D074
:201BB30002D000121D3FC000C002D003D00189828B83122I5AC000C002D003D001D002D
03E
:201BD30000121D8AD083D082E8F0A3EAF0907010C082C083907010F0F8A3E0FAC000C0
02C3
:201BF30090700EE0F8A3E0FAC000C002D003D001D002D000121DAAD083D082E8F0A3E
AF0D4
:201C1300907015C082C083780A7A00D083D082E8F012014E907019C082C08378197A00D0C4
:201C330083D082E8F0220222ID8587E020E5FAD0D0D0E0D0E0D2D574AA75F055900033
C094
:201C530082C0833253883F53892F438920438780888D758B00D28E2275C7AA75C755D2BF80
:201C730022121C6B75C7AA75C755438704D21D2275C7AA75C75553387FBC21D2289828B8
3DA
:201C9300E493C082C08388828A83F06017DC09E4F01581158102ICC4A3A882AA83D083D
0D3
:201CB30082A380DC15811581229023697446D222E490237D74500222E4121CE8021CD9B2A9
:201CD300D5A2D58008B3B0D58003B372D54003021CF7021CFBC2D5EAB50308C2D5E8B
50181
:201CF30002D2D522E4F8FA2278FF7AFF22C3E889F0A4FCADF0E960088AF0A420D2122
DFDFE
:201D1300EB600688F0A420D2072D4004FAA80422D3021D2790239D74140222E490238E7463
```

60

:201D33000A0222E49023BA74280222E4D2D58002C2D5C3E829F8EA3BFA10D504A2D2C
2D2D1
:201D53005003021D2722121D5F021CD8C2D5EB33EA121D83901D68738012D3222200EA
CBF7
:201D7300FAE8C9F8121CE820D501B322121CE822333333C3540622D2D58002C2D5C3E89
9B8
:201D9300F8EA9BFA10D504A2D2C2D25003021D2722121D5F021CD9EB33EA121D83C0
E0909E
:201DB3001DB673800F80278003121DEB121DF4EA5B33401F121D00EA30E70A33487014
D0F4
:201DD300E030D01122D0E030D003121DEB22121DEB80E11581021D27E4C398F8E49AF
AC325
:201DF30022E4C399F9E49BFBC322121D5F021CDDC3E94B7005D3021D2F227F007E007C
0065
:201E13007D0075F010121E48EE33FEEF33FFC3EF9B40117005C3EE99400AC3EE99FEEF
9B8C
:201E3300FFD38001C3121E50D5F0DA8C008D028F038E01C322C3E833F8EA33FA22EC33
FC0F
:201E5300ED33FD22121CE8021CD8121D5F021CD488298A2A22301911C301705D21AD0D
022BE
:201E73002008FDC082C083907071C2D5D21BC21AC0D0800ED0D032C082C08390706FD2
D549
:201E9300C0D0C0E0C0F0C000C001C002C003C004C005C006C007D215E0F8A3E0F58385
00F4
:201EB30082E473301052C0E085278CE52804B4C808E40529B52902052AF528301138201 34D
:201ED3003520120EE52BB52A07E52CB5290280025024201 71F20081CE5C620E217D0E0D2BD
:201EF30013C212C211C082C083901F04C082C08332021E8ED212D0E0D0D032E4F529F52AF1
:201F1300F528F52CF52BC211C212C213228528E075F005A485E00085F00222750105750326
:201F330000121E03850028225389F0D21043A882D28C2290706FE8F0A3EAF043A88222E8B6
:201F530059F822E849F822907071E8F0A3EAF0D219D2BCC2BAD2B943A884229070DE74
7116
:201F7300F0A37400F09070DC7471F0A37400F0751000751101C208C21722C2ACC082C083DB
:201F9300F89070DCE0F9A3E0F5828983E8F0A3E582B45003907100A882E5829070DDF088AB
:201FB300828983A3E582B45003907100A8829070DEA3E0B50008D2AC0000C2AC80F420089E
:201FD30004D208D299D083D082D2AC22209846C299300E030220B5C0E0C082C083C000
C04A

```
:201FF30001C002C0039070DDE0FA9070DEE0F9A3E0FBB50205C2080220A785038285018
3FA
:20201300E0F599A3E582B45003907100E5829070DFF00220A7C0E0E599C298201S0302205C
:20203300B3B40306200F03022113B41304D20E806FB41109300E69D299C20E8063C082C086
:2020530083C000C001C002C003FA907085E0F8907083AB82907086E0F9A3E0F5828983A3D5
:20207300E582B50303907073EAF0E583A982907086F0E9A3F0B5001C907084E0F5838882A2
:20209300A3E582B50303907073E583A982907084F0E9A3F0D003D002D001D000D083D08287
:2020B300D0E0D0D032E060061220C9A380F72008FD222008FD221220D0121F8D227911B482
:2020D3002000040020722B40D03770122B408021722B40A07D0E0D0E0022210222C000C0E041
:2020F300121F8DD0E0D000D8F322740D1220C9740A121F8DE5106005F8E41220EF22C2A
F00
:20211300090211BC082C08332C3721B721A500890212AC082C083323008033099FD85158147
:20213300301D04C2AF80FE222008FDC217122144223010A0C902153C082C083C0D0021E689C
:20215300022121CE8021CD2E0F8A3E0FA22C3A3E0334003022216C021DFD121D5F021CDE
C01C
:20217300A853A8EB907087E0F8907083AB82907084E0F9A3E0B500047800801A8983F58281
:20219300A3E582B50303907073E0F8AB82E583907084F0EBA3F0D0A822907073AA83A98230
:2021B300907084EAF0A3E9F0907086EAF0A3E9F022E49360061220C9A380F62008FD22E814
:2021D30049F8EA4BFA22121CEE021CD9E859F8EA5BFA22121CEE021CD4D007D006D0
05D04D
:2021F30004D003D002D001D000D0F0D0E0D0D020D509D083D082D0D0C21B32D083D08
2D0AB
:20221300D0C21322121CE8021CDDC2111220C580FE7433C3301B02940EF5F0E581B515001D
:20223300400C747FF42581400525F0400122851581022247902418764640222E4C082C0833B
:202253001220FD90244112211C412226AD083D0821221C41220FD229024481221C490715B76
:2022730012215A907151A982AB8312228D90715111220B890244F1221C422C3E84A7006C0CF
:20229300E07C01800E7C00790A1222B2C0010CE84A70F47930D0E029F0A3DCF9E4F022
AF69
:2022B30002AE00AB0178077902EF8BF084AAF0FF18E6D7C48BF084AAF0C4C6C4D7C48
BF098
:2022D30084AAF0D6A806AA07A9F022E8F0A3EAF022B428005001Z2C0E0C000C002C0E
0C095
:2022F30082C0839071591221SA90715B1222DED083D082D0E0B42800400612224F2008FD92
:20231300D002D000D0E090715DF0B4500122B428005001Z2B49601ZZ301B0B301A089023CC
:20233300399C082C08332C2AF80FEC2AF301B0B301A0890234DC082C08332C2AF80FE504557
:20235300533A53544550203Z2E3330004F004500450045004500417272617920496E6465BC
:2023730078Z0546F6F20426967700537472696E67Z05472756E636174656400446976696941D
```

EP 1 442 794 B1

:2023930065206279205A65726F004D617468204F766572666C6F77004D61746820556E643B
:2023B3006572666C6F77004D61746820417267756D656E7420426164004496E705742056E1
:2023D300616C756520546F6F204269670049676E7075742056616C7565204572726F7200491F
:2023F3006E707574205661756C6C7565204E65676174697665500497374616636B2F4235322043F9
:202413006F6E666C6963374004973746163636B204572726F72005665667374F7220565C6FEC
:202433006361746696F6E204641494C454400455244F522000284C696E6652000293A200049
:0E2453005354415254555502F52455345540096
:0000330ICC

Serial snoop processor(IC 16) hex file(Serial5.hex):

:100000006800060061E0A1209080C2A0003042803C9
:1000100006006E8051009EA02060A08022900000A95
:100020000540C130A6C0C2B00EB02140A0008010C90
:1000300060000C0500060C2500000A120C8901C0
:1000400043066504130C890143066505140C8901F8
:1000500043060504150C890143060505160C8901A4
:1000600043062504170C890143062505180C890150
:1000700043064504190C890143064505200C2B0055
:06008000EB02400A000A39
:0203FE00170ADC
:00000001FF

## Claims

1. An apparatus for performing PCR and monitoring the reaction in real time during temperature cycling comprising:

   a plurality of capillary tubes each for holding a PCR sample, each capillary tube comprising an optically clear material, each capillary tube holding less than 1 milliliter of a sample;
   a rotatable carousel to sequentially move the PCR samples one by one to a monitoring location during amplification;
   thermal cycling means comprising:

   a forced air heater for simultaneously heating all the PCR samples;
   means for simultaneously cooling all the PCR samples; and

   control means for repeatedly operating the thermal cycling means to subject the PCR samples to thermal cycling;
   means for optically exciting one of the samples to cause that sample to fluoresce; and
   means for detecting the fluorescence of the excited sample when the excited sample is in the monitoring location.

2. An apparatus for performing PCR and monitoring the reaction in real time during temperature cycling as defined in claim 1 further comprising:

   means for determining at least one reaction parameter in accordance with the detected fluorescence; and
   means for adjusting the control means in accordance with the reaction parameter.

3. An apparatus for performing PCR and monitoring the reaction in real time during temperature cycling as defined in claim 2 in which the control means adjusts the operation of the means for heating and the means for cooling to alter the times the means for heating and the means for cooling operate in accordance with the reaction parameter.

4. An apparatus for performing PCR and monitoring the reaction in real time during temperature cycling as defined in claim 2 in which the control means adjusts the operation of the means for heating and the means for cooling to alter the rate at which the biological samples are heated and cooled in accordance with the reaction parameter.

5. An apparatus for performing PCR and monitoring the reaction in real time during temperature cycling as defined in claim 1 wherein each capillary tube is fabricated at least partially from glass.

6. An apparatus for performing PCR and monitoring the reaction in real time during temperature cycling as defined in claim 1 further comprising means for positioning the means for optically exciting the sample and the means for detecting the fluorescence of the excited sample to optimize the fluorescence which is detected.

7. An apparatus for performing PCR and monitoring the reaction in real time during temperature cycling as defined in claim 1 wherein the means for cooling comprises an air movement mechanism which transports ambient air to the capillary tubes.

8. An apparatus for performing PCR and monitoring the reaction in real time during temperature cycling as defined in claim 1 wherein the control means comprises a microprocessor.

9. An apparatus for performing PCR and monitoring the reaction in real time during temperature cycling as defined in claim 1 wherein the means for optically exciting the sample comprises a photo emitter structure positioned so that the fluorescence emitted therfrom impinges a side of the capillary tube that is holding the excited sample.

10. An apparatus for performing PCR and monitoring the reaction in real time during temperature cycling as defined in claim 9 wherein means for detecting the fluorescence of the excited sample comprises a photo detector structure positioned so that the fluorescence emitted from the side of the excited sample's capillary tube is detected.

11. An apparatus for performing PCR and monitoring the reaction in real time during temperature cycling as defined in claim 1 wherein the means for optically exciting the sample comprises a photo emitter structure positioned so that the fluorescence emitted therfrom impinges an end of the excited sample's capillary tube.

12. An apparatus for performing PCR and monitoring the reaction in real time during temperature cycling as defined in claim 11 wherein the means for detecting the fluorescence of the excited sample comprises a photo detector structure positioned so that the fluorescence emitted from the end of the excited sample's capillary tube is detected.

13. An apparatus for performing PCR and monitoring the reaction in real time during temperature cycling as defined in claim 1 wherein the means for determining at least one reaction parameter in accordance with the detected fluorescence comprises computing means for determining at least one reaction parameter selected from the group consisting of: product melting temperature, product melting time, product reannealing temperature, product reannealing time, probe melting temperature, probe melting time, primer annealing/extension temperature, and primer annealing/extension time.

14. An apparatus for performing PCR and monitoring the reaction in real time during temperature cycling as defined in claim 1 wherein the control means comprises means cooling the samples when the means for detecting the fluorescence of the excited sample detects that the product is completely melted.

15. An apparatus for performing PCR and monitoring reaction in real time during temperature cycling as defined in claim 1 wherein the control means comprises means for heating the samples when the means for detecting the fluorescence of the excited sample detects no more product generation.

16. An apparatus for performing PCR and monitoring the reaction in real time during temperature cycling as defined in claim 1, which comprises monitoring means for ascertaining the biological reaction; and controlling means for controlling the operation of the carousel, thermal cycling means and the monitoring means such that the progress of the biological reaction is detected as thermal cycling occurs, wherein

the means for optically exciting one of the samples comprises

an excitation source emitting excitation fluorescence; and
means for directing the excitation fluorescence to the monitoring position such that the sample located at the monitoring position emits fluorescence; and

the means for detecting the fluorescence of the excited sample comprises

means for converting the emitted fluorescence to an electrical signal;
means for processing the electrical signal to arrive at a reaction parameter;
means for displaying the reaction parameter; and
means for recording the reaction parameter.

17. An apparatus for performing PCR and monitoring the reaction in real time during temperature cycling as defined in claim 16 wherein the reaction parameter is selected from the group consisting of denaturation temperature and time, primer annealing temperature and time, probe annealing temperature and time, enzyme extension temperature and time, and number of cycles.

18. An apparatus for performing PCR and monitoring the reaction in real time during temperature cycling as defined in claim 16 wherein:

the excitation source comprises a photo-emitting source, the photo-emitting source selected from the group consisting of a xenon lamp and a light emitting diode;
the means for converting the emitted fluorescence to an electrical signal comprises a photo-detection device, the photo-detection device selected from the group consisting of a photo-multiplier tube and a photo-diode; and
the means for processing the electrical signal to arrive at a reaction parameter comprises a microprocessor.

19. An apparatus for performing PCR and monitoring the reaction in real time during temperature cycling as defined in claim 18 wherein the means for converting the emitted fluorescence to an electrical signal comprises a first photo-detection device, the first photo-detection device is selected from the group consisting of a photo-multiplier tube and a photo-diode and a second photo-detection device selected from the group consisting of a photo-multiplier tube and a photo-diode.

20. A method of performing nucleic acid amplification while monitoring the progress of the amplification, the method comprising the steps of:

(a) providing a rotating carousel containing a plurality of biological samples each in a capillary tube to undergo nucleic acid amplification;
(b) simultaneously heating all of the capillary tubes with hot air to induce a first temperature transition in the biological samples from a first temperature to a second temperature;
(c) simultaneously cooling all the capillary tubes to induce a second temperature transition in the biological samples from the second temperature to the first temperature;
(d) exciting the biological samples during the temperature transition such that fluorescence is emitted from the sample;
(e) rotating the carousel to sequentially move the samples one by one to a monitoring location during amplification; and
(f) detecting the fluorescence which is emitted from the samples at least once when each capillary tube is in the monitoring location to determine at least one reaction parameter.

21. A method of performing nucleic acid amplification as defined in claim 20 wherein step (f) takes place during the first temperature transition and comprises the step of determining the melting temperature of a product.

22. A method of performing nucleic acid amplification as defined in claim 20 wherein step (f) takes place during the first temperature transition and comprises the step of determining the melting temperature of a probe.

23. A method of performing nucleic acid amplification as defined in claim 20 wherein step (b) further comprises the step of increasing the temperature of the biological samples at a rate of at least as great as 2.5°C per second.

**24.** A method of performing nucleic acid amplification as defined in claim 20 wherein step (b) further comprises the step of increasing the temperature of the biological samples at least 40°C during a period of not more than ten seconds.

**25.** A method of performing nucleic acid amplification as defined in claim 20 wherein step (f) takes place during the first temperature transition and further comprises the step of detecting the fluorescence emitted from the excited sample at least twice during the temperature transition.

**26.** A method of performing nucleic acid amplification as defined in claim 20 wherein step (b) further comprises the step of increasing the temperature of the biological samples until the product in the excited sample is completely melted.

**27.** A method of performing nucleic acid amplification as defined in claim 20 wherein

the excited biological sample includes a substance which emits fluorescence at a first wavelength which is related to the progress of the nucleic acid amplification;
the temperature transition from the first temperature to the second temperature defines a first range;
step (f) further comprises detecting the fluorescence emitted by the excited biological sample a plurality of times when the temperature of the sample is within the first range;

and further comprising the steps of:

(g) adjusting the temperature of all of the samples over a second range including the second temperature and a third temperature, the third temperature being different than the second temperature; and
(h) detecting the fluorescence emitted by the excited biological sample of plurality of times when the temperature of the sample is within the second range.

**28.** A method of performing nucleic acid amplification as set forth in claim 27 further comprising the step of:

(i) adjusting at least one parameter of the amplification reaction in accordance with the fluorescence detected in step (d) or (h).

**29.** A method of performing nucleic acid amplification as set forth in claim 27 wherein step (b) further comprises the step of ceasing to adjust the temperature of the samples over a first range when product melting is substantially complete.

**30.** A method of performing nucleic acid amplification as set forth in claim 27 wherein step (g) further comprises the step of ceasing to adjust the temperature of the samples over a second range when primer annealing substantially ceases.

**31.** A method of performing nucleic acid amplification as set forth in claim 27 further comprising the step of:

(j) adjusting the temperature of all of the samples over a third range and ceasing to adjust the temperature of the samples over the third range when product accumulation reaches an effective level.

**32.** A method of performing nucleic acid amplification as set forth in claim 27 wherein steps (f) and (h) further comprise the step of detecting the fluorescence emitted from the excited biological sample in the range from about 400 nm to about 800 nm.

**Patentansprüche**

**1.** Eine Vorrichtung zur Durchführung einer PCR und Überwachung der Reaktion in Echtzeit während zyklischer Temperaturwechsel, umfassend:

eine Vielzahl an Kapillarröhrchen, jedes zum Aufnehmen einer PCR-Probe, wobei jedes Kapillarröhrchen ein optisch klares Material umfasst, wobei jedes Kapillarröhrchen weniger als 1 Milliliter einer Probe aufnimmt;
ein drehbares Karussell zur sequenziellen Bewegung der PCR-Proben während der Amplifikation eine nach der anderen zu einem Überwachungsposition;
Thermocyclingeinrichtung, umfassend:

eine Gebläseheizung zur gleichzeitigen Erwärmung aller PCR-Proben;
Einrichtung zur gleichzeitigen Kühlung aller PCR-Proben; und

Steuereinrichtung zum wiederholten Betreiben der Thermocyclingeinrichtung, um die PCR-Proben einem Thermocycling zu unterziehen;
Einrichtung zur optischen Anregung einer der Proben, um ein Fluoreszieren dieser Probe zu bewirken; und
Einrichtung zur Erfassung der Fluoreszenz der angeregten Probe, wenn die angeregte Probe sich an der Überwachungsposition befindet.

2. Eine Vorrichtung zur Durchführung einer PCR und Überwachung der Reaktion in Echtzeit während zyklischer Temperaturwechsel nach Anspruch 1, welche ferner umfasst:

Einrichtung zur Bestimmung von zumindest einem Reaktionsparameter gemäß der erfassten Fluoreszenz; und
Einrichtung zur Einstellung der Steuereinrichtung gemäß dem Reaktionsparameter.

3. Eine Vorrichtung zur Durchführung einer PCR und Überwachung der Reaktion in Echtzeit während zyklischer Temperaturwechsel nach Anspruch 2, in welcher die Steuereinrichtung den Betrieb der Heizeinrichtung und der Kühleinrichtung einstellt, um die Zeiten zu ändern, in denen die Heizeinrichtung und die Kühleinrichtung betrieben werden, gemäß dem Reaktionsparameter.

4. Eine Vorrichtung zur Durchführung einer PCR und Überwachung der Reaktion in Echtzeit während zyklischer Temperaturwechsel nach Anspruch 2, in welcher die Steuereinrichtung den Betrieb der Heizeinrichtung und der Kühleinrichtung einstellt, um die Rate zu ändern, mit der die biologischen Proben erwärmt und gekühlt werden, gemäß dem Reaktionsparameter.

5. Eine Vorrichtung zur Durchführung einer PCR und Überwachung der Reaktion in Echtzeit während zyklischer Temperaturwechsel nach Anspruch 1, wobei jedes Kapillarröhrchen zumindest teilweise aus Glas hergestellt ist.

6. Eine Vorrichtung zur Durchführung einer PCR und Überwachung der Reaktion in Echtzeit während zyklischer Temperaturwechsel nach Anspruch 1, welche ferner eine Einrichtung zur Positionierung der Einrichtung zur optischen Anregung der Probe und der Einrichtung zur Erfassung der Fluoreszenz der angeregten Probe umfasst, um die erfasste Fluoreszenz zu optimieren.

7. Eine Vorrichtung zur Durchführung einer PCR und Überwachung der Reaktion in Echtzeit während zyklischer Temperaturwechsel nach Anspruch 1, wobei die Kühleinrichtung einen Luftbewegungsmechanismus umfasst, der Umgebungsluft zu den Kapillarröhrchen transportiert.

8. Eine Vorrichtung zur Durchführung einer PCR und Überwachung der Reaktion in Echtzeit während zyklischer Temperaturwechsel nach Anspruch 1, wobei die Steuereinrichtung einen Mikroprozessor umfasst.

9. Eine Vorrichtung zur Durchführung einer PCR und Überwachung der Reaktion in Echtzeit während zyklischer Temperaturwechsel nach Anspruch 1, wobei die Einrichtung zur optischen Anregung der Probe eine Photoemitteranordnung umfasst, die so angeordnet ist, dass die davon emittierte Fluoreszenz auf eine Seite des Kapillarröhrchens trifft, das die angeregten Probe aufbewahrt.

10. Eine Vorrichtung zur Durchführung einer PCR und Überwachung der Reaktion in Echtzeit während zyklischer Temperaturwechsel nach Anspruch 9, wobei die Einrichtung zur Erfassung der Fluoreszenz der angeregten Probe einen Photodetektoranordnung umfasst, die so angeordnet ist, dass die von der Seite des Kapillarröhrchens der angeregten Probe emittierte Fluoreszenz erfasst wird.

11. Eine Vorrichtung zur Durchführung einer PCR und Überwachung der Reaktion in Echtzeit während zyklischer Temperaturwechsel nach Anspruch 1, wobei die Einrichtung zur optischen Anregung der Probe eine Photoemitteranordnung umfasst, die so angeordnet ist, dass die davon emittierte Fluoreszenz auf ein Ende des Kapillarröhrchens der angeregten Probe trifft.

12. Eine Vorrichtung zur Durchführung einer PCR und Überwachung der Reaktion in Echtzeit während zyklischer Temperaturwechsel nach Anspruch 11, wobei die Einrichtung zur Erfassung der Fluoreszenz der angeregten Probe eine Photodetektoranordnung umfasst, die so positioniert ist, dass die von dem Ende des Kapillarröhrchens der angeregten Probe emittierte Fluoreszenz erfasst wird.

13. Eine Vorrichtung zur Durchführung einer PCR und Überwachung der Reaktion in Echtzeit während zyklischer Temperaturwechsel nach Anspruch 1, wobei die Einrichtung zur Bestimmung von zumindest einem Reaktionsparameter gemäß der erfassten Fluoreszenz eine Berechnungseinrichtung zur Bestimmung von zumindest einem Reaktionsparameter umfasst, der aus der Gruppe ausgewählt ist, bestehend aus: Schmelztemperatur des Produkts, Schmelzzeit des Produkts, Reannealingtemperatur des Produkts, Reannealingzeit des Produkts, Schmelztemperatur der Sonde, Schmelzzeit der Sonde, Annealing/Extensions-Temperatur des Primers und Annealing/Extensions-Zeit des Primers.

14. Eine Vorrichtung zur Durchführung einer PCR und Überwachung der Reaktion in Echtzeit während zyklischer Temperaturwechsel nach Anspruch 1, wobei die Steuereinrichtung eine Einrichtung umfasst, welche die Proben kühlt, wenn die Einrichtung zur Erfassung der Fluoreszenz der angeregten Probe erfasst, dass das Produkt vollständig geschmolzen ist.

15. Eine Vorrichtung zur Durchführung einer PCR und Überwachung der Reaktion in Echtzeit während zyklischer Temperaturwechsel nach Anspruch 1, wobei die Steuereinrichtung eine Einrichtung zur Erwärmung der Probe umfasst, wenn die Einrichtung zur Erfassung der Fluoreszenz der angeregten Probe keine weitere Produkterzeugung erfasst.

16. Eine Vorrichtung zur Durchführung einer PCR und Überwachung der Reaktion in Echtzeit während zyklischer Temperaturwechsel nach Anspruch 1, welche eine Überwachungseinrichtung umfasst zur Feststellung der biologischen Reaktion und eine Steuereinrichtung umfasst zur Steuerung des Betriebs des Karussells, der Thermocyclingeinrichtung und der Überwachungseinrichtung, derart, dass der Fortschritt der biologischen Reaktion während des Ablaufs des Thermocyclings erfasst wird, wobei

das Mittel zur optischen Anregung einer der Proben

eine Anregungsquelle, welche eine Anregungsfluoreszenz emittiert; und
ein Mittel zur Leitung der Anregungsfluoreszenz zu der Beobachtungsposition umfasst, derart, dass die Probe, die sich an der Beobachtungsposition befindet, eine Fluoreszenz emittiert; und

das Mittel zur Erfassung der Fluoreszenz der angeregten Probe

ein Mittel zur Umwandlung der emittierten Fluoreszenz in ein elektrisches Signal;
ein Mittel zur Verarbeitung des elektrischen Signals, um zu einem Reaktionsparameter zu kommen;
ein Mittel zur Anzeige des Reaktionsparameters; und
ein Mittel zur Aufzeichnung des Reaktionsparameters umfasst.

17. Eine Vorrichtung zur Durchführung einer PCR und Überwachung der Reaktion in Echtzeit während zyklischer Temperaturwechsel nach Anspruch 16, wobei der Reaktionsparameter aus der Gruppe ausgewählt ist, bestehend aus Denaturierungstemperatur und -zeit, Annealingtemperatur und -zeit des Primers, Annealingtemperatur und -zeit der Sonde, Extensionstemperatur und - zeit des Enzyms und Anzahl der Zyklen.

18. Eine vorrichtung zur Durchführung einer PCR und Überwachung der Reaktion in Echtzeit während zyklischer Temperaturwechsel nach Anspruch 16, wobei:

die Anregungsquelle eine Photoemissionsquelle umfasst, wobei die Photoemissionsquelle aus der Gruppe ausgewählt ist, bestehend aus einer Xenonlampe und einer lichtemittierenden Diode;
das Mittel zur Umwandlung der emittierten Fluoreszenz in ein elektrisches Signal eine Photodetektorvorrichtung umfasst, wobei die Photodetektorvorrichtung aus der Gruppe ausgewählt ist, bestehend aus einer Photomultiplierröhre und einer Photodiode; und
das Mittel zur Verarbeitung des elektrischen Signals, um zu einem Reaktionsparameter zu kommen, einen Mikroprozessor umfasst.

19. Eine Vorrichtung zur Durchführung einer PCR und Überwachung der Reaktion in Echtzeit während zyklischer Temperaturwechsel nach Anspruch 18, wobei das Mittel zur Umwandlung der emittierten Fluoreszenz in ein elektrisches Signal eine erste Photodetektorvorrichtung umfasst, wobei die erste Photodetektorvorrichtung aus der Gruppe ausgewählt ist, bestehend aus einer Photomultiplierröhre und einer Photodiode, und eine zweite Photodetektorvorrichtung umfasst, die aus der Gruppe ausgewählt ist, besteht aus einer Photomultiplierröhre und einer Photodiode.

**20.** Ein Verfahren zum Durchführen einer Nucleinsäureamplifikation unter Überwachen des Fortschritts der Amplifikation, wobei das Verfahren die folgenden Schritte umfasst:

(a) Bereitstellen eines sich drehenden Karussells, das eine Vielzahl an biologischen Proben jeweils in einem Kapillarröhrchen enthält, um diese einer Nucleinsäureamplifikation zu unterziehen,
(b) gleichzeitiges Erwärmen aller Kapillarröhrchen mit heißer Luft, um einen ersten Temperaturübergang in den biologischen Proben von einer ersten Temperatur zu einer zweiten Temperatur zu induzieren;
(c) gleichzeitiges Kühlen aller Kapillarröhrchen, um einen zweiten Temperaturübergang in den biologischen Proben von der zweiten Temperatur zu der ersten Temperatur zu induzieren;
(d) Anregen der biologischen Proben während des Temperaturübergangs derart, dass von der Probe eine Fluoreszenz emittiert wird;
(e) Drehen des Karussells, um die Proben während der Amplifikation sequenziell eine nach der anderen zu einer Überwachungsposition zu bewegen; und
(f) Erfassen der Fluoreszenz, die von den Proben emittiert wird, zumindest einmal, wenn jedes Kapillarröhrchen sich an der Überwachungspösition befindet, um zumindest einen Reaktionsparameter zu bestimmen.

**21.** Ein Verfahren zum Durchführen einer Nucleinsäureamplifikation nach Anspruch 20, wobei Schritt (f) während des ersten Temperaturübergangs stattfindet und den Schritt des Bestimmens der Schmelztemperatur eines Produkts umfasst.

**22.** Ein Verfahren zum Durchführen einer Nucleinsäureamplifikation nach Anspruch 20, wobei Schritt (f) während des ersten Temperaturübergangs stattfindet und den Schritt des Bestimmens der Schmelztemperatur einer Sonde umfasst.

**23.** Ein Verfahren zum Durchführen einer Nucleinsäureamplifikation nach Anspruch 20, wobei Schritt (b) ferner den Schritt der Erhöhung der Temperatur der biologischen Proben mit einer Rate von mindestens soviel wie 2,5 °C pro Sekunde umfasst.

**24.** Ein Verfahren zum Durchführen einer Nucleinsäureamplifikation nach Anspruch 20, wobei Schritt (b) ferner den Schritt der Erhöhung der Temperatur der biologischen Proben von mindestens 40 °C während eines Zeitraums von nicht mehr als zehn Sekunden umfasst.

**25.** Ein Verfahren zum Durchführen einer Nucleinsäureamplifikation nach Anspruch 20, wobei Schritt (f) während des ersten Temperaturübergangs stattfindet und ferner den Schritt des Erfassens der von der angeregten Probe emittierten Fluoreszenz zumindest zweimal während des Temperaturübergangs umfasst.

**26.** Ein Verfahren zum Durchführen einer Nucleinsäureamplifikation nach Anspruch 20, wobei Schritt (b) ferner den Schritt des Erhöhens der Temperatur der biologischen Proben umfasst, bis das Produkt in der angeregten Probe vollständig geschmolzen ist.

**27.** Ein Verfahren zum Durchführen einer Nucleinsäureamplifikation nach Anspruch 20, wobei

die angeregte biologische Probe eine Substanz einschließt, die bei einer ersten Wellenlänge eine Fluoreszenz emittiert, welche in Beziehung steht mit dem Fortschreiten der Nucleinsäureamplifikation;
der Temperaturübergang von der ersten Temperatur zu der zweiten Temperatur einen ersten Bereich definiert;
Schritt (f) ferner ein mehrmaliges Erfassen der von der angeregten biologischen Probe emittierten Fluoreszenz umfasst, wenn die Temperatur der Probe innerhalb des ersten Bereichs liegt;

und welches ferner die folgenden Schritte umfasst:

(g) Einstellen der Temperatur aller Proben über einen zweiten Bereich, der die zweite Temperatur und eine dritte Temperatur einschließt, wobei die dritte Temperatur von der zweiten Temperatur verschieden ist; und
(h) mehrmaliges Erfassen der von der angeregten biologischen Probe erfassten Fluoreszenz, wenn die Temperatur der Probe innerhalb des zweiten Bereichs liegt.

**28.** Ein Verfahren zum Durchführen einer Nucleinsäureamplifikation nach Anspruch 27, welches ferner den folgenden Schritt umfasst:

(i) Einstellen von zumindest einem Parameter der Amplifikationsreaktion gemäß der in Schritt (d) oder (h) erfassten Fluoreszenz.

29. Ein Verfahren zum Durchführen einer Nucleinsäureamplifikation nach Anspruch 27, wobei Schritt (b) ferner den Schritt des Beendens des Einstellens der Temperatur der Proben über einen ersten Bereich.umfasst, wenn das Schmelzen des Produkts im Wesentlichen vollendet ist.

30. Ein Verfahren zum Durchführen einer Nucleinsäureamplifikation nach Anspruch 27, wobei Schritt (g) ferner den Schritt des Beendens des Einstellens der Temperatur der Proben über einen zweiten Bereich umfasst, wenn das Annealing des Primers im Wesentlichen beendet ist.

31. Ein Verfahren zum Durchführen einer Nucleinsäureamplifikation nach Anspruch 27, welches ferner den folgenden Schritt umfasst:

(j) Einstellen der Temperatur aller Proben über einen dritten Bereich und Beenden des Einstellens der Temperatur der Proben über den dritten Bereich, wenn eine Produktansammlung ein effektives Niveau erreicht.

32. Ein Verfahren zum Durchführen einer Nucleinsäureamplifikation nach Anspruch 27, wobei die Schritte (f) und (h) ferner den Schritt des Erfassens der von der angeregten biologischen Probe emittierten Fluoreszenz im Bereich von ungefähr 400 nm bis ungefähr 800 nm umfassen.

**Revendications**

1. Appareil destiné à réaliser une réaction de polymérisation en chaîne (PCR) et à suivre la réaction en temps réel lors de cycles thermiques comprenant :

une pluralité de tubes capillaires chacun destiné à contenir un échantillon de PCR, chaque tube capillaire comprenant un matériau optiquement transparent, chaque tube capillaire contenant moins de 1 millilitre d'un échantillon ;
un carrousel pouvant tourner pour déplacer séquentiellement les échantillons de PCR un par un jusqu'à une position de suivi pendant l'amplification ;
des moyens de cyclage thermique comprenant :

un dispositif de chauffage d'air forcé destiné à chauffer tous les échantillons de PCR ;
un moyen destiné à refroidir simultanément tous les échantillons de PCR ; et
un moyen de commande destiné à faire fonctionner répétitivement le moyen de cyclage thermique pour soumettre les échantillons de PCR au cyclage thermique ;

un moyen destiné à exciter optiquement l'un des échantillons pour provoquer la fluorescence d'un échantillon ; et
un moyen destiné à détecter la fluorescence de l'échantillon excité quand l'échantillon excité se trouve dans la position de suivi.

2. Appareil destiné à réaliser une PCR et à suivre la réaction en temps réel lors de cycles thermiques tel que défini dans la revendication 1 comprenant en outre :

un moyen destiné à déterminer au moins un paramètre de réaction en conformité avec la fluorescence détectée ; et
un moyen destiné à ajuster le moyen de commande en conformité avec le paramètre de réaction.

3. Appareil destiné à réaliser une PCR et à suivre la réaction en temps réel lors de cycles thermiques tel que défini dans la revendication 2 dans lequel le moyen de commande ajuste le fonctionnement du moyen de chauffage et le moyen de refroidissement pour modifier les durées pendant lesquels le moyen de chauffage et le moyen de refroidissement fonctionnent en conformité avec le paramètre de réaction.

4. Appareil destiné à réaliser de la PCR et à suivre la réaction en temps réel lors de cycles thermiques tel que défini dans la revendication 2 dans lequel le moyen de commande ajuste le fonctionnement du moyen de chauffage et le moyen de refroidissement pour modifier la vitesse à laquelle les échantillons biologiques sont chauffés et refroidis en conformité avec le paramètre de réaction.

5. Appareil destiné à réaliser de la PCR et à suivre la réaction en temps réel lors de cycles thermiques tel que défini dans la revendication 1 dans lequel chaque tube capillaire est fabriqué au moins partiellement à partir de verre.

6. Appareil destiné à réaliser de la PCR et à suivre la réaction en temps réel lors de cycles thermiques tel que défini dans la revendication 1 comprenant en outre un moyen destiné à positionner le moyen destiné à exciter optiquement l'échantillon et le moyen destiné à détecter la fluorescence de l'échantillon excité pour optimiser la fluorescence qui est détectée.

7. Appareil destiné à réaliser de la PCR et à suivre la réaction en temps réel lors de cycles thermiques tel que défini dans la revendication 1 dans lequel le moyen de refroidissement comprend un mécanisme de mouvement d'air qui transporte de l'air ambiant vers les tubes capillaires.

8. Appareil destiné à réaliser de la PCR et à suivre la réaction en temps réel lors de cycles thermiques tel que défini dans la revendication 1 dans lequel le moyen de commande comprend un microprocesseur.

9. Appareil destiné à réaliser de la PCR et à suivre la réaction en temps réel lors de cycles thermiques tel que défini dans la revendication 1 dans lequel le moyen destiné à exciter optiquement l'échantillon comprend une structure de photoémetteur placée de telle façon que la fluorescence émise à partir de celle-ci frappe un côté du tube capillaire qui contient l'échantillon excité.

10. Appareil destiné à réaliser de la PCR et à suivre la réaction en temps réel lors de cycles thermiques tel que défini dans la revendication 9 dans lequel le moyen destiné à détecter la fluorescence de l'échantillon excité comprend une structure de photo-détecteur placée de telle sorte que la fluorescence émise à partir du côté du tube capillaire de l'échantillon excité soit détectée.

11. Appareil destiné à réaliser de la PCR et à suivre la réaction en temps réel lors de cycles thermiques tel que défini dans la revendication 1 dans lequel le moyen destiné à exciter optiquement l'échantillon comprend une structure de photoémetteur placée de telle façon que la fluorescence émise à partir de celle-ci frappe une extrémité du tube capillaire de l'échantillon excité.

12. Appareil destiné à réaliser de la PCR et à suivre la réaction en temps réel lors de cycles thermiques tel que défini dans la revendication 11 dans lequel le moyen destiné à détecter la fluorescence de l'échantillon excité comprend une structure de photo-détecteur placée de telle sorte que la fluorescence émise à partir de l'extrémité du tube capillaire de l'échantillon excité soit détectée.

13. Appareil destiné à réaliser de la PCR et à suivre la réaction en temps réel pendant le cyclage de température tel que défini dans la revendication 1 dans lequel le moyen destiné à déterminer au moins un paramètre de réaction en conformité avec la fluorescence détectée comprend un moyen de calcul informatisé destiné à déterminer au moins un paramètre de réaction choisi parmi le groupe consistant en : température de fusion de produit, temps de fusion de produit, température d'hybridation de produit, temps d'hybridation de produit, température de fusion de sonde, temps de fusion de sonde, température d'hybridation/élongation d'amorce et temps d'hybridation/élongation d'amorce.

14. Appareil destiné à réaliser de la PCR et à suivre la réaction en temps réel lors de cycles thermiques tel que défini dans la revendication 1 dans lequel le moyen de commande comprend un moyen destiné à refroidir les échantillons quand le moyen destiné à détecter la fluorescence de l'échantillon excité détecte que le produit est complètement dénaturé.

15. Appareil destiné à réaliser de la PCR et à suivre la réaction en temps réel pendant le cyclage de température tel que défini dans la revendication 1 dans lequel le moyen de commande comprend un moyen destiné à chauffer les échantillons quand le moyen destiné à détecter la fluorescence de l'échantillon excité ne détecte plus d'amplification du produit.

16. Appareil destiné à réaliser de la PCR et à suivre la réaction en temps réel lors de cycles thermiques tel que défini dans la revendication 1, qui comprend un moyen de suivi destiné à vérifier la réaction biologique ; et un moyen de commande destiné à commander le fonctionnement du carrousel, le moyen de cyclage thermique et le moyen de suivi de telle sorte que la progression de la réaction biologique soit détectée tandis que le cyclage thermique se produit, dans lequel

Le moyen destiné à exciter optiquement l'un des échantillons comprend une source d'excitation émettant une fluorescence d'excitation ; et un moyen destiné à diriger la fluorescence d'excitation vers la position de suivi de telle sorte que l'échantillon situé dans la position de suivi émette une fluorescence ; et le moyen destiné à détecter la fluorescence de l'échantillon excité comprend

un moyen destiné à convertir la fluorescence émise en un signal électrique ;
un moyen destiné à traiter le signal électrique pour arriver à un paramètre de réaction ;
un moyen destiné à afficher le paramètre de réaction ; et
un moyen destiné à enregistrer le paramètre de réaction.

**17.** Appareil destiné à réaliser de la PCR et à suivre la réaction en temps réel pendant le cyclage de température tel que défini dans la revendication 16 dans lequel le paramètre de réaction est choisi parmi le groupe consistant en la température et le temps de dénaturation, la température et le temps d'hybridation d'amorce, la température et le temps d'hybridation de sonde, la température et le temps d'élongation d'enzyme, et le nombre de cycles.

**18.** Appareil destiné à réaliser de la PCR et à suivre la réaction en temps réel lors de cycles thermiques tel que défini dans la revendication 16 dans lequel :

la source d'excitation comprend une source de photo-émission, la source de photo-émission étant choisi parmi le groupe consistant en une lampe à xénon et une diode à émission de lumière ;
le moyen destiné à convertir la fluorescence émise en un signal électrique comprend un dispositif de photo-détection, le dispositif de photo-détection étant choisi parmi le groupe consistant en un tube photomultiplicateur et une photodiode ; et
le moyen destiné à traiter le signal électrique pour arriver à un paramètre de réaction comprend un microprocesseur.

**19.** Appareil destiné à réaliser de la PCR et à suivre la réaction en temps réel lors de cycles thermiques tel que défini dans la revendication 18 dans lequel le moyen destiné à convertir la fluorescence émise en un signal électrique comprend un premier dispositif de photo-détection, le premier dispositif de photo-détection étant choisi parmi le groupe consistant en un tube photo-multiplicateur et une photo-diode et un second dispositif de photo-détection choisi parmi le groupe consistant en un tube photo-multiplicateur et une photo-diode.

**20.** Méthode de réalisation d'une amplification d'acide nucléique tout en suivant la progression de l'amplification, la méthode comprenant les étapes :

(a) de disposer un carrousel rotatif contenant une pluralité d'échantillons biologiques chacun dans un tube capillaire pour subir une amplification d'acide nucléique ;
(b) de chauffer simultanément l'ensemble des tubes capillaires avec de l'air chaud pour induire une première transition de température dans les échantillons biologiques à partir d'une première température jusqu'à une seconde température ;
(c) de refroidir simultanément l'ensemble des tubes capillaires pour induire une seconde transition de température dans les échantillons biologiques à partir de la seconde température jusqu'à la première température ;
(d) d'exciter les échantillons biologiques pendant la transition de température de telle sorte qu'une fluorescence soit émise à partir de l'échantillon ;
(e) de faire tourner le carrousel pour déplacer séquentiellement les échantillons un par un jusqu'à une position de surveillance pendant une amplification ; et
(f) de détecter la fluorescence qui est émise à partir des échantillons au moins une fois quand chaque tube capillaire se trouve dans la position de surveillance pour déterminer au moins un paramètre de réaction.

**21.** Méthode de réalisation d'une amplification d'acide nucléique telle que définie dans la revendication 20 dans laquelle l'étape (f) a lieu pendant la première transition de température et comprend l'étape de déterminer la température de fusion d'un produit.

**22.** Méthode de réalisation d'une amplification d'acide nucléique telle que définie dans la revendication 20 dans laquelle l'étape (f) a lieu pendant la première transition de température et comprend l'étape de déterminer la température de fusion d'une sonde.

**23.** Méthode de réalisation d'une amplification d'acide nucléique telle que définie dans la revendication 20 dans laquelle l'étape (b) comprend en outre l'étape d'accroître la température des échantillons biologiques à une vitesse au moins aussi grande que 2,5 °C par seconde.

**24.** Méthode de réalisation d'une amplification d'acide nucléique telle que définie dans la revendication 20 dans laquelle l'étape (b) comprend en outre l'étape d'accroître la température des échantillons biologiques d'au moins 40 °C pendant un laps de temps de pas plus de dix secondes.

**25.** Méthode de réalisation d'une amplification d'acide nucléique telle que définie dans la revendication 20 dans laquelle l'étape (f) a lieu pendant la première transition de température et comprend en outre l'étape de détecter la fluorescence émise à partir de l'échantillon excité au moins deux fois pendant la transition de température.

**26.** Méthode de réalisation d'une amplification d'acide nucléique telle que définie dans la revendication 20 dans laquelle l'étape (b) comprend l'étape d'accroître la température des échantillons biologiques jusqu'à ce que le produit dans l'échantillon excité soit complètement dénaturé.

**27.** Méthode de réalisation d'une amplification d'acide nucléique telle que définie dans la revendication 20 dans laquelle

l'échantillon biologique excité inclut une substance qui émet une fluorescence à une première longueur d'onde qui se rapporte à la progression de l'amplification d'acide nucléique ;
la transition de température à partir de la première température jusqu'à la seconde température définit une première plage ;
l'étape (f) comprend en outre de détecter la fluorescence émise par l'échantillon biologique excité une pluralité de fois quand la température de l'échantillon se trouve à l'intérieur de la première plage ;

et comprend en outre les étapes :

(g) d'ajuster la température de l'ensemble des échantillons sur une seconde plage incluant la seconde température et une troisième température, la troisième température étant différente de la seconde température ; et
(h) de détecter la fluorescence émise par l'échantillon biologique excité une pluralité de fois quand la température de l'échantillon se trouve à l'intérieur de la seconde plage.

**28.** Méthode de réalisation d'une amplification d'acide nucléique telle qu'elle est exposée dans la revendication 27 comprenant en outre l'étape :

(i) d'ajuster au moins un paramètre de la réaction d'amplification en conformité avec la fluorescence détectée dans l'étape (d) ou (h).

**29.** Méthode de réalisation d'une amplification d'acide nucléique telle qu'elle est exposée dans la revendication 27 dans laquelle l'étape (b) comprend en outre l'étape de cesser d'ajuster la température des échantillons sur la première plage quand la dénaturation du produit est substantiellement achevée.

**30.** Méthode de réalisation d'une amplification d'acide nucléique telle qu'elle est exposée dans la revendication 27 dans laquelle l'étape (g) comprend en outre l'étape de cesser d'ajuster la température des échantillons sur la seconde plage quand l'hybridation d'amorce cesse substantiellement.

**31.** Méthode de réalisation d'une amplification d'acide nucléique telle qu'elle est exposée dans la revendication 27 comprenant en outre l'étape :

(j) d'ajuster la température de l'ensemble des échantillons sur une troisième plage et de cesser d'ajuster la température des échantillons sur la troisième plage quand une accumulation de produit atteint un niveau effectif.

**32.** Méthode de réalisation d'une amplification d'acide nucléique telle qu'elle est exposée dans la revendication 27 dans laquelle les étapes (f) et (h) comprennent en outre l'étape de détecter la fluorescence émise à partir de l'échantillon biologique excité dans la plage allant d'environ 400 nm à environ 800 nm.

**Fig. 1**

**Fig. 2**

**Fig. 3**

**Fig. 4**

**TIME**
**(in Seconds)**

**Fig. 5**

### Effect of Annealing Time on Product
### Yield and Background Amplification

| 92 to 55 °C Ramp Time (sec) | | Time at 55 °C (sec) |
|---|---|---|
| 9 | | <1 |
| 25 | | <1 |
| 25 | | 5 |
| 25 | | 10 |
| 25 | | 20 |
| 25 | | 40 |
| 25 | | 80 |
| | | PhiX174 RF Hae III Digest |

**Fig. 7**

### Effect of Denaturation
### Time on Product Yield

| | Time at 92-94 °C (sec) |
|---|---|
| | <1 |
| | 2 |
| | 4 |
| | 8 |
| | 16 |
| | 32 |
| | 64 |
| | PhiX174 RF Hae III Digest |

**Fig. 6**

100

130

_138_

140

103

140

102

**Fig. 8A**

138    136    130

112A

100

103

112B    110    132

126

128

102

134    110    131

108    112 137    129

124

106

122    120

136    118

122

104    114    104    116

**Fig. 8B**

**Fig. 8C**

Fig. 9A

Fig. 9B

Fig. 9C

*Fig. 9D*

EP 1 442 794 B1

*Fig. 9E*

Fig. 9F

EP 1 442 794 B1

Fig. 9G

Fig. 10

TEMPERATURE TRANSITION RATE (°C/sec)

1        2        5        10        20

← 536 bp product

Fig. 10A

*Fig. 11*

**Fig. 11A**

Fig. 12C

Fig. 12

Fig. 12B

Fig. 12A

*Fig. 13*

*Fig. 14*

Initial
Template Copies

◆ $10^9$
□ $10^8$
▲ $10^7$
× $10^6$
◇ $10^5$
● $10^4$
+ $10^3$
- $10^2$
— $10$
■ $1$
△ $0$

*Fig. 14A*

*Fig. 15*

*Fig. 16*

*Fig. 17*

**Fig. 18A**

**Fig. 18B**

**Fig. 18C**

Fig. 19

Fig. 19A

Fig. 19B

Fig. 19C

Fig. 19D

Fig. 19E

Fig. 19F

Fig. 19G

Fig. 19H

Fig. 19I

Fig. 20

**Fig. 21**

Load
Sample

*Fig. 21A*

Centrifuge

*Fig. 21B*

Seal

*Fig. 21C*

Cycle

*Fig. 21D*

Temperature

Product
Melting

Product
Reannealing

Probe
Annealing

Probe
Melting

Primer
Annealing & Extension

← Product Tm

← Probe Tm

← Primer Tm

Time

*Fig. 22*

**Fig. 22A**

**Fig. 22B**

*Fig. 22C*

**Fig. 22D**

**Fig. 22E**

**Fig. 23**

**Fig. 24**

*Fig. 25*

*Fig. 26*

*Fig. 27B*

*Fig. 27A*

Fig. 28

Fig. 29

Fig. 30A

Fig. 30B

**Fig. 30C**

*Fig. 30D*

**Fig. 30E**

**Fig. 30F**

Fig. 30G

TEMP SIDE

+5V

IC5A
10K

IC15A

DS1 P1.0

IC9A

AC ENABLE

1     2     1     16

DS1 BIP1.0

7404

+5V   IC5B    2      IC9B    COIL CONTROL
14     10K

DS1 P1.1     2    15    DS1 BP1.1

+5V   IC5C    3      IC9C    FAN ENABLE
14     10K

DS1 P1.2     3    14    DS1 BP1.2

+5V   IC5D    4      IC9D    FAN DAT
14     10K

DS1 P1.3     4    13    DS1 BP1.3

+5V   IC5E    5      IC9E    FAN CS
14     10K

DS1 P1.4     5    12    DS1 BP1.4

+5V   IC5F    6      IC9F    FAN CLK
14     10K

DS1 P1.5     6    11    DS1 BP1.5

+5V          +5V

IC5J        IC5K
10K        10K

I O AQUIRE     I O NEXT SEG

DS1 P3.4       DS1 P3.5

**Fig. 30H**

**Fig. 30l**

STEP SIDE

+5V

IC7A
10K

IC10A          STEP ENABLE

DS2 P0.0          1          16          DS2 BP0.0

+5V  IC7B          2          IC10B          RAD CLK
14
10K

DS2 P0.1          2          15          DS2 BP0.1

+5V  IC7C          3          IC10C          RAD DIR
14
10K

DS2 P0.2          3          14          DS2 BP0.2

+5V  IC7D          4          IC10D          CAR CLK
14
10K

DS2 P0.3          4          13          DS2 BP0.3

+5V  IC7E          5          IC10E          CAR DIR
14
10K

DS2 P0.4          5          12          DS2 BP0.4

+5V                    +5V                    +5V

IC10M          IC5L          IC5M
14          14          14
10K          10K          10K

LID FEED BACK          RAD HOME          I O AQ DONE

DS2 P1.5          DS2 P1.6          DS2 P3.4

**Fig. 30J**

**Fig. 30K**

*Fig. 30L*

Fig. 30M

*Fig. 30N*

122

TEMP CIRCUIT

Fig. 300

EP 1 442 794 B1

123

**Fig. 30P**

LED DRIVER

Fig. 30Q

## STATIC MEMORY BATTERY

V LITH

BT1
3V LITHIUM

## INDICATORS

+5V MAIN

R19
470

RED ANODE

+5V

R20
470

GREEN ANODE

## HALL SENSOR

+5V

CAR HOME

DS2 P1.7

JP1

1
2
3

HALL SENSOR

## MAIN BOARD FUSE

J1

1
2

CON2

F1

1

1A

2

UNREG DC

**Fig. 30R**

EP 1 442 794 B1

MAIN I/O

+5V

AC ENABLE +V

AC ENABLE

DS1 BIP1.0
RED ANODE
GREEN ANODE

IND GND

PB

COIL CONTROL

DS1 BP1.1 — FAN ENABLE
DS1 BP1.2 — FAN CLK
DS1 BP1.5 — FAN DAT
DS1 BP1.3 — FAN CS
DS1 BP1.4 — STEP ENABLE
DS2 BP0.0 — RAD CLK
DS2 BP0.1 — RAD DIR
DS2 BP0.2 — CAR CLK
DS2 BP0.3 — CAR DIR
DS2 BP0.4 — LID SWITCH GND

LID FEED BACK

DS2 P1.5 — RAD SWITCH GND

DS2 P1.6 — RAD HOME

+5V

BLUE CATHODE

JP5

1
2
3
4
5
6
7
8
9
10
11
12
13
14
15
16
17
18
19
20
21
22
23
24
25

MAIN HEADER

Fig. 30S

EP 1 442 794 B1

FLUORIMETER I/O

Fig. 30T

Fig. 30U

# CAROUSEL HOME POSITION SENSOR

**Fig. 30V**

**Fig. 31A**

**Fig. 31B**

Fig. 32

EP 1 442 794 B1